(19) Europäisches Patentamt
European Patent Office
Office européen des brevets

(11) EP 4 506 467 B1

(12) **EUROPEAN PATENT SPECIFICATION**

(45) Date of publication and mention of the grant of the patent:
**31.12.2025 Bulletin 2026/01**

(21) Application number: **24189485.6**

(22) Date of filing: **18.07.2024**

(51) International Patent Classification (IPC):
***C12Q 1/6851*** *(2018.01)* ***C12Q 1/6886*** *(2018.01)*
***C12Q 1/6806*** *(2018.01)*

(52) Cooperative Patent Classification (CPC):
(C-Sets available)
**C12Q 1/6851; C12Q 1/6806; C12Q 1/6886;**
C12Q 2521/107; C12Q 2525/191; C12Q 2525/307;
C12Q 2600/158 (Cont.)

(54) **METHOD AND KIT FOR DIAGNOSIS, PROGNOSIS, THERAPY MONITORING OR OUTCOME PREDICTION ASSESSMENT OF A DISEASE**

VERFAHREN UND KIT ZUR DIAGNOSE, PROGNOSE, THERAPIEÜBERWACHUNG ODER ERGEBNISVORHERSAGEBEURTEILUNG EINER KRANKHEIT

PROCÉDÉ ET KIT POUR LE DIAGNOSTIC, LE PRONOSTIC, LA SURVEILLANCE DE THÉRAPIE OU L'ÉVALUATION DE PRÉDICTION DE RÉSULTAT D'UNE MALADIE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **07.08.2023 IT 202300016836**

(43) Date of publication of application:
**12.02.2025 Bulletin 2025/07**

(73) Proprietor: **Immagina Biotechnology S.r.l.**
**38057 Pergine Valsugana (TN) (IT)**

(72) Inventors:
- **DEL PIANO, Alessia**
**38121 Via Grezoni 8, Trento (IT)**
- **KECMAN, Tea**
**38122 Via Giuseppe Grazioli 63, Trento (IT)**
- **CLAMER, Massimiliano**
**38123 Via del Castel 21, Trento (IT)**

(74) Representative: **Buzzi, Notaro & Antonielli d'Oulx S.p.A.**
**Corso Vittorio Emanuele II, 6**
**10123 Torino (IT)**

(56) References cited:
**WO-A1-2017/112666 WO-A1-2021/048720**
**US-A1- 2021 355 548**

- **DEL PIANO ALESSIA ET AL: "Phospho-RNA sequencing with circAID-p-seq", vol. 50, no. 4, 1 December 2021 (2021-12-01), GB, pages e23 - e23, XP093118245, ISSN: 0305-1048, Retrieved from the Internet <URL:https://academic.oup.com/nar/article-pdf/50/4/e23/42617959/gkab1158.pdf> DOI: 10.1093/nar/gkab1158**
- **GIRALDEZ MARIA D ET AL: "Phospho-RNA-seq: a modified small RNA-seq method that reveals circulating mRNA and lncRNA fragments as potential biomarkers in human plasma", vol. 38, no. 11, 3 June 2019 (2019-06-03), Oxford, XP093133669, ISSN: 0261-4189, Retrieved from the Internet <URL:https://www.ncbi.nlm.nih.gov/pmc/articles/PMC6545557/pdf/EMBJ-38-e101695.pdf> DOI: 10.15252/embj.2019101695**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 506 467 B1

(52) Cooperative Patent Classification (CPC): (Cont.)

C-Sets
**C12Q 1/6851, C12Q 2525/191**

## Description

### Field of the invention

The present description concerns a method and kits for diagnosis, prognosis, therapy monitoring or outcome prediction assessment of a disease by detecting and quantifying RNA fragments comprising a 3' phosphate or a 2'/3' cyclic phosphate as molecular marker of the disease.

### Background

A biomarker is a biomolecule whose measurement provides meaningful information about the presence or the progress of a disease and the effects of a treatment[1,2]. RNA-based biomarkers stand out among the diverse range of molecular markers as highly promising candidates for the purposes of disease diagnosis, prognosis, and treatment monitoring in various conditions, including cancer, neurodegenerative disorders, and infectious diseases[3]. To refine the development of biomarkers extensive research has been dedicated to the investigation of diverse RNA types, including messenger RNA (mRNA), microRNA (miRNA), transfer RNA (tRNA), long non-coding RNA (lncRNA), and circular RNA (circRNA)[4-6]. RNA offers several key advantages compared with protein-based biomarkers for this purpose, including its typically abundant presence, ease of detection through cost-effective methods, and its presence in a wide range of biological fluids[7] (e.g., urine, blood, saliva). Besides this, RNAs exhibit differential expression patterns in numerous pathological conditions compared to a healthy state[3,8], emphasizing their potential as distinctive disease markers. Additionally, RNA molecules undergo significant post-transcriptional modification[9], further enhancing their uniqueness, with organ, tissue, and cell type specificity[10-12]. Among known modifications, the presence of a 3' phosphate or a 2'/3' cyclic phosphate group on an RNA molecule (referred to as 3'P RNA), arise from RNA processing events, primarily enzymatic cleavage of non-coding RNAs such as rRNA, snRNA, and tRNA, but also selective degradation of protein coding RNAs[13,14]. This process generates RNA fragments ranging from 10 to 200 nucleotides in length, offering a vast collection of disease-specific markers. Regrettably, the true potential of these RNA products often goes unnoticed due to the current limitations of detection methods, which rely on the presence of a 3'OH group for subsequent processing steps like polyadenylation or ligation. By using complex and time consuming approaches, preliminary studies have begun to illuminate the significance of 3'P RNAs as distinctive markers of essential biological processes [15,16] by acting intracellularly or in biological fluids[17]. Altered expression of specific 3'P RNA fragments has been observed in cancer, viral infection and neurodegeneration[18,19]. This emerging understanding underscores the importance of exploring their role in human health and disease. Depending on the sample type, from 5% to 50% of the overall 3'P RNAs are tRNA fragments (tRFs), meaning fragments derived from cleavage of mature tRNA. For instance, a specific subset of tRNAs cleaved by angiogenin (a nuclease belonging to RNAse A superfamily) were recently found to be secreted from neural cells and found in serum samples of amyotrophic lateral sclerosis patients, holding strong prognostic value[20]. Further, specific tRNA fragments were recently discovered to repress aberrant protein synthesis and predict leukemic progression in myelodysplastic syndrome[21], suggesting a role of 3'P tRNA fragments as potential marker of disease 22-25

Despite significant efforts invested to date, the field still lacks reliable, simple and accurate methods for effectively screening and quantifying 3'P RNA fragments. This absence of robust screening techniques poses a major obstacle in the identification of this type of RNA molecules, limiting their potential utilization for essential purposes such as disease diagnosis, prognosis, and therapy monitoring.

### Summary of the invention

The object of this disclosure is to present a comprehensive method for identifying and quantifying molecular markers associated with a specific disease to facilitate disease diagnosis, prognosis, therapy monitoring or outcome prediction assessment. Particularly, the focus is on molecular markers represented by RNA fragments that are phosphorylated at the 3' end. By leveraging on an innovative approach, the aim is to enable more accurate and reliable diagnostic, prognostic, therapy monitoring and outcome prediction assessments.

A further object of this disclosure is to provide kits for implementing the method for identifying molecular markers of a disease and for using them in diagnosis, outcome prediction, prognosis and therapy monitoring.

According to the invention, the above objects are achieved thanks to the subject matter recalled specifically in the ensuing claims, which are understood as forming an

The present invention concerns an *in vitro* or *ex vivo* method for diagnosis, prognosis, therapy monitoring or outcome prediction assessment of a disease or condition as defined in claim 1, and kit for diagnosing spinal muscular atrophy (SMA) and cutaneous squamous cell carcinoma (cSCC) as defined in claims 10 and 11.

## Brief description of the drawings

The invention will now be described in detail, purely by way of an illustrative and non-limiting example and, with reference to the accompanying drawings, wherein:

- **Figure 1.** Metaprofile showing the frequency of P-sites around translation initiation site and translation termination. The library was generated by Dart-RNA sequencing after ribosome purification and extraction of 3'P ribosome protected fragments from CHO cell pellets.
- **Figure 2.** Dart-RNAseq analysis of mouse liver tissue. Reads length distribution of mouse control and SMA liver tissues.
- **Figure 3.** Percentage of reads mapping on different transcript types.
- **Figure 4.** Per-base coverage of 3'P-tRFs_Val_AAC in control (black line) and SMA (grey line) mouse liver tissue.
- **Figure 5** Heatmap reporting the frequency of cleavage associated to the 5' start and 3' end of reads aligning on tRNA_Val_AAC, in control and SMA liver tissues.
- **Figure 6** Schematic representation of secondary structure of full length transcript of the U2 pseudogene. In dark black is highlighted the Sm binding site and the region where the 3'P Gm22973 fragments is mapping. The name associated to each stem loop is reported as reference.
- **Figure 7. A.** 3'P-qPCR expression analysis of 3'P-tRFs_Val_AAC/CAC and 3'p-tRFs_Val_TAC in control and SMA liver tissues. Results are presented as relative log2 fold change. **B.** Representative images (left) of northern blot analyses of total full-length tRNA Val AAC (up) and total 3'PtRF Val_AAC fragment (bottom) from control and SMA liver tissue. On the right, histogram showing the relative expression (Fold change) of 3'P-tRFs_Val_AAC based on the quantification of the bands in gel is shown on the right. Data are expressed as the mean ± standard error of the mean (s.e.m.). Comparisons between two groups were performed using a Student's t test; n = 3; * P<0.05 and n.s.: not significant.
- **Figure 8. A.** 3'P-qPCR expression analysis of 3'P-Gm22973 fragment in control and SMA liver tissue **B.** 3'P-qPCR expression analysis of full length Gm22973 transcript in control and SMA liver tissue. **C.** histogram showing results of 3'P-qPCR expression analysis of 3'P-U2 in control and SMA liver tissue. **D.** Bar plot showing full-length qPCR expression analysis of U2 snRNA transcript in control and SMA liver tissue. Data are expressed as the mean ± standard error of the mean (s.e.m.). Comparisons between groups were performed using a Student's t test; N = 3; * P < 0.05; ** P< 0.01 and n.s.: not significant.
- **Figure 9.** Percentage of reads mapping on different transcript types in dart-RNAseq analysis of immortalized human keratinocytes (HKC) and SCC cell lines.
- **Figure 10.** Principal component analysis (PCA) of Dart-RNAseq results obtained from HKC and SCC samples.
- **Figure 11. A.** 3'P-qPCR analysis and relative expression of 5'_tRFs_Glu_CTC in keratinocytes (HKC) and cSCC. **B.** 3'P-qPCR analysis and relative expression of 3'_tRFs_Asp_GTC in keratinocytes and cSCC. Data are expressed as the mean ± standard error of the mean (s.e.m.). Comparisons between two groups were performed using a Student's t test; n = 3; *** P<0.001.
- **Figure 12.** Schematic representation of Dart-RNAseq method. **A.** Dart-RNAseq two PCR steps. **B.** Dart-RNAseq one PCR step.
- **Figure 13.** Schematic representation of 3'P-qPCR assay.

## Definitions

By "3'P RNA" is meant an RNA fragment comprising a 3' phosphate or a 2'/3' cyclic phosphate.

By "Dart-RNAseq analysis" is meant the method for identifying a 3'P RNA as a molecular marker of a disease or pathologic condition in a biological sample developed by the present inventors and disclosed herein.

By "3'P-qPCR assay" is meant the method of diagnosing, assessing the risk of developing or prognosing a disease or condition by determining the profile of a molecular marker represented by a 3'P RNA in a biological sample developed by the present inventors and disclosed herein.

By "PCR" or "polymerase chain reaction" is meant the selective amplification of DNA or RNA targets using the polymerase chain reaction. During PCR, short single-stranded (ss) synthetic oligonucleotides or primers are extended on a target template using repeated cycles of heat denaturation, primer annealing, and primer extension.

By "qPCR" or "quantitative polymerase chain reaction" is meant is a PCR-based technique that couples amplification of a target DNA or RNA sequence with quantification of the concentration of that DNA/RNA species in the reaction. This method enables calculation of the starting template concentration.

By "sequencing platform adapter construct" is meant a nucleic acid construct utilized by a commercially available sequencing platform such as, e.g., Illumina® (e.g., the HiSeq™, MiSeq™ and NovaSeq™ sequencing systems); Element Bioscience™ (e.g., LoopSeq for AVITI™ sequencing systems); Singular genomics (e.g., the G4 system); Life Technologies™ (e.g., a SOLD sequencing system); Roche (e.g., the 454 GS FLX+ and/or GS Junior sequencing systems); MGI (e.g., E25, G400, G99, G50 and T7, T10, T20 systems).

A sequencing platform adapter construct includes one or more nucleic acid domains.

By "nucleic acid domain" is meant an oligonucleotide molecule having a length and sequence suitable for the sequencing platform of interest, i.e. enabling a polynucleotide employed by the sequencing platform of interest to specifically bind to the nucleic acid domain.

The nucleic acid domains can have a length from 4 to 200 nts, from 4 to 100 nts, from 6 to 75, from 8 to 50, or from 10 to 40 nts.

The nucleotide sequences of nucleic acid domains useful for sequencing on a sequencing platform of interest may vary and/or change over time. Adapter sequences are typically provided by the manufacturer of the sequencing platform (e.g., in technical documents provided with the sequencing system and/or available on the manufacturer's website). Based on such information, the sequence of adapter, reverse transcription primer, and/or amplification primers, may be designed to include all or a portion of one or more nucleic acid domains in a configuration that enables sequencing the nucleic acid insert object of the analysis on the platform of interest (in the present case the 3'P RNA).

The nucleic acid domains can be selected from: a "capture domain" that specifically binds to a surface-attached sequencing platform oligonucleotide (e.g., the P5 or P7 oligonucleotides attached to the surface of a flow cell in an Illumina® sequencing system); a "sequencing primer binding domain" (e.g., a domain to which the Read 1 or Read 2 primers of the Illumina® platform may bind); a "barcode domain" (e.g., a domain that uniquely identifies the sample source of the nucleic acid being sequenced to enable sample multiplexing by marking every molecule from a given sample with a specific barcode or "tag"); a "barcode sequencing primer binding domain" (a domain to which a primer used for sequencing a barcode binds); a "molecular identification domain" (e.g., a molecular index tag, such as a randomized tag of 4, 6, or other number of nucleotides) for uniquely marking molecules of interest to determine expression levels based on the number of instances a unique tag is sequenced; or any combination of such domains. In certain aspects, a barcode domain (e.g., sample index tag) and a unique molecular identification (UMI) domain (e.g., a molecular index tag) may be included in the same nucleic acid domain.

By "spacer allowing the arrest of a retrotranscriptase enzyme activity" is meant a chemical modification that can be used to mimic the presence of a naturally occurring abasic site resulting from depurination or other mechanisms. The modification involves the replacement of the deoxyribose sugar with a modified sugar molecule lacking the 2'-hydroxyl group. This modification disrupts the normal base pairing and hydrogen bonding interactions between nucleotides in the oligonucleotide. It can be selected among a 1,2'-dideoxyribose modification (dSpacer having the following chemical structure

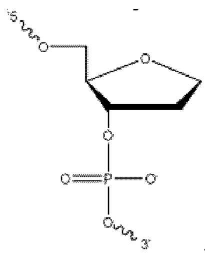,

also known as abasic site), tetrahydrofuran (THF), or apurinic/apyrimidinic (AP) site. Alternatively, it can be a biotinylated blocking spacer, "Int Biotin dT", i.e., a deoxythymidine (dT) conjugated with a biotin molecule, having the following structure

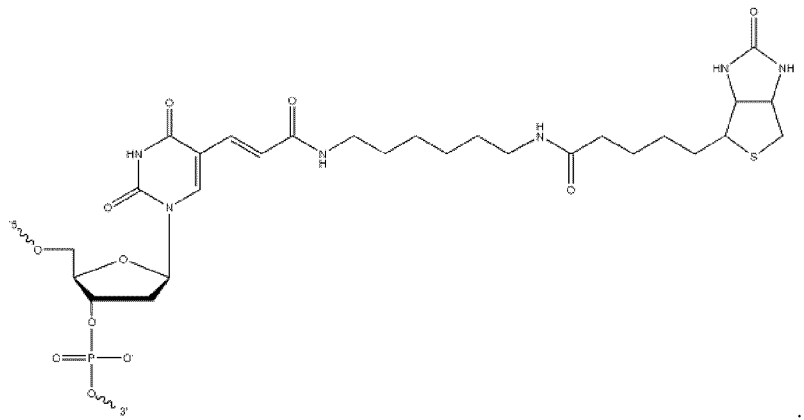

.

By "nucleobase", "nitrogenous base" or simply "base" is meant a nitrogen-containing biological compound that forms a nucleoside, which, in turn, is a component of a nucleotide.

By "p-value" is meant a statistical measure of the significance of a result or observation. The p-value can be determined by means of different statistical parameters. The most known statistical parameter is the null hypothesis, that represents a statement of no effect or no relationship between variables. The p-value helps assess the evidence against the null hypothesis and supports the decision of whether to reject or fail to reject it. Specifically, the p-value represents the probability of obtaining a test statistic as extreme as, or more extreme than, the one observed in the data, assuming that the null hypothesis is true. If the p-value is very small (typically below a predefined significance level, such as 0.05 or 0.01), it suggests that the observed data is unlikely to have occurred under the null hypothesis alone. In such cases, the evidence against the null hypothesis is considered strong, and the null hypothesis is rejected in favor of the alternative hypothesis. Conversely, if the p-value is relatively large (greater than the chosen significance level, usually 0.05), it indicates that the observed data is not unusual or extreme under the null hypothesis. In this situation, there is insufficient evidence to reject the null hypothesis, and it is retained.

Other statistical parameters to define a p-value are known to the skilled man, and among others are:

- Test Statistic: The test statistic is a numerical summary of the data that is used to evaluate the hypothesis being tested. The choice of test statistic depends on the nature of the data and the research question. Examples of commonly used test statistics include t-statistics, chi-square statistics, F-statistics, and z-scores.
- Alternative Hypothesis: The alternative hypothesis represents the opposite of the null hypothesis and reflects the effect or relationship that the researcher is interested in detecting. It is typically formulated as a statement of a specific effect size or a difference between groups.

- Significance Level: The significance level, often denoted as $\alpha$ (alpha), is the predetermined threshold for determining statistical significance. It represents the maximum allowable probability of rejecting the null hypothesis when it is true. Commonly used significance levels are 0.05 (5%) and 0.01 (1%).

Various statistical software packages and libraries provide built-in functions to calculate p-values for different tests, making the process more straightforward for researchers. The p-values should be interpreted in conjunction with effect sizes, confidence intervals, and other relevant measures to make informed conclusions about the data and the research question at hand.

There are differences in how the p-value is calculated and interpreted in the 3'P-qPCR assay and the Dart-RNAseq analysis method.

In 3'P-qPCR, the p-value is typically calculated using statistical tests such as the Student's t-test or analysis of variance (ANOVA). The 3'P-qPCR p-value assesses the likelihood that the observed differences in RNA expression between two groups (e.g., treatment vs. control) occurred by chance alone. A low p-value indicates that the observed differences in RNA expression are statistically significant, suggesting a real difference between the groups being compared. The threshold for significance (often denoted as alpha, $\alpha$) is typically set at 0.05. If the p-value is below this threshold, the results are considered statistically significant.

In the Dart-RNAseq analysis method, the p-value is usually associated with differential expression analysis, which aims to identify genes that show significant changes in expression between two conditions or groups. The p-value is often calculated using statistical methods like edgeR, DESeq2, or limma, which employ count-based models and account for the inherent variability in next-generation sequencing (NGS) data. The p-value represents the probability that the observed differential RNA expression is due to random variation alone. A low p-value indicates that the observed

differential expression is statistically significant, suggesting true differences between the compared conditions. The significance threshold ($\alpha$) for p-values is also commonly set at 0.05 or lower to determine statistically significant differential expression.

It's important to note that the calculation and interpretation of p-values in 3'P-qPCR and Dart-RNAseq analysis depend on the specific statistical methods and algorithms used. Additionally, it's essential to consider other factors such as multiple testing corrections (e.g., Bonferroni correction) to control for false discovery rates when analyzing large-scale datasets in Dart-RNAseq analysis data.

For the sake of clarity, we named "3'P-qPCR p-value" when the p-value is calculated in the 3'P-qPCR assay and we named "Dart-RNAseq p-value" when the p-value is calculated in Dart-RNAseq analysis.

By "multimapping score" is meant a metric that quantifies the alignment ambiguity or the number of potential transcriptomic locations to which a read can be mapped. It assesses the level of uncertainty or multiple mapping possibilities associated with a given read. In sequencing data analysis (as in the Dart-RNAseq analysis), reads are short sequences obtained from the sequenced fragments of RNA molecules. The goal is to align or map these reads to a reference transcriptome to determine their origin or location. However, due to various factors such as the length of the reads, repetitive regions or highly similar sequences in the transcriptome, some reads may map to multiple locations with equal or similar alignment scores. The multimapping score provides a measure of the ambiguity associated with read mapping. It indicates the number of potential transcriptomic positions where a read can be mapped with similar alignment scores. A higher multimapping score implies a higher level of ambiguity, indicating that the read could originate from multiple transcriptomic regions or transcripts with comparable alignment qualities. The multimapping score is commonly used in sequencing data analysis pipelines to assess the reliability of read mapping results and to filter out reads with excessive mapping ambiguity. By considering the multimapping score, a skilled person can make informed decisions about the confidence of read alignments and their subsequent downstream analyses.

By "normalized counts based on sequencing depth" or "a normalized parameter based on the number of counts" is meant counts for differences in sequencing depth and library size between samples to make the read count data comparable across samples and enable meaningful statistical analyses. It is calculated by dividing the raw read count for each gene or transcript by a normalization factor, which is typically based on the total number of reads in the sample or the median read count across all samples in the dataset. Normalization is important in sequencing data analysis because it allows for accurate comparisons between samples and identification of differentially expressed genes or transcripts. Without normalization, differences in sequencing depth and library size can lead to biased results and make it difficult to distinguish true biological changes from technical variation. Normalized counts are typically used for downstream analyses, such as differential gene expression analysis, pathway analysis, and clustering. Normalization is performed by: (i) calculating the reads per kilobase per million mapped reads (RPKM), that normalizes the read count by gene length and total number of mapped reads in the sample, and expresses the result as the number of reads per kilobase of gene length per million mapped reads; or (ii) calculating the transcripts per million (TPM), that similarly to RPKM, normalizes the read count by gene length and total number of mapped reads, but also takes into account the number of isoforms or transcript variants for each gene. Other normalization methods are available for sequencing data analysis, such as the "trimmed mean of M-values" (TMM), "quantile normalization," or "DESeq normalization." The choice of normalization method may depend on the specific analysis pipeline, data characteristics, and objectives.

By "Ct value" is meant the cycle number at which the fluorescence signal of the target RNA reaches a detectable threshold level.

By "fold change" is meant a measure of the relative change in gene expression levels between two conditions or samples. In other words, a measure of the upregulation or downregulation of the target RNA in response to a specific treatment, condition, or experimental setting. It helps to understand the relative differences in RNA expression levels and assess the impact of experimental variables on RNA expression patterns.

The fold change is usually calculated by comparing the expression levels of a target RNA between two conditions or samples, often referred to as the "treatment" and "control" groups.

It's important to note that the calculation of fold change may also involve normalization steps to correct for technical variations and to make the data comparable across samples or conditions. The specific normalization methods may differ between 3'P-qPCR and Dart-RNAseq analysis experiments. Overall, while both methods provide information about gene expression changes, the calculation and interpretation of fold change can vary between these methods due to their different principles and data output formats.

For the sake of clarity, we named "3'P-qPCR fold change" when the fold change (FC) is calculated in the 3'P-qPCR assay and we named "Dart-RNAseq fold change" when the fold change (FC) is calculated in Dart-RNAseq data analysis.

In the 3'P-qPCR the fold change can be calculated according to the following equation:

$$3'\mathrm{P} - \mathrm{qPCR\ fold\ change} = \frac{2^{Ct(contr)_{target} - Ct(Treat)_{target}}}{2^{Ct(contr)_{ref} - Ct(Treat)_{ref}}} = \frac{2^{\Delta Ct(target)}}{2^{\Delta Ct(ref)}} = 2^{\Delta\Delta Ct}$$

wherein

$Ct(contr)_{target}$ is the Ct value for the target RNA determined in the control sample,
$Ct(treat)_{target}$ is the Ct value for the target RNA determined in the biological sample,
$Ct(contr)_{ref}$ is the Ct value for the reference determined in the control sample,
$Ct(treat)_{ref}$ is the Ct value for the reference determined in the biological sample.

There are alternative mathematical methods for calculating the 3'P-qPCR fold change known to the skilled man. Here are a few examples:

- Efficiency Correction Method: This method takes into account the differences in PCR amplification efficiency between the 3'P RNA (target gene) and the RNA-based adapter (reference gene). It involves calculating the relative amplification efficiency (E) for each RNA and using it to correct the Ct values before calculating fold change. The equation for the determination of the fold change according to this method is:

$$\text{fold change} = (E_{_target})\text{\^{}}(Ct(reference) - Ct(target))$$

wherein:

$$E = \text{amplification efficiency} = 10^{\wedge-1}/\text{slope},$$

Slope = the slope of the standard curve, plotted with the y axis as Ct and the x axis as log(quantity).

- Standard Curve Method: This method utilizes a standard curve generated from a series of known template concentrations to determine the relative expression of the target gene. The Ct values of the target gene in each sample are interpolated onto the standard curve to obtain the corresponding template concentration (g/L). The template can be the treated or the control conditions. The "fold change based on the standard curve" is then calculated by comparing the template concentration between the treated and the control conditions according to the following equation[26]:

fold change based on the standard curve = (g/L of treated condition) / (g/L control condition).

- Comparative Ct Method: also known as the 2^-ΔCt method, this approach compares the Ct values of the target gene directly without using a reference gene. The Ct values of the target gene in each condition/sample are normalized to a calibrator sample, typically a reference condition or a control sample. Fold change is calculated as 2^(-ΔCt), where ΔCt represents the difference between the Ct value of each condition/sample and the Ct value of the calibrator.
- Relative Expression Software Tool (REST)[27]: REST is a widely used software tool that calculates fold change based on PCR efficiencies and Ct values. It employs a mathematical model to estimate fold change and provides statistical analysis, including confidence intervals and p-values.

In the Dart-RNAseq analysis the fold change is calculated by comparing the read counts or expression levels of genes between two conditions or samples. The fold change is determined by calculating the ratio of the expression levels of a gene in the treatment group to that in the control group according to the following equation:

$$\text{Dart-RNAseq Fold change} = \left(\frac{nCounts_{Treat}}{nCounts_{CTRL}}\right)$$

wherein

$nCounts_{Treat}$ is the "normalized parameter based on the number of counts" for the target gene determined in the

biological sample,
$nCounts_{CTRL}$ is the "normalized parameter based on the number of counts" for the target gene determined in the control sample.

The expression levels in the Dart-RNAseq analysis are often based on "a normalized parameter based on the number of counts" represented as reads per kilobase of transcript per million mapped reads (RPKM) or fragments per kilobase of transcript per million mapped reads (FPKM). The Dart-RNAseq fold change values are typically logarithmically transformed, such as log2-fold change or log10-fold change. Log-transformed fold change values are commonly used to better represent the magnitude of change and to linearize the data distribution.
By "ribonucleotide having a modified nucleobase conferring nuclease resistance" is meant a ribonucleotide with enhanced stability and resistance against nuclease activity. Various modifications have been developed to confer nuclease resistance to ribonucleotides. These modifications can involve chemical alternations to the nucleobase structure, such as the addition of specific functional groups or substitution of certain atoms. Examples of modified nucleobases that confer nuclease resistance include, but are not limited to:

- 2'-O-Methyl (2'-OMe) Ribonucleotides: In this modification, the 2'-hydroxyl group of the ribose sugar is replaced with a methyl group. This modification enhances nuclease resistance and stability without significantly affecting RNA folding or function.
- 2'-Fluoro (2'-F) Ribonucleotides: The 2'-hydroxyl group of the ribose sugar is replaced with a fluorine atom in this modification. It improves resistance to nucleases and enhances RNA stability.
- Locked Nucleic Acids (LNAs): LNAs involve the introduction of a methylene bridge between the 2' oxygen and 4' carbon of the ribose sugar, effectively "locking" the ribose in the C3'-endo conformation. LNAs improve nuclease resistance and also enhance binding affinity to complementary RNA or DNA sequences.
- Phosphorothioate Linkages: In this modification, one of the non-bridging oxygen atoms of the phosphodiester backbone is replaced with a sulfur atom. This modification enhances nuclease resistance by introducing a chiral center and altering the conformation of the backbone.
- Peptide Nucleic Acids (PNAs): PNAs are synthetic nucleic acid analogs where the sugar-phosphate backbone is replaced with a peptide-like backbone. PNAs exhibit excellent nuclease resistance due to their nonionic nature and unique backbone structure.
- 2'-O-(2-Methoxyethyl) (2'-MOE) Ribonucleotides: This modification involves replacing the 2'-hydroxyl group with a 2-methoxyethyl group. It confers increased nuclease resistance and improves stability while maintaining RNA hybridization properties.

By "5-methylcytosine" (5mC) is meant a modified ribonucleotide, wherein this modification involves adding a methyl group at the 5-position of cytosine.
By "Minor Groove Binder" or MGBs is meant a crescent-shaped molecules that selectively bind non-covalently to the minor groove of DNA, a shallow furrow in the DNA helix.
By "Spacer Molecule" is meant a flexible molecule or stretch of molecules that are used to link 2 molecules of interest together.

## Detailed description of the invention

In the following description, numerous specific details are given to provide a thorough understanding of the embodiments. The embodiments can be practiced without one or more of the specific details, or with other methods, components, materials, etc. In other instances, well-known structures, materials, or operations are not shown or described in detail to avoid obscuring aspects of the embodiments.
Reference throughout this specification to "one embodiment" or "an embodiment" means that a particular feature, structure, or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.
The headings provided herein are for convenience only and do not interpret the scope or meaning of the embodiments.
A major issue in developing RNA biomarker is the lack of technologies to identify robust candidates, combined with simple and cost-effective technology to detect them [28]. To foster biomarker discovery, the present inventors developed a method (named "Dart-RNAseq") that allows for the identification of a 3'P RNA as disease biomarkers and subsequently confirmed the correct identification of the biomarkers by a targeted quantitative PCR assay (named "3'P-qPCR assay"), being a method for diagnosing, assessing the risk of development or prognosing the disease of

interest.

The Dart-RNAseq method schematically shown in Figure 12 essentially requires:

a. the 5' phosphorylation of a 3'P RNA;
b. a first ligation of the 3'P RNA with an RNA-based adapter. In some instances, the RNA-based adapter includes at least part of one nucleic acid domain of a sequencing platform adapter construct and optionally a unique molecular identifier or other barcode to mark each 3'P RNA from a specific source (i.e. unique type of cells or a single cell);
c. a second ligation step, called intra-molecular circularization, to obtain a circular RNA;
d. a single step retro-transcription, with a DNA primer annealing on part of the RNA-based adapter sequence, to obtain a cDNA copy of the circular RNA. It can contain none, or at least one nucleic acid domain of the sequencing platform adapter construct;
e. a PCR amplification carried out in one step (see panel B. of figure 12) or in two sequential steps (see panel A. of figure 12), wherein:

- the one step PCR is made with primers annealing on at least one portion of the RNA-based adapter sequence and containing all the nucleic acid domains of the sequencing platform adapter construct,
- the two sequential steps comprise: a first PCR step carried out with primers annealing on at least one portion of the RNA-based adapter sequence (to amplify the cDNA copies of the circular RNA) and containing one or more nucleic acid domains of the sequencing platform adapter construct, and a second PCR step carried out with primers annealing on the first PCR primer sequences and containing one or more nucleic acid domains of the sequencing platform adapter construct;

f. sequencing the amplification product;
g. repeating steps (a) to (f) on at least one biological sample of a healthy, treated or non-treated subject as a control;
h. identifying the at least one 3'P RNA molecule as the molecular marker of the disease by determining the fulfilment of some specific conditions and thresholds.

The 3'P-qPCR assay, schematically shown in figure 13, is based on an optimal design of hybrid primers for sequence-specific 3'P RNA disease marker(s) amplification, detection and evaluation. The 3'P-qPCR assay requires steps "a - e" as described for the Dart-RNAseq analysis, but with different types of RNA-based adapter sequences and PCR primers. In particular, step "e" of the Dart-RNAseq analysis is substituted with two qPCR amplifications carried out in parallel, wherein the first qPCR is performed employing a pair of primers partially annealing on the defined RNA-based adapter sequence and partially on the 3'P RNA, and the second pair of primers anneals on the RNA-based adapter. Since at least one of the forward or reverse primers of the first qPCR are mapping at the junction between the RNA-based adapter and the 3'P RNA, the assay has a strong specificity for the 3' and 5' termini of the 3'P RNA, with a single nucleotide resolution on the 3' and 5' sequence of annealing. Following the qPCR amplification, some parameters of the 3'P RNA are evaluated and compared with some specific thresholds
in order to determine the diagnosis, prognosis, therapy monitoring or outcome prediction assessment of the disease or condition. The 3'P-qPCR assay has the advantages of being less time consuming and less expensive than other fluorescent and antibody-based methods, as well as sequencing methods. Moreover, the assay can be designed for high-throughput experiments on standard 96-well plates, by screening different samples against a specific 3'P RNA marker or by testing a panel of 3'P RNA markers against a specific sample.

The present invention discloses a method for identifying at least one RNA fragment comprising a 3' phosphate or 2'/3' cyclic phosphate (3'P RNA) as a molecular marker of a disease contained in a biological sample of a subject suffering from the disease, wherein the method comprises the following steps:

(a) phosphorylating the at least one 3'P RNA contained in the biological sample at the 5' end obtaining at least one phosphorylated RNA fragment (step 1. of figure 12);
(b) ligating the 3' end of the at least one phosphorylated RNA fragment to the 5' end of an RNA-based adapter obtaining at least one first ligation product, wherein the RNA-based adapter has formula (I) (step 2. of figure 12):

$$5'\ OH\text{-}N_x\text{-}C1\text{-}L1\text{-}A_z\text{-}PR1\text{-}N_y\text{-}C2\text{-}B\text{-}OH\ 3' \qquad (I)$$

wherein

- N is a ribonucleotide,
- x and y are integer numbers independently selected from 1 to 20,
- L1 is an oligoribonucleotide sequence having a length comprised between 15 and 30,

- A is an abasic site or a spacer allowing the arrest of a retrotranscriptase enzyme activity,
- z is an integer number from 1 to 5,
- PR1 is a first portion of a first nucleic acid domain of a sequencing platform adapter construct having a length comprised between 10 and 80,
- B is none, or a ribonucleotide having a 2'-fluoro-base, or a ribonucleotide having a modified nucleobase conferring nuclease resistance, and
- C1 and C2 are none or a barcode sequence comprising up to 20 nucleotides, provided that at least one between C1 and C2 is none;

(c) self-ligating the at least one first ligation product to form at least one circular RNA molecule (step 3. of figure 12);
(d) performing a reverse transcription of the at least one circular RNA molecule obtaining at least one single strand cDNA molecule comprising the sequence of the at least one 3'P RNA (step 4. of figure 12), wherein the reverse transcription is carried out using a reverse transcription primer having formula (II):

5' OH-R2-$N_z$-D1-OH 3' (II)

wherein

- D1 is the reverse complement deoxyoligoribonucleotide of L1, wherein complementarity of D1 to L1 is comprised between 60% and 100%,
- N is a ribonucleotide,
- z is an integer number from 1 to 20, and
- R2 is a second nucleic acid domain of the sequencing platform adapter construct having a length comprised between 10 and 50;

(e) performing a PCR amplification of the at least one single strand cDNA molecule obtaining at least one amplification product, wherein the PCR amplification is carried out alternatively:

(i) in two sequential steps (step 5. and 6. of figure 12A), wherein:

- the first PCR amplification is carried out using a first pair of primers, the first forward primer and the first reverse primer having formula (III) and (IV), respectively:

5' OH-T1-T2-OH 3' (III)

5' OH-T3-OH 3' (IV)

wherein

❖ T1 is a second portion of the first nucleic acid domain of the sequencing platform adapter construct having a length comprised between 10 and 50,
❖ T2 is a first DNA oligonucleotide sequence having a length comprised between 10 and 30 annealing on at least one part of the first portion of the first nucleic acid domain of the sequencing platform adapter construct (PR1 of formula I), and
❖ T3 is a second DNA oligonucleotide sequence having a length comprised between 10 and 50 annealing on at least one part of the second nucleic acid domain of the sequencing platform adapter construct (R2 of formula II);

- the second PCR amplification is carried out using a second pair of primers, the second forward primer and the second reverse primer having formula (V) and (VI), respectively:

5' OH-Q1-Q2-Q3-OH 3' (V)

5' OH-Q4-Q5-Q6-OH 3' (VI)

wherein

❖ Q1 is a third nucleic acid domain of the sequencing platform adapter construct having a length

comprised between 10 and 50,

❖ Q2 is a fourth nucleic acid domain of the sequencing platform adapter construct having a length comprised between 6 and 20,

❖ Q3 is a third DNA oligonucleotide sequence having a length comprised between 10 and 50 annealing on at least one part of the first nucleic acid domain of the sequencing platform adapter construct (T1 + PR1 of formulas I and III),

❖ Q4 is a fifth nucleic acid domain of the sequencing platform adapter construct having a length comprised between 10 and 50,

❖ Q5 is a sixth nucleic acid domain of the sequencing platform adapter construct having a length comprised between 6 and 20,

❖ Q6 a fourth DNA oligonucleotide sequence having a length comprised between 10 and 50 annealing on at least one part of the second nucleic acid domain of the sequencing platform adapter construct (R2 of formula II);

or

(ii) in one single step (step 5. of figure 12B) using a third pair of primers, the third forward primer and the third reverse primer having formula (VII) and (VIII), respectively:

5’ OH-Q1-Q2-Q7-OH 3’ (VII)

5’ OH-Q4-Q5-Q6-OH 3’ (VIII)

wherein

❖ Q1, Q2, Q4, Q5 and Q6 have the meaning set forth above, and

❖ Q7 is a DNA oligonucleotide sequence having a length comprised between 10 and 50, comprising at the 5’ end a second portion of the first nucleic acid domain of the sequencing platform adapter construct (corresponding to T1 of formula (III)) and annealing at the 3’ end on at least one part of the first portion of the first nucleic acid domain of the sequencing platform adapter construct (PR1 of formula (I));

(f) sequencing the at least one amplification product obtaining the sequence of the at least one 3’P RNA comprised in the at least one single strand cDNA molecule;

(g) repeating steps (a) to (f) on at least one control sample, wherein the control sample is a biological sample of a healthy, treated or non-treated subject;

(h) calculating for the at least one 3’P RNA contained in the amplification products obtained from the biological sample and the control sample: (i) a number of counts, (ii) a Dart-RNAseq p-value, (iii) a cleavage pattern and (iv) a Dart-RNAseq fold change of a normalized parameter based on the number of counts in the biological sample *versus* the control sample according to the following equation:

$$\text{Dart-RNAseq fold change} = \left(\frac{nCounts_{Treat}}{nCounts_{CTRL}}\right)$$

wherein

- $nCounts_{Treat}$ is the normalized parameter based on the number of counts for the 3’P RNA determined in the biological sample,
- $nCounts_{CTRL}$ is the normalized parameter based on the number of counts for the 3’P RNA determined in the control sample;

wherein the at least one 3’P RNA is the at least one molecular marker of the disease if:

- the number of counts is > 200,
- the Dart RNAseq p-value is ≤ 0.05,
- the cleavage pattern is ≤ 40% per-base cleavage frequencies along the 3’P RNA length and ≥ 60% per-base cleavage frequencies on the 5’ and 3’ ends of the 3’P RNA, and
- the Dart RNAseq fold change is ≥ 2 or ≤ 0.5.

Step (h) further comprises at least one of the following operations:

- mapping the sequence of the at least one 3'P RNA contained in the amplification product obtained for the biological sample and the control sample on the reference genome or transcriptome and calculating the multimapping score of the at least one 3'P RNA,
- calculating the length of the at least one 3'P RNA, and
- calculating a normalized counts based on sequencing depth of the at least one 3'P RNA,

wherein the at least one 3'P RNA is the at least one molecular marker of the disease if:

- the length is > 15 and < 200 nucleotides, or
- the normalized counts based on sequencing depth is > 5, or
- the multimapping score is ≤ 100.

The amplification product obtained at the end of phase (e) contains at least one DNA molecule having a composition as shown in figure 12A or figure 12B. The sense strand of the DNA molecule comprises from the 5' end to the 3' end the following elements: the third (Q1), the fourth (Q2), the first (T1 +PR1) nucleic acid domains of the sequencing platform adapter construct (the first nucleic acid domain being given by the combination of its first PR1 and second T1 portions), y deoxyribonucleotides ($N_y$), a barcode sequence or none (C2), none or a deoxyribonucleotide (B), the sequence of the 3'P RNA, x deoxyribonucleotides ($N_x$), none or a barcode sequence (C1), a deoxyribonucleotide (D1), z deoxyribonucleotides ($N_z$), the second (R2), the sixth (Q5) and the fifth (Q4) nucleic acid domains of the sequencing platform adapter construct.

The sequencing platform is selected from those commercialized by Illumina (e.g., the HiSeg™, MiSeg™ and NovaSeq™ sequencing systems); Element Bioscience (e.g., LoopSeq for AVITI™ sequencing systems); Singular genomics (e.g., the G4 system); Life Technologies (e.g., a SOLD sequencing system); Roche (e.g., the 454 GS FLX+ and/or GS Junior sequencing systems); MGI (e.g., E25, G400, G99, G50 and T7, T10, T20 systems). Preferably the sequencing platform is selected from those commercialized by Illumina.

The combination of the third (Q1), the fourth (Q2), the first (T1 + PR1) nucleic acid domains of the sequencing platform adapter construct has a sequence selected from: (i) P1 adaptor by Life Technologies (as reported in "Applied Biosystems SOLiD™ 4 System Library Preparation Guide" April 2010, https://tools.thermofisher.com/content/sfs/manuals/SOLiD4_LibraryPreparation_ man.pdf), (ii) GS adaptor A by Roche (as reported in "GS FLX Titanium General Library Preparation Method Manual", April 2009, USM-00048.B, https://dna.uga.edu/wp-content/uploads/sites/51/2013/ 12/GS-FLX-Titanium-General-Library-Preparation-Method-Manual-Roche.pdf), and (iii) MGI 5' adapter by MGI (as reported in "MGIEasy RNA Directional Library Prep Set User Manual", Cat. No.: 1000006385 (16 RXN) , 1000006386 (96 RXN), Kit Version: V2.1, Manual Version: 5.0).

The combination of the second (R2), the sixth (Q5) and the fifth (Q4) nucleic acid domains of the sequencing platform adapter construct has a sequence selected from : (i) P2 adaptor by Life Technologies (as reported in "Applied Biosystems SOLiID™ 4 System Library Preparation Guide" April 2010, https://tools.thermofisher.com/content/sfs/manuals/SOLiD4_LibraryPreparation_ man.pdf), (ii) GS adaptor B by Roche (as reported in "GS FLX Titanium General Library Preparation Method Manual", April 2009, USM-00048.B, https://dna.uga.edu/wp-content/uploads/sites/51/2013/12/ GS-FLX-Titanium-General-Library-Preparation-Method-Manual-Roche.pdf), and (iii) MGI 3' adapter by MGI (as reported in "MGIEasy RNA Directional Library Prep Set User Manual", Cat. No.: 1000006385 (16 RXN) , 1000006386 (96 RXN), Kit Version: V2.1, Manual Version: 5.0).

It is indeed evident that the expert in the field knows how to "cut and sew" the sequences of the sequencing platform adapter constructs employed by the commercially available sequencing platforms in an appropriate manner to generate the sequences of the RNA-based adapter, reverse transcription primer and PCR primers (and consequently of the different nucleic acid domains as identified above) so that the DNA molecule obtained at the end of step (e) has a sequence as shown in figure 12 and can be sequenced by the sequencing platform that the expert has selected from those available.

When the sequencing (step f) is carried out on a sequencing platform by Illumina, then:

- the sequence of first nucleic acid domain PR1 + T1 of the sequencing platform adapter construct is selected from the sequences SP1;
- the second nucleic acid domain R2 of the sequencing platform adapter construct is selected from the sequences SP2;
- the third nucleic acid domain Q1 of the sequencing platform adapter construct is the sequence P5;
- the fourth nucleic acid domain Q2 of the sequencing platform adapter construct is selected from the sequences i5 (or index5);
- the fifth nucleic acid domain Q4 of the sequencing platform adapter construct is the sequence P7;
- the sixth nucleic acid domain Q5 of the sequencing platform adapter construct is selected from the sequences i7 (or index7).

The sequences SP1, SP2, i5, i7, P5 and P7 are part of the common general knowledge of the skilled man and are fully disclosed in "Illumina Adapter Sequences" Document # 1000000002694 v11, May 2019; https://www.science.smith.edu/cmbs/wp-content/uploads/sites/36/2020/01/illumina-adapter-sequences-1000000002694-11.pdf.

When the sequencing (step f) is carried out on a sequencing platform by Element Bioscience, then:

- the sequence of first nucleic acid domain PR1 + T1 of the sequencing platform adapter construct is selected from the sequences read primer 1;
- the second nucleic acid domain R2 of the sequencing platform adapter construct is selected from the sequences read primer 2;
- the third nucleic acid domain Q1 of the sequencing platform adapter construct is the sequence outer adapter;
- the fourth nucleic acid domain Q2 of the sequencing platform adapter construct is selected from the sequences index 2;
- the fifth nucleic acid domain Q4 of the sequencing platform adapter construct is the sequence outer adapter;
- the sixth nucleic acid domain Q5 of the sequencing platform adapter construct is selected from the sequences adapter.

The sequences outer adapter, index 2, read primer 1, read primer 2, adapter and outer adapter are part of the common general knowledge of the skilled man and are fully disclosed in "Amplicon LoopSeq™ for AVITI™" Document # MA-00023 Rev. C June 2023, go.elementbiosciences.com/amplicon-loopseq-aviti-workflow-guide" and "Element Elevate™ Library Prep" Document # MA-00004 Rev. B, June 2023, go.elementbiosciences.com/elevate-library-prep-workflow-guide.

When the sequencing (step f) is carried out on a sequencing platform by Singular Genomics, then:

- the sequence of first nucleic acid domain PR1 + T1 of the sequencing platform adapter construct is selected from the sequences SP1;
- the second nucleic acid domain R2 of the sequencing platform adapter construct is selected from the sequences SP2;
- the third nucleic acid domain Q1 of the sequencing platform adapter construct is the sequence S1;
- the fourth nucleic acid domain Q2 of the sequencing platform adapter construct is selected from the sequences index 1;
- the fifth nucleic acid domain Q4 of the sequencing platform adapter construct is the S2;
- the sixth nucleic acid domain Q5 of the sequencing platform adapter construct is selected from the sequences index 2.

The sequences S1, S2, SP1, SP2 index 1 and index 2 are part of the common general knowledge of the skilled man and are fully disclosed in "ADAPTING LIBRARIES FOR THE G4™ - INSERT ONLY" Document #600024 Rev. 0, May 2023 https://singulargenomics.com/wp-content/uploads/2023/06/Adapting-Library-Insert-600024.pdf).

The first and second DNA oligonucleotide sequences T2 and T3 anneal on at least 6 nucleotides of the first portion (PR1) of the first nucleic acid domain and second nucleic acid domain (R2) of the sequencing platform adapter construct, respectively.

The third and fourth DNA oligonucleotide sequences Q3 and Q6 anneal on at least 6 nucleotides of the second portion (T1) of the first nucleic acid domain and the second nucleic acid domain (R2) of the sequencing platform adapter construct, respectively.

Before performing step (a) the biological sample and/or the control sample are subjected to a small RNA enrichment operation, meaning that all the RNA fragments smaller than 200 nt (preferably between 10 and 200 nt) are size-selected with any method known by a skilled man (e.g. silica column-based methods).

The biological sample is selected from urine, whole blood, saliva, plasma, skin, fibroblasts, neurons, liver, muscle, primary and immortalized cell line, Induced Pluripotent Stem Cells (iPSC), non-human embryonic stem cells (ESC).

The phosphorylation step (a) is carried out using a phosphorylating enzyme selected from T4 PNK 3' minus, T4 PNK and recombinant versions of T4 PNK (e.g. Optikinase™).

The ligation step (b) is carried out using a first ligase enzyme selected from RtcB, Archease, Arabidopsis Thaliana tRNA ligase, and eukaryotic tRNA ligase.

The self-ligation step (c) is carried out using a second ligase enzyme selected from T4 Rnl1, T4 Rnl2, T4 Rnl2tr, T4 Rnl2 K227Q, Mth Rnl, and ATP-independent ligases that catalyze intramolecular ligation (e.g. CircLigase™, CircLigaseII™).

The reverse transcription step (d) is carried out using a reverse transcriptase (RT) enzyme selected from engineered M MLV-RT enzymes (Moloney Murine Leukemia Virus Reverse Transcriptase) and AMV-RT enzymes (Avian Myeoloblastosis Virus Reverse Transcriptase), preferably selected from Maxima H Minus™, Superscript™ I-II-III-IV, Sunscript™.

The PCR amplification step (e) is carried out using a DNA polymerase enzyme selected from engineered Taq DNA polymerase, preferably with high fidelity activity (e.g. Q5® High-Fidelity DNA Polymerase, Platinum® Taq, KAPA HiFi HotStart).

The Dart-RNAseq analysis method disclosed above allowed the identification of some molecular markers of Spinal Muscular Atrophy and cutaneous Squamous Cell Carcinoma; the 3'P RNA markers of Spinal Muscular Atrophy have the sequences as set forth in SEQ ID NO.: 1 to 83; the 3'P RNA markers of cutaneous Squamous Cell Carcinoma have the sequences as set forth in SEQ ID NO.: 84 to 172.

The present invention also discloses a kit suitable for implementing the Dart-RNAseq analysis method described above for identification of at least one 3'P RNA as a molecular marker of a disease, the kit comprising:

(a) at least one RNA-based adapter having formula (I):

$$5' \text{ OH-}N_x\text{-C1-L1-}A_z\text{-PR1-}N_y\text{-C2-B-OH } 3' \qquad (I)$$

wherein

- N is a ribonucleotide,
- x and y are integer numbers independently selected from 1 to 20,
- L1 is an oligoribonucleotide sequence having a length comprised between 15 and 30,
- A is an abasic site or a spacer allowing the arrest of a retrotranscriptase enzyme activity,
- z is an integer number from 1 to 5,
- PR1 is a part of a first nucleic acid domain of a sequencing platform adapter construct having a length comprised between 10 and 80,
- B is a none, a ribonucleotide having a 2'-fluoro-base, or a ribonucleotide having a modified nucleobase conferring nuclease resistance, and
- C1 and C2 are none or a barcode sequence comprising up to 20 nucleotides, provided that at least one between C1 and C2 is none;

(b) a reverse transcription primer having formula (II):

$$5' \text{ OH-R2-}N_z\text{-D1-OH } 3' \qquad (II)$$

wherein

- D1 is the reverse complement deoxyoligoribonucleotide of L1, wherein complementarity of D1 to L1 is comprised between 60% and 100%, and
- R2 is a second nucleic acid domain of the sequencing platform adapter construct having a length comprised between 10 and 50,
- N is a ribonucleotide,
- z is integer numbers independently selected from 1 to 20; and alternatively,

(c) a first and a second pair of primers, wherein

- the first pair of primers comprises a first forward primer and a first reverse primer having formula (III) and (IV), respectively:

  5' OH-T1-T2-OH 3' (III)

  5' OH-T3-OH 3' (IV)

  wherein

  ❖ T1 is a second portion of the first nucleic acid domain of the sequencing platform adapter construct having a length comprised between 10 and 50,
  ❖ T2 is a first DNA oligonucleotide sequence having a length comprised between 10 and 30 annealing on at least one part of the first portion (PR1) of the first nucleic acid domain of the sequencing platform adapter construct, and
  ❖ T3 is a second DNA oligonucleotide sequence having a length comprised between 10 and 50 annealing on

at least one part of the second (R2) nucleic acid domain of the sequencing platform adapter construct;

- the second pair of primers comprises a second forward primer and a second reverse primer having formula (V) and (VI), respectively:

5' OH-Q1-Q2-Q3-OH 3' (V)

5' OH-Q4-Q5-Q6-OH 3' (VI)

wherein

❖ Q1 is a third nucleic acid domain of the sequencing platform adapter construct having a length comprised between 10 and 50,
❖ Q2 is a fourth nucleic acid domain of the sequencing platform adapter construct having a length comprised between 6 and 20,
❖ Q3 is a third DNA oligonucleotide sequence having a length comprised between 10 and 50 annealing on at least one part of the first nucleic acid domain (T1 + PR1) of the sequencing platform adapter construct,
❖ Q4 is fifth nucleic acid domain of the sequencing platform adapter construct having a length comprised between 10 and 50,
❖ Q5 is a sixth nucleic acid domain of the sequencing platform adapter construct having a length comprised between 6 and 20,
❖ Q6 a fourth DNA oligonucleotide sequence having a length comprised between 10 and 50 annealing on at least one part of the second nucleic acid domain (R2) of the sequencing platform adapter construct;

or
(d) at least one pair of primers, the forward primer and the reverse primer having formula (VII) and (VIII), respectively:

5' OH-Q1-Q2-Q7-OH 3' (VII)

5' OH-Q4-Q5-Q6-OH 3' (VIII)

wherein

❖ Q1, Q2, Q4, Q5 and Q6 have the meaning set forth above, and
❖ Q7 is a DNA oligonucleotide sequence having a length comprised between 10 and 50, comprising at the 5' end a second portion of the first nucleic acid domain of the sequencing platform adapter construct (T1) and annealing at the 3' end on at least one part of the first portion of the first nucleic acid domain of the sequencing platform adapter construct (PR1 of formula (I)).

The kit comprises:

(a) at least one RNA-based adapter having a sequence selected from the sequences set forth in SEQ ID No.: 173-177;
(b) a reverse transcription primer having a sequence set forth in SEQ ID No.: 178;
and alternatively,
(c) one first pair of primers comprising a first forward and a first reverse primer having a sequence as set forth in SEQ ID No.: 179 and 180, respectively, and at least one second pair of primers comprising a second forward and a second reverse primer having formula (IX) and (X), respectively:

5' OH-AATGATACGGCGACCACCGAGATCTACAC**(i5)**ACACTCTTTCCCTACACGACGCTCTTCCGATCT-OH 3' (IX)

5' OH-CAAGCAGAAGACGGCATACGAGAT**(i7)**GTGACTGGAGTTCAGACGTGTGCTCTTCCGATCT-OH 3' (X)

wherein

(i5) is selected from the sequences i5 (or index5) by Illumina, and
(i7) is selected from the sequences i7 (or index7) by Illumina;
or

(d) at least one pair of primers comprising a forward and a reverse primer having formula (IX) and (X) as set forth above.

The kit further comprises at least one of:

(e) Reagents for RNA extraction and isolation from the biological sample. They include a lysis buffer, silica-based spin columns, and collection tubes to efficiently extract RNA smaller than 200 nt while minimizing contamination. Example of a lysis buffer is: Tris-HCl (pH 7.5): 10 mM; EDTA (pH 8.0): from 0 mM to 1 mM; Sodium Chloride (NaCl): from 10 mM to 0.5 M; Sodium Dodecyl Sulfate (SDS): 0.5%; Sodium Dodecyl Cholate (SDC): 0% to 0.5%; proteinase K: from 0 μg/ml to 100 μg/ml; HEPES: from 0 nM to 100 mM.
(f) A PNK enzyme (for the phosphorylation of the 5' end of the 3'P RNA) and optionally a PNK solution and a buffer).
(g) A first ligase enzyme (e.g. RtcB) (needed to perform the first ligation reaction, and optionally a ligation solution and a buffer).
(h) A second ligase enzyme (e.g. T4 RNA ligase) (for intramolecular circularization of the first ligation product, and optionally a ligation solution and a buffer).
(i) A reverse transcriptase (RT) enzyme, and optionally nucleotides (dNTPs), solutions and buffers.
Examples of RT enzymes usable with the present kit are:

- the Moloney Murine Leukemia Virus Reverse Transcriptase (M-MLV RT) (this enzyme is a DNA polymerase enzyme that catalyzes the synthesis of complementary DNA (cDNA) from an RNA template during the process of reverse transcription. It possesses both RNA-dependent DNA polymerase activity and RNase H activity);
- the Avian Myeloblastosis Virus Reverse Transcriptase (AMV RT) (this enzyme is another RNA-dependent DNA polymerase derived from the avian myeloblastosis virus);
- the HIV-1 Reverse Transcriptase (this enzyme is derived from the human immunodeficiency virus type 1);
- the Thermoscript Reverse Transcriptase (this enzyme is a modified version of M-MLV RT that exhibits enhanced stability and activity at higher temperatures);
- the SuperScript Reverse Transcriptase (this enzyme is a proprietary enzyme developed by Thermo Fisher Scientific. It is available in several variations, including SuperScript II and SuperScript III. These enzymes are engineered for enhanced thermostability, increased cDNA yield, and reduced RNase H activity);
- the Transcriptor Reverse Transcriptase (this enzyme is a reverse transcriptase enzyme from Roche Diagnostics. It exhibits high thermostability, making it suitable for reverse transcription reactions performed at elevated temperatures).

(j) A PCR Master Mix: a ready-to-use mixture that contains all the components necessary for PCR amplification. It includes a Taq DNA polymerase, dNTPs and reaction buffers. The PCR Master Mix may also contain stabilizers, enhancers, and other additives to improve PCR performance.

- Taq DNA Polymerase: Platinum Taq DNA Polymerase, AccuPrime Taq DNA Polymerase, GoTaq DNA Polymerase, GoTaq Green and GoTaq Flexi DNA Polymerases, KAPA Taq DNA Polymerase, Phusion Taq DNA Polymerase, Q5 High-Fidelity DNA Polymerase. Usually a hot-start DNA polymerase that remains inactive during the reaction setup and is activated during the initial denaturation step.
- dNTPs (Deoxynucleotide triphosphates): A mix of all four nucleotides (dATP, dCTP, dGTP, and dTTP) at a concentration of around 200-400 μM each.
- Buffer: A reaction buffer optimized for the specific DNA polymerase used. It typically contains salts and stabilizers to maintain optimal enzyme activity and reaction conditions.
- $MgCl_2$: Magnesium chloride at a concentration of about 1.5-5 mM, which serves as a cofactor for the DNA polymerase enzyme.
- Primers: Specific forward and reverse primers targeting the DNA region of interest. The concentration of each primer can vary but is typically in the range of 200-900 nM.
- Stabilizers and enhancers: Various additives, such as BSA (bovine serum albumin) or glycerol, may be included to improve the stability and performance of the qPCR reaction.

(k) Nuclease-free Water. This is a molecular-grade water that is free from nucleases, which could degrade the RNA or interfere with the reaction. It is used for reconstituting lyophilized components, diluting samples, and preparing control reactions.
(l) Reaction Tubes and Plates. These tubes or plates are designed to provide optimal thermal conductivity and compatibility with the qPCR instrument being used.
(m) User Manual and Protocols. It includes step-by-step protocols, guidelines for optimizing reaction conditions, and recommendations for data analysis.

In one embodiment, the present description concerns an *in vitro* or *ex vivo* method of diagnosis, prognosis, therapy monitoring or outcome prediction assessment of a disease or condition in a subject by detecting and quantifying at least one molecular marker of the disease or condition contained in a biological sample of the subject (see figure 13 showing a schematic representation of the method), wherein the at least one molecular marker is an RNA fragment comprising a 3' phosphate or a 2'/3' cyclic phosphate (3'P RNA), the method comprising the following steps:

(a') phosphorylating the 5' end of the at least one 3'P RNA contained in the biological sample and obtaining at least one phosphorylated RNA fragment (step 1. of figure 13);
(b') ligating the 3' end of the at least one phosphorylated RNA fragment to the 5' end of an RNA-based adapter obtaining at least one first ligation product (step 2. of figure 13), wherein the RNA-based adapter has formula (Ia):

$$5' \text{ OH-E1-}A_z\text{-E2-OH } 3' \qquad \text{(Ia)}$$

wherein

- E1 is a first oligoribonucleotide sequence having a length comprised between 15 and 30,
- A is an abasic site or a spacer allowing the arrest of a retrotranscriptase enzyme activity,
- z is an integer number from 1 to 5,
- E2 is a second oligoribonucleotide sequence having a length comprised between 15 and 30;

(c') self-ligating the at least one first ligation product to form at least one circular RNA molecule (step 3. of figure 13);
(d') performing a reverse transcription of the at least one circular RNA molecule obtaining at least one single strand cDNA molecule comprising the sequence of the at least one 3'P RNA (step 4. of figure 13), wherein the reverse transcription is carried out using a reverse transcription primer having formula (IIa):

5' OH-G-F1-OH 3' (IIa)

wherein

- F1 is the reverse complement deoxyoligoribonucleotide of E1, wherein complementarity of F1 to E1 is comprised between 60% and 100%, and
- G is a first DNA oligonucleotide having a length comprised between 10 and 30;

(e') performing in parallel a first and a second qPCR amplifications of the at least one single strand cDNA molecule obtaining a first and a second amplification product (step 5. of figure 13), wherein:

- the first qPCR amplification is carried out using a first pair of primers annealing on at least one part of the 3'P RNA sequence, the first pair of primers being selected from pairs of primer Set1, Set2 and Set3, wherein each pair of primers comprises a forward and reverse primer, wherein the forward and reverse primers of the pair of primers Set1 have a sequence as set forth in formulas (IIIa) and (IVa), respectively, the forward and reverse primers of the pair of primers Set2 have a sequence as set forth in formulas (IIIa') and (IVa'), respectively, and the forward and reverse primers of the pair of primers Set3 have a sequence as set forth in formulas (IIIa") and (IVa"), respectively:

Set1

5' OH-H2-N-OH 3' (IIIa)

5' OH-H1-M-OH 3' (IVa)

Set2

5’ OH-H2-N-OH 3’ (IIIa’)

5’ OH-H1-OH 3’ (IVa’)

Set3

5’ OH-H2-OH 3’ (IIIa")

5’ OH-H1-M-OH 3’ (IVa")

wherein

- ❖ H1 is a second DNA oligonucleotide annealing on E1, wherein complementarity of H1 to E1 is comprised between 30% and 100%, and
- ❖ M is a third DNA oligonucleotide having a length comprised between 6 and 30 and annealing on at least 6 nucleotides of the 3’ end of the 3’P RNA sequence,
- ❖ H2 is a fourth DNA oligonucleotide annealing on E2, wherein complementarity of H2 to E2 is comprised between 30% and 100%, and
- ❖ N is a fifth DNA oligonucleotide having a length comprised between 6 and 30 and annealing on at least 6 nucleotides of the 5’ end of the 3’P RNA sequence;

- the second qPCR amplification is carried out using a second pair of primers annealing on the RNA-based adapter sequence, the second pair of primers comprising a second forward and a second reverse primer having formula (Va) and (VIa), respectively:

5’ OH-H2-OH 3’ (Va)

5’ OH-I1-OH 3’ (VIa)

wherein

- ❖ H2 has the meaning set forth above, and
- ❖ I1 is a sixth DNA oligonucleotide annealing on G, wherein complementarity of I1 to G is comprised between 60% and 100%; and

(f) repeating steps (a’) to (e’) on at least one control sample, wherein the control sample is a biological sample of a healthy, treated or non-treated subject;
(g’) determining Ct values for the at least one 3’P RNA and for the RNA-based adapter in the first and second amplification products for either the biological sample and the control sample,
(h’) calculating a 3’P-qPCR fold change of the Ct values determined in step (g’) according to the following equation:

$$3'\text{P qPCR fold change} = \frac{2^{Ct(B)_{3'P}-Ct(A)_{3'P}}}{2^{Ct(B)_{adp}-Ct(A)_{adp}}} = \frac{2^{\Delta Ct(3'P)}}{2^{\Delta Ct(adp)}} = 2^{\Delta\Delta Ct}$$

wherein

- $Ct(A)_{3'P}$ is the Ct value for the 3’P RNA determined in the biological sample,
- $Ct(B)_{3'P}$ is the Ct value for the 3’P RNA determined in the control sample,
- $Ct(A)_{adp}$ is the Ct value for the RNA-based adapter determined in the biological sample,
- $Ct(B)_{adp}$ is the Ct value for the RNA-based adapter determined in the control sample;

wherein a 3’P-qPCR fold change value ≥ 2 or ≤ 0.5 is indicative of the diagnosis, prognosis, therapy monitoring or outcome prediction assessment of the disease or condition.

In one embodiment, the method further comprises in step (h’) calculating a 3’P-qPCR p-value of the 3’P-qPCR fold change, wherein a 3’P-qPCR p-value < 0.5 is indicative of the diagnosis, prognosis, therapy monitoring or outcome

prediction assessment of the disease or condition.

In one embodiment, the RNA-based adapter of formula (Ia) has a length comprised between 50 and 100 nt.

In one embodiment, the spacer A contained in the RNA-based adapter of formula (Ia) is selected from 1,2'-dideoxyribose modification (dSpacer), tetrahydrofuran (THF), apurinic/apyrimidinic (AP) site or a biotinylated blocking spacer.

In one embodiment, the reverse transcription primer of formula (IIa) has a length comprised between 10 and 100 nt.

In one embodiment, the primers (both the first and the second primer pairs) employed in the two qPCR amplifications have at least one of the following features:

- they contain from 1 to 20 modified nucleotides; preferably the modification encompasses one of a phosphorothioate modification, a locked nucleic acid (LNA), a 2'-O-Methyl modification, a 5-methylcytosine modification, a minor groove binder, a spacer molecule, PNA. These modifications can be used individually or in combination to optimize primer performance, including primer specificity, stability, and melting temperature.
- they have a length comprised between 15 and 25 nucleotides;
- they have a minimum free energy comprised between -3 and -150 kcal/mol;
- they do not contain strong or mild secondary structures and/or do not form dimers;
- they have a melting temperature comprised between 57 and 70 °C;
- they have a melting temperature difference between the forward and reverse primer of the pair less than or equal to 3 °C.

In one embodiment, at least one of the forward and reverse primers of the first pair of primers used in the first qPCR amplification anneal on at least 6, preferably 10, nucleotides of the 3'P RNA at the 3' end and the 5' end, respectively.

In one embodiment, the phosphorylation step (a') is carried out using a phosphorylating enzyme selected from T4 PNK 3' minus, T4 PNK and recombinant versions of T4 PNK (e.g. Optikinase™).

In one embodiment, the ligation step (b') is carried out using a first ligase enzyme selected from RtcB, Archease, Arabidopsis Thaliana tRNA ligase, and eukaryotic tRNA ligase.

In one embodiment, the self-ligation step (c') is carried out using a second ligase enzyme selected from T4 Rnl1, T4 Rnl2, T4 Rnl2tr, T4 Rnl2 K227Q, Mth Rnl, and ATP-independent ligases that catalyze intramolecular ligation (e.g., CircLligase™, CircLligaseII™).

In one embodiment, the reverse transcription step (d') is carried out using a reverse transcriptase (RT) enzyme selected from engineered M MLV-RT enzymes (Moloney Murine Leukemia Virus Reverse Transcriptase) and AMV-RT enzymes (Avian Myeoloblastosis Vvirus Reverse Transcriptase), preferably selected from Maxima H Mminus™, SuperscriptTM I-II-III-IV, Sunscript™.

In one embodiment, the qPCR amplifications step (e') is carried out using a DNA polymerase enzyme selected from engineered Taq DNA polymerase enzymes, which preferably remain inactive during the reaction setup and are activated during the initial denaturation step.

Examples of Taq DNA Polymerase enzymes usable in the 3'P-qPCR assay are: Platinum Taq DNA Polymerase, AccuPrime Taq DNA Polymerase, GoTaq DNA Polymerase, GoTaq Green and GoTaq Flexi DNA Polymerases, KAPA Taq DNA Polymerase, Phusion Taq DNA Polymerase, Q5 High-Fidelity DNA Polymerase.

In one embodiment, the disease is Spinal Muscular Atrophy (SMA) or cutaneous Squamous Cell Carcinoma (cSCC).

In one embodiment, the at least one molecular marker of Spinal Muscular Atrophy (SMA) is selected from 3'P RNAs having a sequence as set forth in SEQ ID No.: 1 - 83.

In one embodiment, the at least one molecular marker of cutaneous Squamous Cell Carcinoma (cSCC) is selected from 3'P RNAs having a sequence as set forth in SEQ ID No.: 84 - 172.

In one embodiment, the present description concerns kits for the diagnosis, prognosis, therapy monitoring or outcome prediction assessment of Spinal Muscular Atrophy (SMA) and cutaneous Squamous Cell Carcinoma (cSCC).

In one embodiment, the kit for the diagnosis, prognosis, therapy monitoring or outcome prediction assessment of the Spinal Muscular Atrophy (SMA) comprises:

(a') an RNA-based adapter having a sequence as set forth in SEQ ID No.: 185;

(b') a reverse transcription primer having a sequence as set forth in SEQ ID No.: 186;

(c') seven first pairs of primers for performing a first qPCR amplification, the first forward and reverse primers of the seven primers pairs having a sequence as set forth in the following sequence pairs: SEQ ID No.: 187 and 188, SEQ ID No.: 187 and 189, SEQ ID No.: 190 and 191, SEQ ID No.: 192 and 193, and SEQ ID No.: 198 and 199, SEQ ID No.: 200 and 201, SEQ ID No.: 202 and 203;

(d') a second pair of primers for performing a second qPCR amplification, the second forward and second reverse

primers having a sequence as set forth in SEQ ID No.: 204 and 205, respectively.

In one embodiment, the kit for the diagnosis, prognosis, therapy monitoring or outcome prediction assessment of cutaneous Squamous Cell Carcinoma (cSCC) comprises:

(a') an RNA-based adapter having a sequence as set forth in SEQ ID No.: 185,
(b') a reverse transcription primer having a sequence as set forth in SEQ ID No.: 186,
(c') two first pairs of primers for performing a first qPCR amplification, the first forward and reverse primers of the two primers pairs having respectively a sequence as set forth in the following sequence pairs: SEQ ID No.: 194 and 195, SEQ ID No.: 196 and 197
(d') a second pair of primers for performing a second qPCR amplification, the second forward and second reverse primers having a sequence as set forth in SEQ ID No.: 204 and 205, respectively.

In one embodiment, the kits further comprise at least one of:

(e') Reagents for RNA extraction and isolation from the biological sample. They include a lysis buffer, silica-based spin columns, and collection tubes to efficiently extract RNA smaller than 200 nt while minimizing contamination. Example of a lysis buffer is: Tris-HCl (pH 7.5): 10 mM; EDTA (pH 8.0): from 0 mM to 1 mM; Sodium Chloride (NaCl): from 10 mM to 0.5 M; Sodium Dodecyl Sulfate (SDS): 0.5%; Sodium Dodecyl Cholate (SDC): 0% to 0.5%; proteinase K: from 0 μg/ml to 100 μg/ml; HEPES: from 0 nM to 100 mM.
(f') A PNK enzyme (for the phosphorylation of the 5' end of the 3'P RNA), and optionally a PNK solution and a buffer.
(g') A first ligase enzyme (e.g. RtcB) (needed to perform the first ligation reaction), and optionally a ligation solution and a buffer.
(h') A second ligase enzyme (e.g. T4 RNA ligase) (for intramolecular circularization of the first ligation product), and optionally a ligation solution and a buffer.
(i') A reverse transcriptase (RT) enzyme, and optionally nucleotides (dNTPs), solutions and buffers.
Examples of RT enzymes usable with the present kit are:

- the Moloney Murine Leukemia Virus Reverse Transcriptase (M-MLV RT) (this enzyme is a DNA polymerase enzyme that catalyzes the synthesis of complementary DNA (cDNA) from an RNA template during the process of reverse transcription. It possesses both RNA-dependent DNA polymerase activity and RNase H activity);
- the Avian Myeloblastosis Virus Reverse Transcriptase (AMV RT) (this enzyme is another RNA-dependent DNA polymerase derived from the avian myeloblastosis virus);
- the HIV-1 Reverse Transcriptase (this enzyme is derived from the human immunodeficiency virus type 1);
- the Thermoscript Reverse Transcriptase (this enzyme is a modified version of M-MLV RT that exhibits enhanced stability and activity at higher temperatures);
- the SuperScript Reverse Transcriptase (this enzyme is a proprietary enzyme developed by Thermo Fisher Scientific. It is available in several variations, including SuperScript II and SuperScript III. These enzymes are engineered for enhanced thermostability, increased cDNA yield, and reduced RNase H activity);
- the Transcriptor Reverse Transcriptase (this enzyme is a reverse transcriptase enzyme from Roche Diagnostics. It exhibits high thermostability, making it suitable for reverse transcription reactions performed at elevated temperatures).

(j') qPCR Master Mix: a ready-to-use mixture that contains all the components necessary for PCR amplification. It includes a Taq DNA polymerase, dNTPs, reaction buffers, and fluorescent dyes or probes for real-time detection of amplification. The qPCR Master Mix may also contain stabilizers, enhancers, and other additives to improve PCR performance.
A qPCR master mix can thus contain:

- A Taq DNA Polymerase selected from: Platinum Taq DNA Polymerase, AccuPrime Taq DNA Polymerase, GoTaq DNA Polymerase, GoTaq Green and GoTaq Flexi DNA Polymerases,KAPA Taq DNA Polymerase, Phusion Taq DNA Polymerase, Q5 High-Fidelity DNA Polymerase. Usually a hot-start DNA polymerase that remains inactive during the reaction setup and is activated during the initial denaturation step.
- dNTPs (Deoxynucleotide triphosphates): A mix of all four nucleotides (dATP, dCTP, dGTP, and dTTP) at a concentration of around 200-400 μM each.
- A buffer: a reaction buffer optimized for the specific DNA polymerase used. It typically contains salts and stabilizers to maintain optimal enzyme activity and reaction conditions.
- $MgCl_2$: Magnesium chloride at a concentration of about 1.5-5 mM, which serves as a cofactor for the DNA

polymerase enzyme.

- Primers: Specific forward and reverse primers targeting the DNA region of interest. The concentration of each primer can vary but is typically in the range of 200-900 nM.
- Stabilizers and enhancers: Various additives, such as BSA (bovine serum albumin) or glycerol, may be included to improve the stability and performance of the qPCR reaction.

(k') Positive and Negative Controls to validate the RT-qPCR assay. Positive controls contain known quantities of the target 3'P RNA, allowing for the determination of assay sensitivity and the establishment of a standard curve. Negative controls verify the absence of contamination or nonspecific amplification.
(l') Nuclease-free Water. This is a molecular-grade water that is free from nucleases, which could degrade the RNA or interfere with the reaction. It is used for reconstituting lyophilized components, diluting samples, and preparing control reactions.
(m') Reaction Tubes and Plates. These tubes or plates are designed to provide optimal thermal conductivity and compatibility with the qPCR instrument being used.
(n') User Manual and Protocols. It includes step-by-step protocols, guidelines for optimizing reaction conditions, and recommendations for data analysis.

In the following, a description of a protocol for carrying out the Dart-RNAseq analysis method applied to the Illumina sequencing platform and the 3'P-qPCR assay are provided.

***Schematic description of Dart-RNAseq analysis***

*Small RNA enrichment.*

Before starting with Dart-RNA seq analysis, small RNA (smRNA) enrichment is performed using column-based or beads-based approach. Cell lines, flash frozen tissues and whole blood can be used as source for Dart-RNA seq analysis.
For smRNA enrichment, several commercial kits can be used, such as mirVana (ThermoFisher), miRNeasy (Qiagen), RNA Clean and Concentrator (Zymo), Agencourt beads (Beckman).

*Step (a). Phosphorylation.*

Upon small RNA enrichment (<200 nt), 3'P RNA will be subjected to 5' phosphorylation by T4 Polynucleotide kinase (T4 PNK 3' Minus), according to the protocol indicated in Table 1.

**Table 1**

| Component | Preferred Amount | amount |
|---|---|---|
| 10x Buffer | 1X | 1X-1.5x |
| 10mM ATP | 1 mM | From 1 mM to 10 mM |
| 10 U/ μL T4 PNK 3' minus | 10U | From 1U to 20 U |
| smRNAs | 2 μg | 0.05 - 5 μg |
| $H_2O$ | Up to 50 μL | Up to 50 μL |

Incubate the reaction for 1h at 37 °C in a thermal cycler.
Purify the reaction through the RNA Clean & Concentrator™-5 kit, following the protocol for small RNAs and performing the final elution in a volume of 6 μL of nuclease-free water (NFW).

*Step (b). Ligation.*

3'P RNA phosphorylated at both termini is ligated to an RNA-based adapter (having a sequence selected from SEQ ID No.: 173 - 177) containing 2-3 abasic sites, 8 degenerated nucleotides, a Fluor Uridine at 3' terminus and partial SP1 sequence, *via* RtcB ligase.
The RNA-based adapter has formula (I) as disclosed above. The elements constituting formula (I) reads on:

- SEQ ID NO.: 173 as follows: $N_x$: 1-4 nt; L1: 5-28 nt; $A_z$: 29-31; PR1 (the first nucleic acid domain of the Illumina adapter

construct): 32-51 nt; $N_y$: 52-55 nt; B: 56 nt;

- SEQ ID NO.: 174 as follows: $N_x$: 1-4 nt; L1: 5-28 nt; $A_z$: 29-31; PR1 (the first portion of the first nucleic acid domain of the Illumina adapter construct): 32-51 nt; $N_y$: 52-55 nt; C2: 56-63; B: 64 nt;
- SEQ ID NO.: 175 as follows: $N_x$: 1-4 nt; L1: 5-28 nt; $A_z$: 29-31, PR1 (the first portion of the first nucleic acid domain of the Illumina adapter construct): 32-51 nt; $N_y$: 52-55 nt; C2: 56-63; B: 64 nt;
- SEQ ID NO.: 176 as follows: $N_x$: 1-4 nt; C1: 5-12 nt; L1: 13-36 nt; $A_z$: 37-39; PR1 (the first portion of the first nucleic acid domain of the Illumina adapter construct): 40-59 nt; $N_y$: 60-63 nt; B: 64 nt;
- SEQ ID NO.: 177 as follows: $N_x$: 1-4 nt; C1: 5-12 nt; L1: 13-36 nt; $A_z$: 37-39; PR1 (the first portion of the first nucleic acid domain of the Illumina adapter construct): 40-59 nt; $N_y$: 60-63 nt; B: 64 nt.

RtcB ligase will join 5'OH termini of RNA-based adapter to a 3'P/3'cP termini of small RNAs, when present, according to the protocol indicated in Table 2.

**Table 2**

| Component | preferred amount | Amount |
|---|---|---|
| 10x Buffer | 1x | 1X-1.5x |
| 3mM GTP | 0.15 mM | 0.1-0.5 mM |
| 30 mM $MnCl_2$ | 1.8 mM | 1-3 mM |
| 15 pmol/μL RtcB ligase | 15 pmol | 15-30 pmol |
| RNA-based adapter (1uM) | 1.4 pmol | 0.25-2.4 pmol* |
| 5'P-smRNAs | 2 ug | 0.05 - 5 ug |
| $H_2O$ | Up to 10 μL | Up to 10 μL |

The amount of RNA-based adapter depends on the smRNAs amount starting material, as described in table 3 below.

**Table 3**

| RNA-based adapter | smRNAs |
|---|---|
| 0.25 pmol | 100 -500 ng |
| 0.4 pmol | 500-9000 ng |
| 0.7 pmol | 900-1500 ng |
| 1.4 pmol | 1600-2000 ng |
| 2.4 pmol | 2100-5000 ng |

Incubate 1 hour at 37 °C in a thermocycler.

Add nuclease free water up to 50 μL final volume, then purify the reaction through the RNA Clean & Concentrator™-5 kit, following the protocol for small RNAs and performing the final elution in a volume of 8 μL of nuclease free water.

*Step (c). Circularization*

The RtcB ligation product is subjected to circularization trough the ligation of 5'P termini and 3'OH termini by T4 RNA ligase 1. Reaction conditions are indicated in Table 4.

**Table 4**

| Component | preferred amount | Amount |
|---|---|---|
| 10x Buffer home made | 1x | 1x -1.5 x |
| 1mM ATP | 0.05 | 0.02-1 mM |
| 50% PEG800 | 20% | 10-22 % |

(continued)

| Component | preferred amount | Amount |
|---|---|---|
| 10 U/μL T4 RNA Ligase | 10U | 10 U |
| RtcB lig. product | 8uL | 8 μL |

Incubation for 2h at 25 °C.

Add nuclease free water up to 50 μL final volume, then purify the reaction through RNA Clean & Concentrator™-5 kit, following the protocol for small RNAs and performing the final elution in a volume of 10 μL of nuclease free water. OPTIONAL STOPPING POINT (store at -80°C).

*Step (d). Reverse Transcription (Superscript III)*

For the generation of single strand cDNA, the reverse transcription reaction is carried out using a reverse transcription primer (SEQ ID No.: 178) having formula (II) as disclosed above. The elements constituting formula (II) reads on the SEQ ID NO.: 178 as follows: R2 (the second nucleic acid domain of the Illumina adapter construct): 1-34 nt; Nz: 35-38; D1: 39-59 nt.

The reagents are mixed in the amounts indicated in Table 5 below.

**Table 5**

| Component | Preferred amount | Amount |
|---|---|---|
| 10 mM dNTPs | 0.5 mM | 0.5 mM |
| Circular RNA (from step 3) | 2000 ng | 50-5000 ng |
| 10 μM RT primer | 10 pmol | 1-20 pmol |
| $H_2O$ | Up to 14 μL | Up to 14 μL |

Heat the circular RNA-primer mix at 70°C for 5 minutes, and then incubate on ice for at least 1 minute. Add to the annealed RNA the reagents in the amounts indicated in Table 6.

**Table 6**

| Component | Preferred amount | Amount |
|---|---|---|
| 5x Buffer | 1X | 1x - 1.5x |
| DTT 0.1M | 5 mM | 1-10mM |
| 200 U/μL RT enzyme | 200 U | 20-200 U |

Incubate 40 mins at 50 °C, then heat the mix for 5 min at 80 °C.

*Step (e). PCR amplifications*

*First PCR*

KAPA Master mix or Phusion Master mix can be used. The first PCR amplification is carried out using a first pair of primers (SEQ ID No.: 179 and 180) having formula (III) and (IV) as disclosed above. The elements constituting formula (III) reads on the SEQ ID NO.: 179 as follows: T1 (the second portion of the first nucleic acid domain of the Illumina adapter construct): 1-13 nt; T2: 14-33 nt. Element T3 of formula (IV) corresponds to the entire sequence SEQ ID No.: 180.

The reagents are mixed in the amount indicated in Table 7 applying the reaction conditions indicated in Table 8.

**Table 7**

| Component | Preferred amount | Amount |
|---|---|---|
| Master Mix 2x | 1 X | 1 X - 1.5x |
| 10 μM First Fw primer | 0.08 uM | 0.05 - 1 uM |

(continued)

| Component | Preferred amount | Amount |
|---|---|---|
| 10 μM First Rev primer | 0.08 μM | 0.05 - 1 uM |
| cDNA (from step 4) | 20 μL | 20 μL |
| Nuclease free water | Up to 100 μL | Up to 100 μL |

**Table 8**

| Step KAPA | Temperature | Time |
|---|---|---|
| Initial denaturation | 95°C | 3 min |
| 6-9 Cycle | 98°C<br>61°C<br>72°C | 20 sees<br>15secs<br>15 sees |
| Hold | 4°C | |
| **Step Phusion** | **Temperature** | **Time** |
| Initial denaturation | 98°C | 30sec |
| 6-9 Cycle | 98°C<br>61°C<br>72°C | 10 sees<br>30secs<br>30 sees |
| Hold | 4°C | |

Purify the reaction using Ampure XP beads 1.6x ratio. Final product is eluted in a total volume of 40 μL of nuclease free water.

*Second PCR.*

KAPA Master mix or Phusion Master mix can be used. The second PCR amplification is carried out using a second pair of primers having the formula (IX) and formula (X), respectively, as disclosed above. The elements constituting formula (IX) are preferably the following ones: Q1 is the third nucleic acid domain of the Illumina adapter construct and has the nucleotide sequence set forth in SEQ ID No.: 181; Q2 is the fourth nucleic acid domain of the Illumina adapter construct and has a sequence selected from the sequences i5 by Illumina (10 nt); Q3 has the nucleotide sequence set forth in SEQ ID No.: 182. The elements constituting formula (X) are preferably the following ones: Q4 is the fifth nucleic acid domain of the Illumina adapter construct and has the nucleotide sequence set forth in SEQ ID No.: 183; Q5 is the sixth nucleic acid domain of the Illumina adapter construct and has a sequence selected from the sequences i7 by Illumina (10 nt); Q6 has the nucleotide sequence set forth in SEQ ID No.: 184.

The reagents are mixed in the amount indicated in Table 9 applying the reaction conditions indicated in Table 10.

**Table 9**

| Component | Amount (uL) | Amount |
|---|---|---|
| Master Mix 2x | 1 X | 1 X - 1.5x |
| UDIs 10 uM | 0.15 uM | 0.05 -1 uM |
| I PCR (from step 5) | 40 μL | 40 μL |
| Nuclease free water | Up to 100 μL | Up to 100 μL |

**Table 10**

| Step KAPA | Temperature | Time |
|---|---|---|
| Initial denaturation | 95°C | 3 min |

(continued)

| Step KAPA | Temperature | Time |
|---|---|---|
| 4-7 Cycle | 98°C<br>60°C<br>72°C | 20 sees<br>15secs<br>15 sees |
| Hold | 4°C | |

| Step Phusion | Temperature | Time |
|---|---|---|
| Initial denaturation | 98°C | 30 see |
| 4-7 Cycle | 98°C<br>60°C<br>72°C | 10 sees<br>30secs<br>30 sees |
| Hold | 4°C | |

Use Agencourt XP beads (1.6x ratio) or NucleoSpin Gel and PCR CleanUp kit to purify the entire 100 μl PCR reaction.

Agencourt XP beads: follow manufacturer's instructions and elute the sample in 40 μL of nuclease-free water.

Nucleospin Gel columns: follow the standard protocol in Section 5.1 of the manufacture manual. Elute each sample in 20 μl of NFW. Run the final PCR on a native 10% acrylamide gel and cut out the band at around 200 nt (figure 8).

The quality of the final library is checked at the bioanalyzer or similar (e.g. tapestation, QIAxcel) to test the length distribution of the PCR product and to define the average length of the library, which has to be between 190 nt and 300 nt.

The final concentration of the library is tested by a qPCR with P5 (AATGATACGGCGACCACCGAGATCTACAC - SEQ ID No.: 206) and P7 primers (CAAGCAGAAGACGGCATACGAGAT - SEQ ID No.: 207). The concentration should be higher than 0.5 nM.

The library quality check is performed as follows:

1.1 Evaluate each size selected library by Agilent 2100 Bioanalyzer using the Agilent High Sensitivity DNA Kit.
1.2 Use the library profile results to determine whether each sample is suitable for sequencing. Successful library production should yield a major peak at ~200 bp.
1.3 Perform a qPCR analysis using P5 and P7 primers on each final Dart-RNAseq library Successful library production should yield a final concentration of at least 0.1 nM.

*Step (f). Sequencing of the amplification product*

Sequencing of the amplified product is described by, but not limited to, the following steps:

1. Library denaturation and clustering: In this step, the library is denatured to separate the two DNA strands and then loaded onto a flow cell or sequencing chip. The library molecules are immobilized and amplified into clusters through bridge amplification or other cluster generation methods. Each cluster represents a cluster of identical DNA fragments.
2. Actual sequencing: Once the clusters are formed, sequencing is performed. The specific sequencing method may depend on the platform used (in the present case Illumina). The sequencing-by-synthesis method, where fluorescently-labeled nucleotides are added sequentially and their incorporation detected, is commonly employed.
3. Base calling and image analysis: During sequencing, the fluorescence signals or other detection signals are captured and converted into base calls. The base calls represent the nucleotide sequence of the DNA template. Image analysis software processes the raw data to generate base calls for each cluster.
4. Data processing and analysis: after sequencing, the raw data is processed to remove sequencing errors, adapter sequences, and low-quality reads. The resulting high-quality reads are then aligned to a reference transcriptome to generate the final sequence information.
5. Data Interpretation: The final step involves interpreting the sequenced data to extract meaningful biological information. This is performed as described in *"NGS data analysis"* in the section entitled "Materials and Methods".

*Step (g). The control*

Steps (a) to (f) are carried out on at least one control sample, wherein the control sample is a biological sample of a healthy, treated or non-treated subject.

*Step (h). Identification of the 3'P RNA as disease biomarker*

The identification of the 3'P RNA as disease biomarker is carried out as follows:

a. mapping on the reference genome or transcriptome the sequence of the at least one 3'P RNA contained in the amplification product obtained for the biological sample of the diseased or treated subject and the control; and
b. calculating for the at least one 3'P RNA contained in the amplification products obtained for the biological sample and the control sample: (i) the number of counts, (ii) the Dart-RNAseq p-value, (iii) the Dart-RNAseq fold change of the number of counts or the Dart-RNAseq fold change of a normalized parameter based on the number of counts in the biological sample *versus* the control sample, (iv) the cleavage pattern, (v) the normalized counts based on sequencing depth, (vi) the multimapping score and (vii) the length of the 3'P RA sequence.

The 3'P RNA is a molecular marker of the disease if the 3'P RNA contained in the amplification product of the biological sample of the diseased subject fulfils the following conditions:

- number of counts > 200,
- Dart-RNAseq p-value: ≤ 0.05,
- Dart-RNAseq fold change ≥ 2 or ≤ 0.5, and
- cleavage pattern ≤ 40% per-base cleavage frequencies along the 3'P RNA length and ≥ 60% per-base cleavage frequencies on the 5' and 3' ends of the 3'P RNA.

Further conditions useful for determining if the 3'P RNA is a molecular marker of the disease are:

- normalized counts based on sequencing depth > 5,
- multimapping score ≤ 100,
- length > 15 and < 200 nt.

***Schematic description of 3'P-qPCR assay***

*Step (a'). Small RNA Phosphorylation.*

Upon small RNA enrichment (<200 nt), 3'P RNAs will be subjected to 5' phosphorylation by T4 PNK 3' Minus, according to the protocol indicated in Table 11.

**Table 11**

| Component | Preferred amount | Amount |
|---|---|---|
| 10x Buffer | 1X | 1X-1.5x |
| 10mM ATP | 1 mM | From 1 mM to 10 mM |
| 10 U/ μL T4 PNK 3' minus | 10U | From 1U to 20 U |
| smRNAs | 2 μg | 0.05 - 5 μg |
| $H_2O$ | Up to 50 μL | Up to 50 μL |

Incubate the reaction for 1h at 37 °C in a thermal cycler.
Purify the reaction through the RNA Clean & Concentrator™-5 kit, following the protocol for small RNAs and performing the final elution in a volume of 6 μL of nuclease-free water (NFW).

*Step (b'). 3'P ligation.*

Small RNA phosphorylated at 5' termini, will be ligated to an RNA-based adapter (SEQ ID No.: 185) having formula (Ia) as disclosed above, with 3 abasic sites, *via* RtcB ligase. The elements constituting formula (Ia) reads on the

SEQ ID NO.: 185 as follows: E1: 1-24 nt; $A_z$: 25-27, E2: 28-48 nt. RtcB ligase will join 5'OH termini of RNA-based adapter to a 3'P/3'cP termini of small RNAs, when present, according to the protocol indicated in Table 12.

**Table 12**

| Component | preferred amount | Amount |
|---|---|---|
| 10x Buffer | 1x | 1X-1.5x |
| 3mM GTP | 0.15 mM | 0.1-0.5 mM |
| 30 mM $MnCl_2$ | 1.8 mM | 1-3 mM |
| 15 pmol/µL RtcB ligase | 15 pmol | 15-30 pmol |
| RNA-based adapter (1uM) | 1.4 pmol | 0.25-2.4 pmol* |
| 5'P-smRNAs | 2 ug | 0.05 - 5 ug |
| $H_2O$ | Up to 10 µL | Up to 10 µL |

The amount of RNA based adaptor (Linker_qPCR, seq ID 185) depends on the smRNAs starting material, as described in table 13 below.

**Table 13**

| RNA-based adapter | smRNAs |
|---|---|
| 0.1 | 10-100 ng |
| 0.25 pmol | 100 -500 ng |
| 0.4 pmol | 500-9000 ng |
| 0.7 pmol | 900-1500 ng |
| 1.4 pmol | 1600-2000 ng |
| 2.4 pmol | 2100-5000 ng |

Incubate 1 hour at 37 °C in a thermocycler.

Add nuclease free water up to 50 µL final volume, then purify the reaction through the RNA Clean & Concentrator™-5 kit, following the protocol for small RNAs and performing the final elution in a volume of 8 µL of nuclease free water.

*Step (c'). Circularization*

The RtcB ligation product is subjected to circularization trough the ligation of 5'P termini and 3'OH termini by T4 RNA ligase 1. Reaction conditions are indicated in Table 14.

**Table 14**

| Component | preferred amount | Amount |
|---|---|---|
| 10x Buffer home made | 1x | 1x -1.5 x |
| 1mM ATP | 0.05 | 0.02-1 mM |
| 50% PEG800 | 20% | 10-22 % |
| 10 U/µL T4 RNA Ligase | 10U | 10 U |
| RtcB lig. product | 8uL | 8 µL |

Incubation: 2h at 25 °C.

Add nuclease free water up to 50 µL final volume, then purify the reaction through RNA Clean & Concentrator™-5 kit, following the protocol for small RNAs and performing the final elution in a volume of 10 µL of nuclease free water. OPTIONAL STOPPING POINT (store at -80°C).

*Step (d). Reverse Transcription (Superscript III)*

For the generation of single strand cDNA, the reagents are mixed in the amounts indicated in Table 15. The reverse transcription primer (SEQ ID NO.: 186) has formula (IIa) as disclosed above. The elements constituting formula (IIa) reads on the SEQ ID NO.: 186 as follows: G: 1-19 nt; F1= 20-35 nt.

**Table 15**

| Component | Preferred amount | Amount |
|---|---|---|
| 10 mM dNTPs | 0.5 mM | 0.5 mM |
| Circular RNA (from step 3) | 2000 ng | 50-5000 ng |
| RT 3'qPCR primer | 10 pmol | 1-20 pmol |
| $H_2O$ | Up to 14 μL | Up to 14 μL |

Heat the circular RNA-primer mix at 70°C for 5 minutes, and then incubate on ice for at least 1 minute. Add to the annealed RNA the reagents in the amounts indicated in Table 16.

**Table 16**

| Component | Preferred Amount | Amount |
|---|---|---|
| 5x Buffer | 1X | 0.5X - 1.5 |
| DTT 0.1M | 5 mM | 1-10 mM |
| 200 U/ μL RT enzyme | 200 U | 1-200 U |

Incubate 40 mins at 50 °C, then heat the mix for 5 min at 80 °C.

*Step (e'). 3'P-qPCR*

A first and a second qPCR amplification of the at least one single strand cDNA molecule are carried out in parallel, wherein:

(i) the first qPCR amplification is carried out using a first pair of primers selected from Set1, Set2 and Set3. The elements H1, and H2 of formulas (IIIa, IIIa', IIIa", IVa, IVa' and IVa") have a fixed sequence independently from the disease under investigation, while the elements M and N must have a specific sequence annealing on the 3'P RNA marker under analysis;
(ii) the second qPCR amplification is carried out using a second pair of primers comprising a second forward and a second reverse primer (SEQ ID No.: 204 and 205) having formula (Va) and (VIa), respectively.

The qPCR amplification was performed by SYBR™ Green PCR Master Mix for all qPCR amplification steps.

**Table 17**

| Component | Amount (uL) | Amount |
|---|---|---|
| Syber Green Master Mix 2x | 5 uL | 1X |
| 10 μM Fw primer | 0.2uL | 0.2 uM |
| 10 μM Rev primer | 0.2uL | 0.2 μM |
| cDNA diluted 1:3 (from step 4) | 1 μL | 1 μL |
| Nuclease free water | 3.6 uL | Up to 10 μL |

**Table 18**

| Step KAPA | Temperature | Time |
|---|---|---|
| Initial denaturation | 95°C | 3 min |

(continued)

| Step KAPA | Temperature | Time |
|---|---|---|
| 50 Cycles | 98°C<br>58-65°C* | 20 sees<br>15secs |
| Hold | 4°C | |

The melting temperature must be adjusted depending on the specific primers used for amplification.

*Step (f'). The control*

Steps (a') to (e') are carried out on at least one control sample, wherein the control sample is a biological sample of a healthy, treated or non-treated subject.

*Step (g'). Determining the Ct values*

The quantitative analysis of qPCR is obtained through analysis of the quantification of cycle values (Ct or threshold cycles) given by the qPCR instrument. As the cycle value (Ct) increases, the detected fluorescence also increases. When the fluorescence crosses an arbitrary line, the device records the cycles value until then, which is known as the Ct value. The quantity of the 3'P RNA in a given sample is then determined using a relative or comparative quantification.

The Ct values for the at least one 3'P RNA are determined in the first and second amplification products of each biological sample.

*Step (h'). Calculation of the 3'P-qPCR fold change*

Relative or comparative quantification uses the difference in Ct as a determinant of the differences in concentration of the 3'P RNA in the biological sample and the control sample.

The calculation of the 3'P-qPCR fold change of the Ct values for the at least one 3'P RNA is done according to the following formula:

$$3'\text{P qPCR fold change} = \frac{2^{Ct(B)_{3'P}-Ct(A)_{3'P}}}{2^{Ct(B)_{adp}-Ct(A)_{adp}}} = \frac{2^{\Delta Ct(3'P)}}{2^{\Delta Ct(adp)}} = 2^{\Delta\Delta Ct}$$

wherein

wherein

- $Ct(A)_{3'P}$ is the Ct value for the 3'P RNA determined in the biological sample,
- $Ct(B)_{3'P}$ is the Ct value for the 3'P RNA determined in the control sample,
- $Ct(A)_{adp}$ is the Ct value for the RNA-based adapter determined in the biological sample,
- $Ct(B)_{adp}$ is the Ct value for the RNA-based adapter determined in the control sample;

A 3'P-qPCR fold change value ≥ 2 or ≤ 0.5 is indicative of the diagnosis, prognosis, therapy monitoring, outcome prediction assessment of the disease or condition.

**Example**

To closely examine the potential 3'P RNA fragments as biomarkers, the inventors focused on two case studies: (i) Spinal Muscular Atrophy (SMA) and (ii) cutaneous Squamous Cell Carcinoma (cSCC). The specific embodiments disclosed in the present disclosure are not to be interpreted as limiting the scope of protection of the present application, as the methods disclosed herein can be used for identifying molecular markers of different diseases as well as for diagnosing, prognosticating and monitoring different diseases.

The diagnosis of SMA is well established through the genetic detection of a SMN1 mutation and loss of SMN1 protein; but, very poor markers for disease progression and treatment efficacy are available[29], even so three drug treatments are approved so far.

For SMA, Dart-RNAseq analysis of liver tissues from an early symptomatic SMA mouse model revealed a global

downregulation of 3'P RNAs, many of which were classified as tRNA fragments (tRFs). Previous reports found that tRFs have potential as biomarkers for various diseases, including cancer and neurological disorders[5,19,30-32]. As a proof of concept, with the present approach only the subpopulation of tRFs having 3'P was screened, thus ensuring a better resolution on this specific category of fragments. Among them, 3'P-tRFs_Val showed 3-fold decrease in SMA liver compared to controls, with a clear cleavage site at anticodon loop and at the CCA tRNA tail, forming a fragment of 39 nt known also as 3'tRNA half[33]. As upregulated RNAs the 3'P Gm22973 was identified, a fragment that arise from the Gm22973 transcript, also known as U2 snRNA pseudogene in the mouse transcriptome. Interestingly, loss of function of SMN protein in SMA pathology is linked to alteration in snRNPs assembly, suggesting a correlation between SMA and the overexpression of 3'P-Gm22973 fragment.

Cutaneous squamous cell carcinoma is characterized by abnormal growth of squamous cells. Most cSCCs can be treated by surgery, but a fraction of them recurs and metastasize, leading to death with a high (> 50%) probability. cSCC incidence is increasing year over year, but still there are not reliable molecular markers of cancer progression, recrudescence and methastasis[34]. Here, we aim to combine a low input 3'P RNA next generation Sequencing (NGS) method with a targeted 3'P-qPCR assay to profile and quantify 3'P RNA fragments.

For cSCC, immortalized squamous carcinoma cells and healthy keratinocytes cell were analysed. The expression of 3'P RNA fragments specific for cSCC were explored.

3'P tRFs deriving from the 5' end of tRNA_glutammmate (5'_tRFs_Glu_CTC) and 3' end of tRNA_aspartate_GTC (3'_tRFs_Asp_GTC) were identified as potential markers of disease. Overall, by 3'P-qPCR the inventors successfully confirmed Dart-RNAseq data on selected cSCC targets samples while being able to discriminate among highly conserved tRNA sequences.

In conclusion, the present description demonstrates that the combination of Dart-RNAseq analysis and the 3'P-qPCR assay are useful to screen, identify and validate potential new marker of disease, unrevealing 3'P RNAs-omics as biomarkers of diseases.

## Results

### *3'P RNA fragments in Spinal Muscular Atrophy*

To suit low input requirements needed for biomarkers studies we designed a method named Dart-RNAseq. This method evolved from a previously developed circAID technology - based on a protocol for 3'P RNA nanopore sequencing[35] - by introducing two main improvements. First, a ligation step with specific adapters containing, (i) an internal retro-transcription stopping site, (ii) 12 nt as unique molecular identifier (UMI) for PCR duplicates identification and (iii) a barcode sequence (8 nt) for pooling multiple samples in a single run. Second, a retro-transcription coupled with a 2-step PCR thus making the workflow ideal for NGS sequencing. This approach allows to sequence and profile transcriptome-wide 3'P RNAs. We tested the workflow by adding an exogenous nuclease (RNAse I) to a crude cell lysate of a CHO cell lysate, followed by ribosomes isolation and RNA extraction, a procedure called ribosome profiling and used to identify ribosome footprints (RPF)[36]. Our results retrieved an enrichment of RPF in the coding sequence with a clear 3-nucleotide periodicity of the ribosome P-site, confirming the ability of the method to sequence 3'P RNAs generated by enzymatic cleavage of RNAse I (Figure 1). We named the method Dart-RNAseq.

To identify suitable starting material for a biomarker screening we analysed publicly available RNA seq data sets. After screening RNA-seq datasets[37] of SMA models we observed that early symptomatic (P5) SMA mouse livers showed an increasing level of angiogenin, a well-known enzyme forming 3'P tRFs[38,39]. Dart-RNAseq analysis of P5 SMA and healthy livers was performed starting from 500 ng of size-selected small RNAs (< 200 nt in length). The reads length distribution of the sequencing output for all RNA fragments ranged from 15 to 70 nt, with two major peaks around 20 and 35 nt (Figure 2). The majority of reads mapped on non-coding RNAs, mainly rRNA (25% for the control and 23% for the SMA) and tRNA (29% for the control and 25% for the SMA) (Figure 3).

Surprisingly, differential expression analysis of Dart-RNAseq (SMA vs control) indicates a global reduction of 3'P RNAs in P5 liver compared to control (Table 19 - Number of up- and down-regulated 3'P RNA fragments detected), suggesting that Angiogenin overexpression is not a primary cause of 3'P RNA fragment in those samples, but other nucleases should be involved as well. All the 3'P RNA fragments highlighted in table are filtered for Dart-RNAseq log2FC > 1 or log2FC < -1 and Dart-RNAseq pval <0.05.

**Table 19**

| 3'P RNA fragments | Upregulated in SMA | Downregulated in SMA |
|---|---|---|
| 3'P RNA without tRFs | 3 | 14 |
| 3'P tRFs only | 0 | 36 |

(continued)

| 3'P RNA fragments | Upregulated in SMA | Downregulated in SMA |
|---|---|---|
| Total 3'P RNAs | 3 | 50 |

More specifically, we found 50 downregulated 3'P RNAs (p-val<0.05; Dart-RNA seq log2FC < -1), of which 38 are tRNA fragments. On the contrary, only three fragments resulted upregulated (Dart RNAseq p-val<0.05; Dart-RNAseq log2FC >1). To select the most robust hits for further investigation, we applied more stringent filtering steps based on (i) read counts (> 300), (ii) Dart-RNAseq fold change (log2FC >2 or log2Fc <-2), (iii) Dart RNA-seq pval <0.05 and, (iv) fragment length > 15 nt), (iv) shape of the cleavage pattern. To measure the last parameter, we plot the reads count for each fragment as a function of the nucleotide position. Only fragments with less than 40% per-base cleavage frequencies along the entire length and with at least 60% per-base cleavage frequencies on the 5' and 3' termini (Figure 5) were considered. We included in the analysis only fragments longer than 15nt, uniquely mapping on a target or with a low multimapping score (≤ 100 multimapping read - see material and methods). We targeted three downregulated fragments, all deriving from tRNA Valine (tRFs Val-AAC, tRFs val-CAC and tRFs Val-TAC), and 1 upregulated fragment, deriving from Gm22973 (U2 snRNA pseudogene). Specifically, tRFs Val were the only fragments presenting a clear cleavage at the nucleotide 37 of the anticodon loop (Figure 4), generating a fragment of 39 nt ending at nt 75 of the CCA tail, defined as 3' tRNA half [33]. Gm22973 fragments arise from the full length Gm22973 transcript. Interestingly the 3'P RNA fragments detected are aligning to the Sm protein binding RNA binding site (Figure 6). Sm proteins are a set of proteins associated with snRNA to form small nuclear ribonucleo-particles (snRNPs) involved in splicing. Of note, that SMN is involved in the recruitment and assembly of Sm protein into snRNPs and loss of SMN protein, cause an altered assembling in SMA[40].

To precisely quantify 3'P RNA fragments with a defined 5' and 3' ends, we designed a dedicated 3'P-qPCR assay based on adapter ligation, circularization and selective amplification. The adapter and the final qPCR step have different features than the one used in Dart-RNAseq (see Methods). We designed primers at the junction between the adapter and the fragment, annealing to the target sequence for at least 6 nt on each end of the fragment of interest. The downregulated valine isodecoders (3'P tRFs Val-AAC, tRFs Val-CAC and tRFs Val-TAC) have identical cleavage pattern and were chosen as good candidate as initial validation. We designed primers at the adapter-fragment junction for each of the three selected candidates (3'P tRFs Val-AAC/CAC from 3'P tRFs Val-TAC). While doing this, we were able to discriminate 3'P tRFs Val TAC. As expected, we did not discriminate 3'P tRFs Val-AAC from tRFs Val-CAC due to the full identity of the last 10 nucleotides at the 5' and 3' ends of the two fragments. After 3'P-qPCR we observed a 2-fold downregulation of 3'P tRFs Val AAC/CAC in SMA liver tissue compared to control (Figure 7 A), in agreement with Dart-RNAseq data. On the contrary no significative downregulation is observed for 3'P tRFs Val-TAC, revealing that a targeted qPCR approach is needed for the evaluation of a specific fragment. To further validate our results, we performed northern blot analysis. The 3'P tRFs Val-AAC/CAC fragment band in the gel showed a 2-fold lower intensity in mouse SMA liver samples compared with healthy mouse liver, confirming 3'P-qPCR results (Figure 7B). Altogether, our data suggest that 3'P tRFs VAl AAC/CAC is a good marker of disease within the biological model under consideration.

Next, we sought to validate the Gm22973 derived fragment identified by sequencing. This RNA is upregulated in SMA liver samples and presents only 1 point mutation, in position 15, compared to another fragment present in our sequencing output and mapping on U2 transcript, which does not significantly change between control and SMA samples. Of note the U2-derived fragments is much more abundant compared to the pseudogene derived fragments (counts U2 fragment: 24000; counts GM22973 fragment: 600. Both as mean counts in SMA samples). To discriminate between the two types of fragments, which differ only for a single nucleotide, we designed two couples of primers. The forward is placed at the adapter-fragment junction and it is in common between the two fragments, while the reverse primers differ only for the last nucleotide at 3' end, which map at the position of the mismatch between the Gm22973 derived fragment and the U2 derived fragment. We observed a 4-fold increase in Gm22973 specific primers, while no changing is showed for the U2 derived fragment (Figure 8A and C), demonstrating that our 3'P-qPCR approach has a single nucleotide resolution in the detection of specific 3'P RNA fragments of interest.

We further wanted to assess if the increase in abundance in SMA samples reflects a change in the full-length pseudogene transcript or if it is only related to the specific fragment. To do that, we performed a standard qPCR on the full-length Gm22973 and U2 transcripts. We observed that the increase of Gm22973 fragment also reflect the higher expression of full-length pseudogene in SMA samples (Figure 8B), while the full length U2 transcript is decreasing (Figure 8D). Altogether our data suggest that the full-length Gm22973 transcripts as well as derived fragmentation products, are potential marker of disease.

To further investigate if the method applied on mouse samples could be used on human derived SMA cells, we applied Dart-RNAseq to fibroblast human cells obtained from SMA I patients, SMA II patients and healthy individuals.

After differential expression analysis we identified a list of differentially expressed 3'P RNAs between healthy vs SMA1, healthy vs SMA2 and SMA1 vs SMA2 (Table 20). Among them we confirm tRFs Val-AAC/CAC as a target, although

with a different fragment sequence compared to mouse data.

Our results confirmed the robustness of the method across species and highlight the potential of tRFs Val-AAC/CAC and other 3'P RNA (listed in Table 20) as biomarkers for SMA.

**Table 20**

| 3'P RNA_name | Sequence | Length | SEQ ID No. |
|---|---|---|---|
| tRNA-iMet-CAT | AGCAGAGUGGCGCAGC | 16 | 1 |
| tRNA-iMet-CAT | AGCAGAGUAGCGCAGC | 16 | 2 |
| tRNA-Ala-CGC | GGGGGUGUAGCUCAGUGGUAGAGCGUGCU UC | 31 | 3 |
| tRNA-Leu-TAA | ACCAGGAUGGCCGAGUGG | 18 | 4 |
| tRNA-Leu-TAA | ACCAGGAUGGCCGAG | 15 | 5 |
| tRNA-Ala-AGC | GGGGAAUUAGCUCAAGUGGU | 20 | 6 |
| tRNA-Glu-CTC | AGUGGUUAGGAUUCGGCGCUCU | 22 | 7 |
| tRNA-Glu-CTC | GGUUAGGAUUCGGCGCUC | 18 | 8 |
| tRNA-Leu-TAA | ACCGGGAUGGCCGAGUGG | 18 | 9 |
| tRNA-iMet-CAT | AGCAGAGUGGCGCAGCGGAAGCGU | 24 | 10 |
| tRNA-iMet-CAT | AGCAGAGUUGCGCAGC | 16 | 11 |
| tRNA-iMet-CAT | AGCAGAGUGGCGCAG | 15 | 12 |
| tRNA-iMet-CAT | AUAACCCAGAGGUCGAUGGAUCG | 23 | 13 |
| tRNA-Ala-AGC | GGGGGUGUAGCUCAGUGGUAGAGCGUGCU U | 30 | 14 |
| tRNA-Ala-AGC | GGGGGUGUAGCUCAGUGGUAGAGCGUGCU UG | 31 | 15 |
| tRNA-Ala-AGC | GGGGGUGUAGCUCAGUGGUAGAGCGCGUG CUUGG | 34 | 16 |
| tRNA-Ala-AGC | GGGGGUAUAGCUCAGCGGUAGAGCGCGUG CUUGG | 34 | 17 |
| tRNA-Ala-AGC | GGGGGUGUAGCUCAGUGGUAGAGCGCGUG CU | 31 | 18 |
| tRNA-Ala-AGC | GGGGAUGUAGCUC | 13 | 19 |
| tRNA-Ala-AGC | GGGGAAUUAGCUC | 13 | 20 |
| tRNA-Ala-TGC | GGGGGUGUAGCUCAGUGGUAGAGCGUGCU U | 30 | 21 |
| tRNA-Val-AAC | GUUUCCGUAGUGUAGUGGUU | 20 | 22 |
| tRNA-Val-AAC | GUUUCCGUAGUGUAGUGG | 18 | 23 |
| tRNA-Val-AAC | GUUUCCGUAGUGUAGU | 16 | 24 |
| tRNA-Val-AAC | GUUUCCGUAGUGUAGUG | 17 | 25 |
| tRNA-Val-AAC | GUUUCCGUAGUGUAGUGGU | 19 | 26 |
| tRNA-Val-AAC | GUUUCCGUAGUGU | 13 | 27 |
| tRNA-Gly-TCC | GCGUUGGUGGUAUAGUGGUUAGCAUAGCU GCCUUCC | 36 | 28 |
| tRNA-Gly-TCC | GCGUUGGUGGUAUAGUGGUG | 20 | 29 |

(continued)

| 3'P RNA_name | Sequence | Length | SEQ ID No. |
|---|---|---|---|
| tRNA-Gly-TCC | GCGUUGGUGGUAUAGUGGUUAGCAUAGCUGCCUUC | 35 | 30 |
| tRNA-Gly-TCC | GCGUUGGUGGUAUAGUGGUUAGCAUAGCUGCCUU | 34 | 31 |
| tRNA-Gly-TCC | GCGUUGGUGGUAUAGUGGUAAGCAUAGCUGCCUUC | 35 | 32 |
| tRNA-Gly-TCC | GCGUUUGUGGUAUAGUGGUGAGCAUAGCUGCCUUC | 35 | 33 |
| tRNA-Gly-TCC | GCGUUUGUGGUAUAGUGGUUAGCAUAGCUGCCUUCC | 36 | 34 |
| tRNA-Gly-TCC | GCGUUGUGGUAUAGUGGUGAGCAUAGCUGCCUUC | 34 | 35 |
| tRNA-Gly-TCC | GCGUUGGUGGUAUAGUGGUGAGC | 23 | 36 |
| tRNA-Gly-TCC | GCGUUGGUGGUAUAGUGGUAAGCAUAGCUGCCUU | 34 | 37 |
| tRNA-Gly-TCC | GCGUUUGUGGUAUAGUGGUG | 20 | 38 |
| tRNA-Gly-TCC | GCGUUGUGGUAUAGUGGUUAGCAUAGCUGCCUUCC | 35 | 39 |
| tRNA-Gly-TCC | GCGUUGGUGGUAUAGUGGUAAGCAUAGCUGCCU | 33 | 40 |
| tRNA-Gly-TCC | GCGUUGUGGUAUAGUGGUGAGCAUAGCUGCCUU | 33 | 41 |
| tRNA-Gly-TCC | GCGUUGGUGGUAUAGUGGUUAGC | 23 | 42 |
| tRNA-Glu-TTC | UCCCAUAUGGUCUAGCGGUUAGGAUUCCUGGUUUUC | 36 | 43 |
| tRNA-Glu-TTC | AGGAUUCGGCGC | 12 | 44 |
| tRNA-Gly-CCC | GCAUUGGUGGUUCA | 14 | 45 |
| tRNA-Gly-CCC | GCAUUGGUGGUUCAGUG | 17 | 46 |
| tRNA-Leu-TAA | ACCGGGAUGGCCGAGUGGUU | 20 | 47 |
| tRNA-Leu-TAA | ACCAGAAUGGCCGAGUGGUU | 20 | 48 |
| tRNA-Leu-TAA | ACCAGGAUGGCCGAGUGGU | 19 | 49 |
| tRNA-Val-AAC | GUUUCCGUAGUGUAGUGGUUA | 21 | 50 |
| tRNA-Glu-CTC | GGUUAGGAUUCG | 12 | 51 |
| tRNA-Glu-TTC | UAGGAUUCGGCGC | 13 | 52 |
| tRNA-Glu-CTC | AGUGGUUAGGAUU | 13 | 53 |
| tRNA-Leu-TAA | ACCAGAAUGGCCGAGUGGUUA | 21 | 54 |
| tRNA-Leu-TAA | ACCAGGAUGGCCGAGUG | 17 | 55 |
| tRNA-iMet-CAT | AGCAGAGUGGCGCAGCGGAAGCGUGCUGGGCCCAUAACC | 39 | 56 |

(continued)

| 3'P RNA_name | Sequence | Length | SEQ ID No. |
|---|---|---|---|
| tRNA-Val-AAC | GUUUCCGUAGUGUAGUGGUUAUCA | 24 | 57 |
| tRNA-Val-AAC | GUUUCCGUAGUGUAGUGGUUAU | 22 | 58 |
| tRNA-Val-AAC | GUUUCCGUAGUG | 12 | 59 |
| tRNA-Val-AAC | GUUUCCGUAGUGUA | 14 | 60 |
| tRNA-Val-CAC | GCUUCUGUAGUGUAGUGG | 18 | 61 |
| tRNA-Leu-TAA | ACCGGGAUGGCCGAGUGGUUA | 21 | 62 |
| tRNA-iMet-CAT | AGCAGAGUUGCGCAGCGGAAGCGU | 24 | 63 |
| tRNA-iMet-CAT | AGCAGAGUGGCGCAGCGGAAGC | 22 | 64 |
| tRNA-iMet-CAT | AGCAGUUGCGCAGCGGAAGCGUGCUGGGC CC | 31 | 65 |
| tRNA-iMet-CAT | AGCGGAAGCGUGCUGGGCCC | 20 | 66 |
| tRNA-iMet-CAT | AUAACCCAGAGGUCGAUGGAUC | 22 | 67 |
| tRNA-iMet-CAT | GGAAGCGUGCUGGGC | 15 | 68 |
| tRNA-iMet-CAT | AUAACCCAGAGGUCGAUGGA | 20 | 69 |
| tRNA-iMet-CAT | GGAAGCGUGCUGGGCC | 16 | 70 |
| tRNA-Ala-AGC | GGGGGUGUAGCUCAGUGGUAGAGCGCGUG C | 30 | 71 |
| tRNA-Ala-AGC | GGGGGUAUAGCUCAGCGGUAGAGCGUGCU UGG | 32 | 72 |
| tRNA-Ala-AGC | GGGGGUAUAGCUCAGCGGUAGAGCGUGCU UGGC | 33 | 73 |
| tRNA-Ala-AGC | GGGGGUAUAGCUCAGCGGUAGAGCGCGUG CU | 31 | 74 |
| tRNA-Ala-AGC | GGGGGUAUAGCUCAGCGGUAGAGCGCGUG C | 30 | 75 |
| tRNA-Gly-TCC | GCGUUUGUGGUAUAGUGGUAAGCAUAGCU GCCUUC | 35 | 76 |
| tRNA-Glu-CTC | GGUCUAGUGGUUAGGAUUCGGCGCUCUC | 28 | 77 |
| tRNA-Glu-CTC | GGUUAGGAUUCGGCGCUCU | 19 | 78 |
| tRNA-Glu-CTC | CUAGUGGUUAGGAUU | 15 | 79 |
| tRNA-Leu-TAA | ACCGGGAUGGCCGAGUGGU | 19 | 80 |
| tRNA-Leu-TAA | ACCAGAAUGGCCGAGUGG | 18 | 81 |
| tRNA-Val-AAC | GUUUCCGUAGUGUAGUGGUC | 20 | 82 |
| tRNA-Ile-AAT | GGCCGGUUAGCUCAGUUGGU | 20 | 83 |
| tRNA-Asn-GTT | GAAATCAGGAGCTCAGCCATGTCTCTGTGG CGCAATCGGC | 40 | 208 |
| tRNA-Asn-GTT | TTCGAACCCACCCAGAGGCGTCGCTGATATT TTATAACTC | 40 | 209 |
| HoXB7#1 | CAACAUGAAACUACCUA | 17 | 210 |
| HoxB7#2 | ACCCAACAACAUGAAACUACCUA | 23 | 211 |

(continued)

| 3'P RNA_name | Sequence | Length | SEQ ID No. |
|---|---|---|---|
| U1#1 | ACGAAGGUGGUUUUCUCAGGGCGAGGCUU AUCCAUUGC | 38 | 212 |
| U1#2 | GCAAUGGAUAAGCCUCGCCCUGAGAAAAC CACCUUCGU | 38 | 213 |

Finally, we evaluated changes in 3'P RNA profiles following treatment with Risdiplam and Nusinersen, two drugs designed to modulate the SMN2 gene and increase SMN protein production. This analysis aimed to determine how these treatments alter 3'P RNA expression, thereby providing insights into their molecular effects and the potential for 3'P RNAs to serve as markers for treatment response.

The 3'P RNA molecular markers of treatment response identified are listed in table 21 below.

**Table 21**

| 3'P RNA name | Sequence | Treatment | SEQ ID No. |
|---|---|---|---|
| U6#1 | AACCAGGCCNGACCCUGCUUAGCUUCCGAGAUCAG | Risdiplam | 214 |
| U6#2 | CAGGCCCGACCCUGCUUAGCUUCCGAGAUCA | Risdiplam | 215 |
| SNORA70 | GGCCCGACCCUGCUUAGCUUCCGAGAUCA | Risdiplam | 216 |
| tRNA-Pro-CGG | GCGAGAGGUCCCGGGUUCAAAUCCCGGACGAGCCC | Spinraza | 217 |

***3'P RNA fragments in cutaneous Squamous Cell Carcinoma***

To identify potential molecular marker of malignant skin lesions we first performed Dart-RNAseq analysis on 4 replicates of HSC-1, a human skin squamous cell carcinoma cell line, comparing the results with those obtained form 4 control samples of keratinocytes. After applying Dart-RNAseq, the results show similar mapping results as the one obtained for SMA samples. Reads mapped mostly on non-coding RNAs, of which 25% are tRNAs (Figure 9). cSCC and keratinocytes samples were clearly clustered by the principal component analysis (PCA) on 3'P tRFs (Figure 10), suggesting that this subgroup of 3'P RNAs can be used as indicator of disease state. Differential expression analysis of normal vs tumour samples shows 105 significantly downregulated tRNA fragments out of 1930 hits and 77 out of 1108 tRNA fragments significantly upregulated. Interestingly, tRFs up or down regulated according to the charge of encoded amino acid (i.e. 3'P tRFs downregulated encode for negative amino acid, upregulated to a positive amino acid), suggesting a possible preferential codon usage in cSCC compared with control cells. By looking at the type of tRFs, we observed a clear downregulation of 5' and 3' long tRFs, while 3'P short tRFs are mainly upregulated (Table 22 - Number of up- and down-regulated total 3'P tRNA fragments (tRFs, lane 1) and reported for each tRFs sub-type (from lane 2 to lane 6). All the 3'P tRNA fragments highlighted in table are filtered for dart-RNAseq log2FC > 1 or log2FC < -1 and Dart-RNAseq pval <0.05.

**Table 22**

| Lane | Classification tRFs | Downregulated in cSCC | Upregulated in cSCC |
|---|---|---|---|
| 1 | total | 20 | 35 |
| 2 | 5' long | 13 | 0-2 |
| 3 | 5' short | 0 | 2 |
| 4 | 3' long | 7 | 0 |
| 5 | 3' short | 0 | 18 |
| 6 | other | 0 | 13 |

The best hits for further investigation with 3'P-qPCR were selected based on the previously described filtering steps: (i) read counts (>200) (ii) Dart-RNAseq fold change (log2FC >1 or log2Fc <-1) (iii) Dart-RNAseq pval <0.05 and (iv) sharp cleavage pattern. As first validation, we designed primers for a 3'P tRFs deriving from the 5' end of tRNA_glutammmate (5'_tRFs_Glu_CTC) and 3' end of tRNA_aspartate_GTC (3'_tRFs_Asp_GTC). By the 3'P-qPCR assay we confirmed the downregulation of both tRFs in cSCC cell line compared with healthy keratinocyte (Figure 11). The 3'P RNA molecular markers of cSCC identified are listed in table 23.

**Table 23**

| tRNA_name | Sequence | Length | SEQ ID No. |
|---|---|---|---|
| tRNA-Asp-GTC | CACGCGGGAGACCGGGGUUCGAUUCCCCGAC | 31 | 84 |
| tRNA-Asp-GTC | GCGGGAGACCGGGGUUCGAUUCCCCGACGGGGAGC | 35 | 85 |
| tRNA-Asp-GTC | ACGCGGGAGACCGGGGUUCGAUUCCCCGACGGGGAGCC | 38 | 86 |
| tRNA-Asp-GTC | ACGCGGGAGACCGGGGUUCGUUUCCCCGACGGGGAGC | 37 | 87 |
| tRNA-Asp-GTC | ACGCGGGAGACCGGGGUUCAAUUCCCCGACGGGGAGC | 37 | 88 |
| tRNA-Asp-GTC | GCGGGAGACCGGGGUUCGUUUCCCCGACGGGGAGC | 35 | 89 |
| tRNA-Asp-GTC | CACGCGGGAGACCGGGGUUCGAUUCCCCGACGGGGAGCC | 39 | 90 |
| tRNA-Asp-GTC | ACGCGGGAGACCGGGGUUCAAUUCCCCGACGGGGAGCC | 38 | 91 |
| tRNA-Asp-GTC | ACGCGGGAGACCGGGGUUCGUUUCCCCGACGGGGAGCC | 38 | 92 |
| tRNA-Asp-GTC | ACGCGGGAGACCGGGGUUCGGUUCCCCGACGGGGAGC | 37 | 93 |
| tRNA-Asp-GTC | CACGCGGGAGACCGGGGUUCAAUUCCCCGACGGGGAGCC | 39 | 94 |
| tRNA-Asp-GTC | GCGGGAGACCGGGGUUCGGUUCCCCGACGGGGAGC | 35 | 95 |
| tRNA-Asp-GTC | CACGCGGGAGACCGGGGUUCGUUUCCCCGACGGGGAGCC | 39 | 96 |
| tRNA-Glu-CTC | UCCCUGGUGGUCUAGUGGUUAGGAUUCGGCGC | 32 | 97 |
| tRNA-Glu-CTC | UCCCUGGUGGUCUAGUGGUUAGGAUUCGGCGCU | 33 | 98 |
| tRNA-Glu-CTC | UCCCUGGUGGUCUAGUGGUUAGGAUUCGGCGCUCU | 35 | 99 |
| tRNA-Glu-CTC | UCCCUGGUGGUCUAGUGGUUAGGAUUCGGCGCUC | 34 | 100 |
| tRNA-Glu-CTC | UCCCUGUGGUCUAGUGGUUAGGAUUCGGCGC | 31 | 101 |
| tRNA-Glu-CTC | UCCCUGUGGUCUAGUGGUUAGGAUUCGGCGCU | 32 | 102 |
| tRNA-Glu-CTC | UCCCUGGUGGUCUAGUGGUUAGGAUUCGGCG | 31 | 103 |
| tRNA-Glu-CTC | UCCCUGUGGUCUAGUGGUUAGGAUUCGGCGCUCU | 34 | 104 |
| tRNA-Glu-CTC | UCCCUGGUGGUCUAGUGGUUAGGAUUCGG | 29 | 105 |
| tRNA-Glu-CTC | UCCCUGUGGUCUAGUGGUUAGGAUUCGGCGCUC | 33 | 106 |
| tRNA-Glu-CTC | CCCCUGUGGUCUAGUGGUUAGGAUUCGGCGCU | 32 | 107 |
| tRNA-Glu-CTC | UCCCUGUGGUCUAGUGGUUAGGAUUCGGCG | 30 | 108 |
| tRNA-Glu-CTC | UCCCUGUGGUCUAGUGGUUAGGAUUCGG | 28 | 109 |
| tRNA-Glu-CTC | CCCUGGUGGUCUAGUGGUUAGGAUUCGGCGCUCU | 34 | 110 |
| tRNA-Glu-CTC | CCCCUGUGGUCUAGUGGUUAGGAUUCGGCGCUCU | 34 | 111 |
| tRNA-Glu-CTC | CCCCUGUGGUCUAGUGGUUAGGAUUCGGCGCUC | 33 | 112 |
| tRNA-Glu-CTC | UCCCUGGUGGUCUAGUGGU | 19 | 113 |
| tRNA-Glu-CTC | CCCUGGUGGUCUAGUGGU | 18 | 114 |
| tRNA-Glu-TTC | UCCCUGGUGGUCUAGUGGCUAGGAUUCGGCGCUUU | 35 | 115 |
| tRNA-Glu-TTC | UCCCACAUGGUCUAGCGGUUAGGAUUCCUGGUUUU | 35 | 116 |
| tRNA-Glu-TTC | UCCCUGUGGUCUAGUGGCUAGGAUUCGGCGCUUU | 34 | 117 |
| tRNA-Lys-CTT | UCGGUAGAGCAUGAGACU | 18 | 118 |
| tRNA-Lys-CTT | UCGGUAGAGCAUGAGAC | 17 | 119 |

(continued)

| tRNA_name | Sequence | Length | SEQ ID No. |
|---|---|---|---|
| tRNA-Lys-CTT | UCGGUAGAGCAUGAGACUC | 19 | 120 |
| tRNA-Lys-CTT | AGGGUCGUGGGUUCGUGCCCCACG | 24 | 121 |
| tRNA-Lys-CTT | AGGGUCGUGGGUUCGGGCCCCACGUUGGGCGC | 32 | 122 |
| tRNA-Gly-TCC | GCGUUGGUGGUAUAGUGGUUAGCAUAGCUGCCUU | 34 | 123 |
| tRNA-Gly-TCC | GCGUUGGUGGUAUAGUGGUGAGCAUAGCUGCCUU | 34 | 124 |
| tRNA-Gly-TCC | GCGUUGGUGGUAUAGUGGUUAGCAUAGCUGCCUUC | 35 | 125 |
| tRNA-Gly-TCC | GCGUUGGUGGUAUAGUGGUUAGCAUAGCUGCCUUCC | 36 | 126 |
| tRNA-Gly-TCC | GCGUUGGUGGUAUAGUGGUUAGCAUAGCUGCCU | 33 | 127 |
| tRNA-Gly-TCC | GCGUUGGUGGUAUAGUGGUUAGCAUAGCUG | 30 | 128 |
| tRNA-Gly-TCC | GCGUUUGUGGUAUAGUGGUGAGCAUAGCUGCCUU | 34 | 129 |
| tRNA-Gly-TCC | GCGUUUGUGGUAUAGUGGUUAGCAUAGCUGCCUU | 34 | 130 |
| tRNA-Gly-TCC | GCGUUGUGGUAUAGUGGUUAGCAUAGCUGCCUU | 33 | 131 |
| tRNA-Gly-TCC | GCGUUGUGGUAUAGUGGUGAGCAUAGCUGCCUU | 33 | 132 |
| tRNA-Gly-TCC | GCGUUUGUGGUAUAGUGGUUAGCAUAGCUGCCUUCC | 36 | 133 |
| tRNA-Gly-TCC | GCGUUGUGGUAUAGUGGUUAGCAUAGCUGCCUUCC | 35 | 134 |
| tRNA-Gly-TCC | GCGUUGUGGUAUAGUGGUUAGCAUAGCUGCCUUC | 34 | 135 |
| tRNA-Gly-TCC | GCGUUUGUGGUAUAGUGGUUAGCAUAGCUGCCU | 33 | 136 |
| tRNA-Gly-TCC | GCGUUGUGGUAUAGUGGUUAGCAUAGCUGCCU | 32 | 137 |
| tRNA-Gly-GCC | ACGCGGGAGGCCCGGGUUC | 19 | 138 |
| tRNA-Arg-TCT | AGCGCAUUGGAUUUC | 15 | 139 |
| tRNA-Arg-TCT | AGCGCAUUGGAGUUC | 15 | 140 |
| tRNA-Gln-CTG | AAUCCAGCGAUCCGAGUUC | 19 | 141 |
| tRNA-Gln-CTG | CUCGGUGGAACCUCC | 15 | 142 |
| tRNA-Leu-AAG | GGUAGCGUGGCCGAGCGGUCUAAGGCUC | 28 | 143 |
| tRNA-Leu-AAG | GGUAGCGUGGCCGAGCGGUCUAAGGCUCU | 29 | 144 |
| tRNA-Leu-AAG | GGUAGCGUGGCCGAGCGGUCUAAGGCC | 27 | 145 |
| tRNA-Leu-AAG | GGUAGCGUGGCCGAGCGGUCUAAGGCUCUGGAU | 33 | 146 |
| tRNA-Leu-AAG | GGUAGCGUGGCCGAGCGGUCUAAGGCUCUGGAUU | 34 | 147 |
| tRNA-Leu-AAG | GGUAGCGUGGCCGAGCGGUCUAAGGCCU | 28 | 148 |
| tRNA-Thr-TGT | AGGGGUCGCGAGUUC | 15 | 149 |
| tRNA-Ile-AAT | GCCAAGGUCGCGGGUU | 16 | 150 |
| tRNA-Pro-TGG | GGGUGCGAGAGGUCCCGGGUUC | 22 | 151 |
| tRNA-Pro-TGG | GGGUGCGAGAGGUCCCGGGUU | 21 | 152 |
| tRNA-Ser-CGA | GCUGUGAUGGCCGAGUGGUU | 20 | 153 |
| tRNA-Cys-GCA | AAGAGGUCCCCGGUU | 15 | 154 |
| tRNA-Cys-GCA | AAGAGGUCCCCGGUUC | 16 | 155 |
| tRNA-Met-CAT | AUAAUCUGAAGGUCGUGAGUU | 21 | 156 |
| tRNA-Met-CAT | CAUAAUCUGAAGGUC | 15 | 157 |
| tRNA-Trp-CCA | AGAAGGUUGCGUGUUC | 16 | 158 |

(continued)

| tRNA_name | Sequence | Length | SEQ ID No. |
|---|---|---|---|
| tRNA-Trp-CCA | GAUCAGAAGGUUGCGUGUUC | 20 | 159 |
| tRNA-Trp-CCA | AUCAGAAGGUUGCGU | 15 | 160 |
| tRNA-Trp-CCA | AGAAGGUUGCGUGUU | 15 | 161 |
| tRNA-Lys-CTT | UCGGUAGAGCAUGGGACUC | 19 | 162 |
| tRNA-Lys-CTT | AGGGUCGUGGGUUCGGGCCCCACG | 24 | 163 |
| tRNA-Lys-CTT | UCGGUAGAGCAUGAG | 15 | 134 |
| tRNA-Thr-CGT | AGGAGAUCCUGGGUUC | 16 | 165 |
| tRNA-Thr-TGT | CAGGGGUCGCGAGUUC | 16 | 166 |
| tRNA-Asn-GTT | AACCGAAAGGUUGGUGGUUC | 20 | 167 |
| tRNA-Lys-TTT | AGUCGGUAGAGCAUC | 15 | 168 |
| tRNA-Cys-GCA | AAGAGGUCCCUGGUUC | 16 | 169 |
| tRNA-Cys-GCA | CAAGAGGUCCCCGGUUC | 17 | 170 |
| tRNA-Met-CAT | CAUAAUCUGAAGGUCGUGAGUUC | 23 | 171 |
| tRNA-Met-CAT | CUGAAGGUCGUGAGUUC | 17 | 172 |

Having established that 3'P tRFs are differentially expressed in cSCC in-vitro model, we investigated their presence in human plasma samples. To this end, we analyzed by Dart-RNAseq, plasma samples from 9 healthy donors and 9 cSCC patients. The 3'P RNA molecular markers of cSCC in plasma identified are listed in table 24 below.

**Table 24**

| tRNA_name | Sequence | Length | SEQ ID No. |
|---|---|---|---|
| tRNA-Ala-AGC | GGGGGUGUAGCUCAGUGGUAGAGCGCGUGCU | 30 | 218 |
| tRNA-Asp-GTC | ACGCGGGAGACCGGGGUUCGAUUCCCCGACGGGGAGCCA | 39 | 219 |
| tRNA-Glu-CTC | UCCCUGGUGGUCUAGUGGUUAGGAUUCGGCGCUCUC | 36 | 220 |
| tRNA-Gly-GCC | GCAUUGGUGGUUCAGUGGUAGAAUUCUCGCCUGCC | 35 | 221 |

<u>**Materials and Methods**</u>

***Mouse liver tissues***

Liver tissues were collected as early symptomatic (postnatal day 5 - P5) from "Taiwanese" mouse model of severe SMA. Phenotypically normal littermates (Smn±; SMN2tg/0) were used as controls. After dissection, liver tissues were snap-frozen and stored at -80 °C until use.

***Cell lines***

A cell line of a squamous cell carcinoma of human skin (HSC-1, accession number JCRB1015) was purchased from JRB (https://cellbank.nibiohn.gojp/english/) and cultured in a 10 cm plate in Dulbecco's modified Eagle's medium with 20 % fetal bovine serum. Cells were harvested after treatment with 0.02% EDTA and 0.05% trypsin for three minutes; atmosphere air 95%, carbon dioxide 5% (CO2). Subculture cells every 2 weeks.

Human Epidermal Keratinocytes were purchased from ATCC (cat. PCS-200-011). Human epidermal keratinocytes were cultured according to providers' instructors.

Human derived fibroblast for SMA experiment were purchased from Coriell Institute (see table 25 below) and cultured according to providers' instructions. Fibroblast were treated with nursinersen (Biogen) or risdiplam (Sanbio, cat. N °29028-1). Treatment with nusinersen and risdiplam were performed at 75-80% of confluency. For nursinersen treatment, the drug was used at final concentration of 10 nM and transfected with Lipofectamine™ LTX Reagent with PLUS™ Reagent kit (Invitrogen, cat. n°A12621) following manufacturer's recommendations . For risdiplam treatment, cells were treated at a

final concentration of 0.5 μM. The treatment was repeated every 24 hours for 2 times.

**Table 25**

| Fibroblasts ID | SMA subtype |
|---|---|
| **GM00232** | SMA I |
| **GM09677** | SMA I |
| **GM03813** | SMA II |
| **GM22592** | SMA II |
| **GM03814** | Carrier |
| **GM03815** | Carrier |
| **GM05659** | healthy |

**Human plasma**

Patients plasma were purchased at Proteogenex (Proteogenex, Inc. California, USA). All blood products are collected under IRB approval by certified phlebotomists and blood processing is done under strict Standard Operating Procedures (confidential document available upon request). Below the list of healthy donors and SCC patients.

**Table 26**

| Sample ID | Sex | Age | Histological diagnosis | Grade | TNM | Stage |
|---|---|---|---|---|---|---|
| 181639P | F | 91 | squamous cell carcinoma | G2 | T3NOMO | III |
| 181664P | M | 63 | squamous cell carcinoma | G3 | T3NOMO | III |
| 181767P | M | 74 | squamous cell carcinoma | G1 | T3NOMO | III |
| 182248(II)P | M | 83 | squamous cell carcinoma | G2 | T3NOMO | III |
| 181819P | M | 59 | squamous cell carcinoma | G2 | T3NOMO | III |
| 181842P | M | 54 | squamous cell carcinoma | G1 | T3NOMO | III |
| 181922P | M | 55 | squamous cell carcinoma | G2 | T3NOMO | III |
| 181942P | M | 61 | squamous cell carcinoma | G2 | T3NOMO | III |
| 181948P | M | 62 | squamous cell carcinoma | G2 | T2NOMO | II |
| D12690P | M | 72 | normal donor | n.d | n.d | n.d |
| D12745P | M | 60 | normal donor | n.d | n.d | n.d |
| D12749P | M | 64 | normal donor | n.d | n.d | n.d |
| D12756P | M | 58 | normal donor | n.d | n.d | n.d |
| D12760P | M | 67 | normal donor | n.d | n.d | n.d |
| D12761P | M | 63 | normal donor | n.d | n.d | n.d |
| D12763P | M | 69 | normal donor | n.d | n.d | n.d |
| D12802P | M | 70 | normal donor | n.d | n.d | n.d |
| D12809P | M | 67 | normal donor | n.d | n.d | n.d |

***RNA extraction***

Total RNA and small RNA enrichment was performed by MirVana Kit (ThermoFisher cat n. AM1561) according to manufacturer's instructions. Briefly, mouse liver tissues were pulverized using a mortar and pestle under liquid nitrogen. The powder was then transferred in a 1.5 mL tube, where cells were disrupted by adding Mirvana lysis buffer and miRNA additive, followed by column-purification. Cell lines were lysed and processed according to MirVana Kit specifications. After RNA purification, total RNA was quantified by Nanodrop, and small RNA fraction was quantified by QuBit miRNA

assay (ThermoFisher cat. Q32880). RNA integrity was checked by Total RNA nano chip (Agilent cat n. 5067-1511).

***Dart-RNAseq analysis***

*5'phosphorylation and adapter ligation*

Small RNA fractions were used as input for library preparation. In particular, 500 ng of small RNA were subjected to 5' phosphorylation with T4 PNK 3' minus (NEB, cat no. M0236S), according to manufacturer's instructions. Small RNAs were purified using RNA Clean & Concentrator™-5 column (Zymo Research, cat. no. R1013) and ligated to an RNA adapter, via RtcB (NEB cat. N° M0458S), according to the following conditions: 500 ng of small RNA, 0.7 pmol of adapter, 15 pmol RtcB, 1x RtcB Buffer (50 mM Tris-HCl, 75 mM KCl, 10mM DTT), 150 μM GTP, 1.8 mM mM $MnCl_2$ in a final volume of 10 μl. The reaction was incubated 1 h at 37°C and then purified by RNA Clean & Concentrator™-5 column. The RNA-based adapter (RNA-based adapter, listed in Table 24) includes (i) part of SP1 sequence necessary for Illumina sequencing, (ii) 8 degenerated nucleotide used as unique molecular identifiers (UMIs), (iii) 3 abasic sites, that allow for RT enzyme stop and generation of single strand cDNA, and (iv) a final fluoro-uridine that prevents RNAse degradation.

*Circularization*

The circularization of the adapter-ligated RNA (RNA:adapter) was carried out at 25°C for 2 h, in a total volume of 20 μl containing 10 U of T4 RNA Ligase 1 (NEB, cat. no. M0204L), 1× T4 RNA ligase buffer (50 mM Tris-HCl, 1 mM $MgCl_2$, 1 mM DTT), 20% PEG8000, 50 μM ATP. Circular RNA was purified by using RNA Clean & Concentrator™-5 column (Zymo research, cat. no. R1013).

*RT and PCR amplification*

For the generation of single strand cDNA, circular RNA was subjected to reverse transcription using Superscript III enzyme (Thermo Fisher cat. N° 18080093) according to the following conditions: 200 uM dNTPs mix, 10 uM RT primer (listed in table 25), 1x RT buffer, 5 mM DTT. The RT primer include full SP2 sequence necessary for Illumina sequencing and 4 degenerated nucleotides for UMIs. The mix was incubated at 70°C for 5 min to allow circular RNA denaturation, followed by 2 min on ice, 40 min at 50°C and 5 min at 80°C to heat inactivate the RT enzyme. After linear single strand cDNA formation, RT reaction mix was amplified by two PCR steps. The first PCR amplification led to cDNA amplification and inclusion of full SP1 sequence by forward primer. The second PCR amplification step is required for integration of Unique dual indexes (UDIs) adapter needed for Illumina sequencing.

Briefly, first PCR step was performed according to following conditions: 20 uL of RT reaction, 0.8 uM SP1 Fw primer and 0.8 SP2 rev primer, 1x Phusion high-fidelity master mix (Thermo Fisher, cat. N° F531S), in a final volume of 100 uL. PCR mix was amplified in 0.2 tube in a thermocycler as follow: 1min 98°C, 8x cycles at 98°C for 30 sec, 61°C for 30 sec, 72°C for 10 sec. The reaction was then purified by 1.6x volume Agencourt AMPure XP beads (Agencort, cat. N° A63882) according to manufacturer's instruction.

Purified DNA was used for second PCR step: 40 uL of PCR 1, 1.5 uM UDIs adapter (Eurofins, set n°48/1), 1x Phusion high-fidelity master mix (Thermo Fisher, cat. N° F531S), in a final volume of 100 uL. PCR mix was amplified in 0.2 tube in a thermocycler as follow: 1min 98°C, 6x cycles at 98°C for 30 sec, 60°C for 30 sec, 72°C for 10 sec. The reaction was then purified by NucleoSpin Gel and PCR CleanUp kit. All the sequence used for Dart-RNAseq library preparation are listed in table 24 (SEQ ID No.: 173-184).

*Library gel purification and sequencing*

The final library was loaded on 10% TBE-gel (Thermo Fisher, cat n°EC6275BOX), run at 200 V for 1h, stained with Sybr™ Gold (Invitrogen, cat. no. S11494) and scanned using Chemidoc (GE Healthcare, Piscataway, NJ). To remove adapter dimer contamination, the correct band at 200 nt was isolated from the gel, crushed and soaked overnight in Buffer II (Immagina Biotechnology srl, cat. no. #KGE002) at room temperature with constant rotation. The aqueous gel debris was filtered with Millipore ultrafree MC tubes and then precipitated with isopropanol (Sigma, cat. no. I9516) at -80°C for 2 h or overnight. After precipitation, samples were centrifuged for 30 min at 12 000 g, 4°C. The pellet was washed once with 70% ethanol, centrifuged at 12 000 g for 5 min at 4°C, air-dried and resuspended in 12 uL of nuclease free water. To evaluate the correct length, each size selected library was checked by Agilent 2100 Bioanalyzer using the Agilent High Sensitivity DNA Kit, while a qPCR using P5 and P7 primers was used for high accurate library quantification. The final pool was sequenced with 100 cycles single-read on an Illumina Novaseq.

*NGS data analysis*

NGS data obtained from cell line or mouse liver tissues were trimmed with Cutadapt by removing 3' terminal adapter. UMIs were extracted using UMI-tools extract (Smith, 2017). Trimmed reads of length under 10 nucleotides were discarded. The remaining reads were then aligned to the correspondent genome. The generated BAM file, used for following analysis using tRAX pipeline (Holmes AD et al 2022) published on bioRxiv (a free online archive and distribution service for unpublished preprints in the life sciences). Differential expression analysis was performed using DEseq2[41] .

Hits from differential expression analysis were selected according to the following filters:

- Read counts: ≥ 200 counts
- Dart-RNAseq Log2 FC: ≥ 1 for upregulated and ≤ -1 for downregulated compared with control or not treated sample.
- Dart-RNAseq p-val: ≤ 0.05, or any statistical parameter that ensure a statistically robust threshold among samples.
- cleavage pattern: the fragment of interest has to show clear cleavage site, i.e., ≤ 40% per-base cleavage frequencies along fragment length and ≥ 60% per-base cleavage frequencies on the 5' and 3' termini of the fragment.
- Multimapping score:

    o (For all fragments): only fragments with ≤ 100 multimap are accepted
    o (for tRNA fragments): only fragments with ≤ 100 multimap are accepted. Of those we further classify tRFs as follow:

        ▪ transcript specific (reads that uniquely map to the corresponding tRNA transcript sequence);
        ▪ isodecoder specific (reads that map uniquely to the transcripts with the corresponding anticodon);
        ▪ isotype specific (reads that map only to transcripts of the corresponding tRNA isotype);
        ▪ not amino specific (reads that map to more than one tRNA isotype)
        ▪ In particular the following tRFs are preferred: transcript specific > isodecoder specific > isotype specific. Not amino specific are not taken into consideration.

***3'P-qPCR***

3'P-qPCR shared all the steps of dart-RNAseq analysis until retro-transcription, but it uses a different RNA-based adapter. The minimum amount of small RNA input material tested was 100 ng (quantified by QuBit miRNA assay). Specific RNA-based adapter and RT primer used for 3'P-qPCR are listed in table 27. For 3'P-qPCR amplification, each couple of primers were designed according to following rules:

- Forward and reverse primers have a length ranging between 15 and 23 nt and must be designed at the junction between the adapter and the 3'P RNA of interest, to maintain the specificity at 5' and 3' end of the 3'P RNA.
- Forward and reverse primers should anneal for at least 6 nt with the 3'P RNA of interest, to confer sequence specificity.
- The melting temperature should range between 58° and 63°C, with maximum 1.5° of differences between forward and reverse primer for each couple.
- Forward and reverse primers should have minimum secondary structure and no possibility for hetero- and homo-dimer formation.

The list of primers used for 3'P RNA fragments validation are listed in table 28. All the qPCR amplification were performed by SYBR™ Green PCR Master Mix (Thermo Fisher, cat. N°: 4309155). Ct values for each 3'P RNA are normalized using the total amount of RNA-based adapter. Primers for normalization are listed in table 28.

**Table 27**

| Oligo name | Sequence (5'-3') | Step | Note | SEQ ID No. |
|---|---|---|---|---|
| RNA-based adapter | **NNNN**UCUCCUUGCAUAAUCACCAACCAU/idSp//idSp//idSp/ACACGACGCUCUUCCGAUCU**NNNN**/*3FU* | dart-RNA sequencing | RNA-based adapter | 173 |
| Linker_MC1 | **NNNN**UCUCCUUGCAUAAUCACCAACCAU/idSp//idSp//idSp/ACACGACGCUCUUCCGAUCU**NNNN** *guaccuug*/*3FU* | dart-RNA sequencing | RNA-based adapter barcode 1 | 174 |

(continued)

| Oligo name | Sequence (5'-3') | Step | Note | SEQ ID No. |
|---|---|---|---|---|
| Linker_MC2 | **NNNN**UCUCCUUGCAUAAUCACCAACCAU/idSp//idSp//idSp/ACACGACGCUCUUCCGAUCU**NNNN***uaaugccg*/*3FU* | dart-RNA sequencing | RNA-based adapter barcode2 | 175 |
| Linker_MC3 | **NNNN***ugacugac*UCUCCUUGCAUAAUCACCAACCAU/idSp//idSp//idSp/ACACGACGCUCUUCCGAUCU**NNNN**/*3FU* | dart-RNA sequencing | RNA-based adapter Barcode3 | 176 |
| Linker_MC4 | **NNNN***guaccuug*UCUCCUUGCAUAAUCACCAACCAU/idSp//idSp//idSp/ACACGACGCUCUUCCGAUCU**NNNN**/*3FU* | dart-RNA sequencing | RNA-based adapter barcode4 | 177 |
| RT_ primer | GTGACTGGAGTTCAGACGTGTGCTCTTCCGATCT**NNNN**GGTTGGTGATTATGCAAGGAG | dart-RNA sequencing | DNA RT primer | 178 |
| Fw PCR 1 | ACACTCTTTCCCTACACGACGCTCTTCCGATCT | dart-RNA sequencing | DNA primer (PCR 1) | 179 |
| Rev PCR 1 | GTGACTGGAGTTCAGACGTGT | dart-RNA sequencing | DNA primer (PCR 1) | 180 |
| Fw PCR 2 | AATGATACGGCGACCACCGAGATCTACAC**(i5)**ACACTCTTTCCCTACACGACGCTCTTCCGATCT | dart-RNA sequencing | DNA primer (PCR 2) | 181 and 182 |
| Rev PCR 2 | CAAGCAGAAGACGGCATACGAGAT**(i7)**GTGACTGGAGTTCAGACGTGTGCTCTTCCGATCT | dart-RNA sequencing | DNA primer (PCR 2) | 183 and 184 |
| Linker_qPCR | UCUCCUUGCAUAAUCACCAACCAU/idSp//idSp//idSp/GAUGGAAGACGCCAAAAACAU | 3'P-qPCR | RNA-based adapter | 185 |
| RT_3'qPCR | GTGACTGGAGTTCAGACGATGGTTGGTGATTATGC | 3'P-qPCR | DNA RT primer | 186 |
| - N are ribonucleotides that represents UMIs for PCR duplication removal after sequencing.<br>- Underlined sequence in RNA-based adapter, linker_MC1, linker_MC2, linker_MC3, linker_MC4 correspond to part of SP1 sequence (the first portion of the first nucleic acid domain PR1)<br>- idSp: abasic sites to allow RT stop<br>- *3FU:* Fluoro_Uridine, to stabilize RNA from RNAse degradation.<br>- Italic lowercase: 8nt barcode for multiplexing | | | | |

Table 28

| Primer | 5'-3' | Target | organism | SEQ ID No. |
|---|---|---|---|---|
| Fw_Gm22973/U2 | AGACGCCAAAAACATAAATGGA | 3'P_Gm22973/U2 fragment | mouse | 187 |
| Rev_Gm22973 | TATGCAAGGAGACTCCTACT**C** | 3'P_Gm22973 fragment | mouse | 188 |

(continued)

| Primer | 5'-3' | Target | organism | SEQ ID No. |
|---|---|---|---|---|
| Rev_U2 | TATGCAAGGAGACTCCTACT **T** | 3'P_U2 fragment | mouse | 189 |
| Fw_ValAAC/CAC | AAAACATACGCGAAAGGTC C | 3'P_ValAAC/CAC fragment | mouse | 190 |
| Rev_ValAAC/CAC | TGCAAGGAGAGGTGTTTCC | 3'P_ValAAC/CAC fragment | mouse | 191 |
| Fw_ValTAC | AAAACATACGCAGAAGGTC CT | 3'P_ValTAC fragment | mouse | 192 |
| Rev_ValTAC | AAGGAGAGGTGGTTCCACT | 3'P_ValTAC fragment | mouse | 193 |
| Fw_Glu_CTC | AAAACATTCCCTGGTGGTC | 3'P_Glu_CTC_fragm ent | Human skin | 194 |
| Rev_Glu_CTC | CAAGGAGAAGCGCCGAAT | 3'P_Glu_CTC_fragm ent | Human skin | 195 |
| Fw_Asp_GTC | AAAACATACGCGGGAGACC | 3'P_Asp_GTC_fragm ent | Human skin | 196 |
| Rev_Asp_GTC | AAGGAGAGCTCCCCGTC | 3'P_Asp_GTC_fragm ent | Human skin | 197 |
| Fw Val_AAC | CCAAAAACATGTTTCCGTAG | 3'P_ValAAC fragment | Human fibroblast | 198 |
| Rev Val_AAC | TATGCAAGGAGATGATAAC C | 3'P_ValAAC fragment | Human fibroblast | 199 |
| Fw Leu_TAA | GCCAAAACATACCGGGAT | 3'P_Leu_TAA fragment | Human fibroblast | 200 |
| Rev Leu_TAA | TATGCAAGGAGACCACTC | 3'P_Leu_TAA fragment | Human fibroblast | 201 |
| Fw Gly_TCC | CCAAAAACATGCGTTTGTG | 3'P_Gly_TCC fragment | Human fibroblast | 202 |
| Rev Gly_TCC | CAAGGAGAGAAGGCAGCT | 3'P_Gly_TCC fragment | Human fibro-blast | 203 |
| Fw_linker | GATGGAAGACGCCAAAAAC A | Linker_qPCR | Data normali-zation | 204 |
| Rev_linker | GTGACTGGAGTTCAGACGA | Linker qPCR | Data normalization | 205 |
| Fw_Glu_CTC_36 | CCAAAACATTCCCTGGTGGT C | 3'P_Glu_CTC_fragm ent 36 | Human plasma | 222 |
| REV_Glu_CTC_36 | AAGGAGAGAGAGCGCCGAA | 3'P_Glu_CTC_fragm ent 36 | Human plasma | 223 |
| Fw_Ala_AGC | CCAAAACATGGGGGTGTAG C | 3'P_Ala_AGC_fragm ent | Human plasma | 224 |
| Rev_Ala_AGC | AAGGAGAGCACGCGCT | 3'P_Ala_AGC_fragm | Human plasma | 225 |

**References**

1. Slamon, D. J. et al. Use of chemotherapy plus a monoclonal antibody against HER2 for metastatic breast cancer that overexpresses HER2. N Engl J Med 344, 783-92 (2001).
2. Goossens, N., Nakagawa, S., Sun, X. & Hoshida, Y. Cancer biomarker discovery and validation. Transl Cancer Res 4, 256-269 (2015).
3. Sparano, J. A. et al. Adjuvant Chemotherapy Guided by a 21-Gene Expression Assay in Breast Cancer. New England Journal of Medicine 379, 111-121 (2018).
4. Wang, K. et al. Circular RNA mediates cardiomyocyte death via miRNA-dependent upregulation of MTP18 expression. Cell Death Differ 24, 1111-1120 (2017).
5. Honda, S. et al. Sex hormone-dependent tRNA halves enhance cell proliferation in breast and prostate cancers. Proceedings of the National Academy of Sciences 112, E3816-E3825 (2015).
6. Tang, J. et al. A novel biomarker Linc00974 interacting with KRT19 promotes proliferation and metastasis in hepatocellular carcinoma. Cell Death Dis 5, e1549-e1549 (2014).
7. Pardini, B., Sabo, A. A., Birolo, G. & Calin, G. A. Noncoding RNAs in Extracellular Fluids as Cancer Biomarkers: The New Frontier of Liquid Biopsies. Cancers (Basel) 11, 1170 (2019).
8. Paik, S. et al. Gene Expression and Benefit of Chemotherapy in Women With Node-Negative, Estrogen Receptor-Positive Breast Cancer. Journal of Clinical Oncology 24, 3726-3734 (2006).
9. Roundtree, I. A., Evans, M. E., Pan, T. & He, C. Dynamic RNA Modifications in Gene Expression Regulation. Cell 169, 1187-1200 (2017).
10. Frye, M., Harada, B. T., Behm, M. & He, C. RNA modifications modulate gene expression during development. Science (1979) 361, 1346-1349 (2018).
11. Meyer, K. D. & Jaffrey, S. R. Rethinking m 6 A Readers, Writers, and Erasers. Annu Rev Cell Dev Biol 33, 319-342 (2017).
12. Zhou, J. et al. Dynamic m6A mRNA methylation directs translational control of heat shock response. Nature 526, 591-594 (2015).
13. Shigematsu, M., Morichika, K., Kawamura, T., Honda, S. & Kirino, Y. Genome-wide identification of short 2',3'-cyclic phosphate-containing RNAs and their regulation in aging. PLoS Genet 15, e1008469 (2019).
14. Shigematsu, M., Kawamura, T. & Kirino, Y. Generation of 2',3'-Cyclic Phosphate-Containing RNAs as a Hidden Layer of the Transcriptome. Front Genet 9, (2018).
15. Sidrauski, C. & Walter, P. The Transmembrane Kinase Irelp Is a Site-Specific Endonuclease That Initiates mRNA Splicing in the Unfolded Protein Response. Cell 90, 1031-1039 (1997).
16. Trotta, C. R. et al. The Yeast tRNA Splicing Endonuclease: A Tetrameric Enzyme with Two Active Site Subunits Homologous to the Archaeal tRNA Endonucleases. Cell 89, 849-858 (1997).
17. Zhu, L. et al. Exosomal tRNA-derived small RNA as a promising biomarker for cancer diagnosis. Mol Cancer 18, 74 (2019).
18. Maute, R. L. et al. tRNA-derived microRNA modulates proliferation and the DNA damage response and is down-regulated in B cell lymphoma. Proceedings of the National Academy of Sciences 110, 1404-1409 (2013).
19. Goodarzi, H. et al. Endogenous tRNA-Derived Fragments Suppress Breast Cancer Progression via YBX1 Displacement. Cell 161, 790-802 (2015).
20. Hogg, M. C. et al. 5'ValCAC tRNA fragment generated as part of a protective angiogenin response provides prognostic value in amyotrophic lateral sclerosis. Brain Commun 2, (2020).
21. Guzzi, N. et al. Pseudouridine-modified tRNA fragments repress aberrant protein synthesis and predict leukaemic progression in myelodysplastic syndrome. Nat Cell Biol 24, 299-306 (2022).
22. Schimmel, P. The emerging complexity of the tRNA world: mammalian tRNAs beyond protein synthesis. Nat Rev Mol Cell Biol 19, 45-58 (2018).
23. Yu, M. et al. tRNA-derived RNA fragments in cancer: current status and future perspectives. J Hematol Oncol 13, 121 (2020).
24. Wang, Q. et al. Identification and Functional Characterization of tRNA-derived RNA Fragments (tRFs) in Respiratory Syncytial Virus Infection. Molecular Therapy 21, 368-379 (2013).
25. Ivanov, P. et al. G-quadruplex structures contribute to the neuroprotective effects of angiogenin-induced tRNA fragments. Proceedings of the National Academy of Sciences 111, 18201-18206 (2014).
26. Boulter, N. et al. A simple, accurate and universal method for quantification of PCR. BMC Biotechnol 16, 27 (2016).
27. Pfaffl, M. W. Relative expression software tool (REST(C)) for group-wise comparison and statistical analysis of relative expression results in real-time PCR. Nucleic Acids Res 30, 36e-336 (2002).
28. Xi, X. et al. RNA Biomarkers: Frontier of Precision Medicine for Cancer. Noncoding RNA 3, 9 (2017).
29. Mercuri, E., Sumner, C. J., Muntoni, F., Darras, B. T. & Finkel, R. S. Spinal muscular atrophy. Nat Rev Dis Primers 8, 52 (2022).
30. Oberbauer, V. & Schaefer, M. tRNA-Derived Small RNAs: Biogenesis, Modification, Function and Potential Impact on Human Disease Development. Genes (Basel) 9, 607 (2018).

31. Kumar, P., Mudunuri, S. B., Anaya, J. & Dutta, A. tRFdb: a database for transfer RNA fragments. Nucleic Acids Res 43, D141-D145 (2015).
32. Kumar, P., Anaya, J., Mudunuri, S. B. & Dutta, A. Meta-analysis of tRNA derived RNA fragments reveals that they are evolutionarily conserved and associate with AGO proteins to recognize specific RNA targets. BMC Biol 12, 78 (2014).
33. Shen, Y. et al. Transfer RNA-derived fragments and tRNA halves: biogenesis, biological functions and their roles in diseases. J Mol Med 96, 1167-1176 (2018).
34. Fania, L. et al. Cutaneous Squamous Cell Carcinoma: From Pathophysiology to Novel Therapeutic Approaches. Biomedicines 9, 171 (2021).
35. Del Piano, A. et al. Phospho-RNA sequencing with circAID-p-seq. Nucleic Acids Res 50, e23-e23 (2022).
36. Clamer, M. et al. Active Ribosome Profiling with RiboLace. Cell Rep 25, 1097-1108.e5 (2018).
37. Doktor, T. K. et al. RNA-sequencing of a mouse-model of spinal muscular atrophy reveals tissue-wide changes in splicing of U12-dependent introns. Nucleic Acids Res 45, 395-416 (2017).
38. Ivanov, P., Emara, M. M., Villen, J., Gygi, S. P. & Anderson, P. Angiogenin-Induced tRNA Fragments Inhibit Translation Initiation. Mol Cell 43, 613-623 (2011).
39. Saikia, M. et al. Angiogenin-Cleaved tRNA Halves Interact with Cytochrome c, Protecting Cells from Apoptosis during Osmotic Stress. Mol Cell Biol 34, 2450-2463 (2014).
40. Singh, R. N., Howell, M. D., Ottesen, E. W. & Singh, N. N. Diverse role of survival motor neuron protein. Biochimica et Biophysica Acta (BBA) - Gene Regulatory Mechanisms 1860, 299-315 (2017).
41. Love, M. I., Huber, W. & Anders, S. Moderated estimation of fold change and dispersion for RNA-seq data with DESeq2. Genome Biol 15, 550 (2014).

## Claims

**1.** An *in vitro* or *ex vivo* method of diagnosis, prognosis, therapy monitoring or outcome prediction assessment of a disease or condition in a subject by detecting and quantifying at least one molecular marker of the disease or condition contained in a biological sample of the subject, wherein the at least one molecular marker is an RNA fragment comprising a 3' phosphate or a 2'/3' cyclic phosphate (3'P RNA), the method comprising the following steps:

(a') phosphorylating the 5' end of the at least one 3'P RNA contained in the biological sample and obtaining at least one phosphorylated RNA fragment;
(b') ligating the 3' end of the at least one phosphorylated RNA fragment to the 5' end of an RNA-based adapter obtaining at least one first ligation product, wherein the RNA-based adapter has formula (Ia):

$$5' \text{ OH-E1-}A_z\text{-E2-OH } 3' \qquad \text{(Ia)}$$

wherein

- E1 is a first oligoribonucleotide sequence having a length comprised between 15 and 30,
- A is an abasic site or a spacer allowing the arrest of a retrotranscriptase enzyme activity,
- z is an integer number from 1 to 5,
- E2 is a second oligoribonucleotide sequence having a length comprised between 15 and 30;

(c') self-ligating the at least one first ligation product to form at least one circular RNA molecule;
(d') performing a reverse transcription of the at least one circular RNA molecule obtaining at least one single strand cDNA molecule comprising the sequence of the at least one 3'P RNA, wherein the reverse transcription is carried out using a reverse transcription primer having formula (IIa):

$$5' \text{ OH-G-F 1-OH } 3' \qquad \text{(IIa)}$$

wherein

- F1 is the reverse complement deoxyoligoribonucleotide of E1, wherein complementarity of F1 to E1 is comprised between 60% and 100%, and
- G is a first DNA oligonucleotide having a length comprised between 10 and 30;

(e') performing in parallel a first and a second qPCR amplifications of the at least one single strand cDNA molecule

obtaining a first and a second amplification product, wherein:

- the first qPCR amplification is carried out using a first pair of primers annealing on at least one part of the 3'P RNA sequence, the first pair of primers being selected from pairs of primer Set1, Set2 and Set3, wherein each pair of primers comprises a forward and reverse primer, wherein the forward and reverse primers of the pair of primers Set1 have a sequence as set forth in formulas (IIIa) and (IVa), respectively, the forward and reverse primers of the pair of primers Set2 have a sequence as set forth in formulas (IIIa') and (IVa'), respectively, and the forward and reverse primers of the pair of primers Set3 have a sequence as set forth in formulas (IIIa") and (IVa"), respectively:

Set1

5' OH-H2-N-OH 3' (IIIa)

5' OH-H1-M-OH 3' (IVa)

Set2

5' OH-H2-N-OH 3' (IIIa')

5' OH-H1-OH 3' (IVa')

Set3

5' OH-H2-OH 3' (IIIa")

5' OH-H1-M-OH 3' (IVa")

wherein

❖ H1 is a second DNA oligonucleotide annealing on E1, wherein complementarity of H1 to E1 is comprised between 30% and 100%, and
❖ M is a third DNA oligonucleotide having a length comprised between 6 and 30 and annealing on at least 6 nucleotides of the 3' end of the 3'P RNA sequence,
❖ H2 is a fourth DNA oligonucleotide annealing on E2, wherein complementarity of H2 to E2 is comprised between 30% and 100%, and
❖ N is a fifth DNA oligonucleotide having a length comprised between 6 and 30 and annealing on at least 6 nucleotides of the 5' end of the 3'P RNA sequence;

- the second qPCR amplification is carried out using a second pair of primers annealing on the RNA-based adapter sequence, the second pair of primers comprising a second forward and a second reverse primer having formula (Va) and (VIa), respectively:

5' OH-H2-OH 3' (Va)

5' OH-I1-OH 3' (VIa)

wherein

❖ H2 has the meaning set forth above, and
❖ I1 is a sixth DNA oligonucleotide annealing on G, wherein complementarity of I1 to G is comprised between 60% and 100%; and

(f) repeating steps (a') to (e') on at least one control sample, wherein the control sample is a biological sample of a healthy, treated or non-treated subject;
(g') determining Ct values for the at least one 3'P RNA and for the RNA-based adapter in the first and second amplification products for either the biological sample and the control sample,

(h') calculating a 3'P-qPCR fold change of the Ct values determined in step (g') according to the following equation:

$$3'\text{P qPCR fold change} = \frac{2^{Ct(B)_{3'P}-Ct(A)_{3'P}}}{2^{Ct(B)_{adp}-Ct(A)_{adp}}} = \frac{2^{\Delta Ct(3'P)}}{2^{\Delta Ct(adp)}} = 2^{\Delta\Delta Ct}$$

wherein

- $Ct(A)_{3'P}$ is the Ct value for the 3'P RNA determined in the biological sample,
- $Ct(B)_{3'P}$ is the Ct value for the 3'P RNA determined in the control sample,
- $Ct(A)_{adp}$ is the Ct value for the RNA-based adapter determined in the biological sample,
- $Ct(B)_{adp}$ is the Ct value for the RNA-based adapter determined in the control sample;

wherein a 3'P-qPCR fold change value $\geq 2$ or $\leq 0.5$ is indicative of the diagnosis, prognosis, therapy monitoring or outcome prediction assessment of the disease or condition.

**2.** The method according to claim 1 further comprising in step (h') calculating a 3'P-qPCR p-value of the 3'P-qPCR fold change, wherein a 3'P-qPCR p-value < 0.5 is indicative of the diagnosis, prognosis, therapy monitoring or outcome prediction assessment of the disease or condition.

**3.** The method according to any one of the preceding claims, wherein the RNA-based adapter of formula (Ia) has a length comprised between 50 and 100 nt.

**4.** The method according to any one of the preceding claims, wherein the reverse transcription primer of formula (IIa) has a length comprised between 10 and 100 nt.

**5.** The method according to any one of the preceding claims, wherein the primers employed in the two qPCR amplifications of step (e') have at least one of the following features:

- they contain from 1 to 20 modified nucleotides;
- they have a length comprised between 15 and 25 nucleotides;
- they have a minimum free energy comprised between -3 and -150 kcal/mol;
- they do not contain strong or mild secondary structures and/or do not form dimers;
- they have a melting temperature comprised between 57 and 70 °C;
- they have a melting temperature difference between the forward and reverse primer of the pair less than or equal to 3 °C.

**6.** The method according to any one of the preceding claims, wherein at least one of the forward and reverse primers of the first pair of primers used in the first qPCR amplification of step (e') anneal on at least 6, preferably 10, nucleotides of the 3'P RNA at the 3' end and the 5' end, respectively.

**7.** The method according to any one of the preceding claims, wherein the biological sample is selected from urine, whole blood, saliva, plasma, skin, fibroblasts, neurons, liver, muscle, primary cell lines, immortalized cell lines, Induced Pluripotent Stem Cells (iPSC), non-human embryonic stem cells (ESCs).

**8.** The method according to any one of the preceding claims, wherein the disease is Spinal Muscular Atrophy (SMA) and the at least one molecular marker of Spinal Muscular Atrophy (SMA) is selected from 3'P RNAs having a sequence as set forth in any of SEQ ID No.: 1 - 83, 208 - 217, or a sequence having an identity equal to or higher than 90%, preferably 95%, to any of SEQ ID No.: 1 - 83, 208 - 217.

**9.** The method according to any one of the preceding claims, wherein the disease is cutaneous Squamous Cell Carcinoma (cSCC), and the at least one molecular marker of cutaneous Squamous Cell Carcinoma (cSCC)is selected from 3'P RNAs having a sequence as set forth in any of SEQ ID No.: 84 - 172, 218 - 221, or a sequence having an identity equal to or higher than 90%, preferably 95%, to any of SEQ ID No.: 84 - 172, 218 - 221.

**10.** A kit for the diagnosis, prognosis, therapy monitoring or outcome prediction assessment of the Spinal Muscular Atrophy (SMA) comprising:

(a') an RNA-based adapter having a sequence as set forth in SEQ ID No.: 185;
(b') a reverse transcription primer having a sequence as set forth in SEQ ID No.: 186;
(c') seven first pairs of primers for performing a first qPCR amplification, the first forward and reverse primers of the seven primers pairs having a sequence as set forth in the following sequence pairs: SEQ ID No.: 187 and 188, SEQ ID No.: 187 and 189, SEQ ID No.: 190 and 191, SEQ ID No.: 192 and 193, and SEQ ID No.: 198 and 199, SEQ ID No.: 200 and 201, SEQ ID No.: 202 and 203; and
(d') a second pair of primers for performing a second qPCR amplification, the second forward and second reverse primers having a sequence as set forth in SEQ ID No.: 204 and 205, respectively.

**11.** A kit for the diagnosis, prognosis, therapy monitoring or outcome prediction assessment of cutaneous Squamous Cell Carcinoma (cSCC) comprising:

(a') an RNA-based adapter having a sequence as set forth in SEQ ID No.: 185,
(b') a reverse transcription primer having a sequence as set forth in SEQ ID No.: 186,
(c') four first pairs of primers for performing a first qPCR amplification, the first forward and reverse primers of the two primers pairs having respectively a sequence as set forth in the following sequence pairs: SEQ ID No.: 194 and 195, SEQ ID No.: 196 and 197, SEQ ID No.: 222 and 223, SEQ ID No.: 224 and 225, and
(d') a second pair of primers for performing a second qPCR amplification, the second forward and second reverse primers having a sequence as set forth in SEQ ID No.: 204 and 205, respectively.

**12.** The kit according to claim 10 or claim 11, further comprising at least one of:

(e') Reagents for RNA extraction and isolation from the biological sample;
(f') A PNK enzyme;
(g') A first ligase enzyme;
(h') A second ligase enzyme;
(i') A reverse transcriptase (RT) enzyme;
(j') A qPCR Master Mix;
(k') Positive and Negative Controls to validate the RT-qPCR assay;
(l') Nuclease-free Water;
(m') Reaction Tubes and Plates;
(n') User Manual and Protocols.

## Patentansprüche

**1.** *In-vitro*- oder Ex-vivo-Verfahren zur Diagnose, Prognose, Therapieüberwachung oder Ergebnisvorhersage einer Krankheit oder eines Zustands bei einem Patienten durch Nachweis und Quantifizierung mindestens eines molekularen Markers der Krankheit oder des Zustands, der in einer biologischen Probe des Patienten enthalten ist, wobei der mindestens eine molekulare Marker ein RNA-Fragment ist, das ein 3'-Phosphat oder ein 2'/3'-zyklisches Phosphat (3'P-RNA) umfasst, wobei das Verfahren die folgenden Schritte umfasst:

(a') Phosphorylieren des 5'-Endes der mindestens einen 3'P-RNA, die in der biologischen Probe enthalten ist, und Erhalten mindestens eines phosphorylierten RNA-Fragments;
(b') Ligieren des 3'-Endes des mindestens einen phosphorylierten RNA-Fragments an das 5'-Ende eines RNA-basierten Adapters, um mindestens ein erstes Ligationsprodukt zu erhalten, wobei der RNA-basierte Adapter die Formel (Ia) hat:

$$5'\ OH\text{-}E1\text{-}A_z\text{-}E2\text{-}OH\ 3' \qquad (Ia)$$

wobei

- E1 eine erste Oligoribonukleotidsequenz mit einer Länge zwischen 15 und 30 ist,
- A eine abasische Stelle oder ein Spacer ist, der die Hemmung der Aktivität eines Retrotranskriptase-Enzyms ermöglicht,
- z eine ganze Zahl von 1 bis 5 ist,
- E2 eine zweite Oligoribonukleotidsequenz mit einer Länge zwischen 15 und 30 ist;

(c') Selbstligieren des mindestens einen ersten Ligationsprodukts, um mindestens ein zirkuläres RNA-Molekül zu bilden;
(d') Durchführen einer reversen Transkription des mindestens einen zirkulären RNA-Moleküls, um mindestens ein einzelsträngiges cDNA-Molekül zu erhalten, das die Sequenz der mindestens einen 3'-P-RNA umfasst, wobei die reverse Transkription unter Verwendung eines reversen Transkriptionsprimers der Formel (IIa) durchgeführt wird:

5' OH-G-F1-OH 3' (IIa)

wobei

- F1 das reverse Komplement-Desoxyoligoribonukleotid von E1 ist, wobei die Komplementarität von F1 zu E1 zwischen 60 % und 100 % liegt, und
- G ein erstes DNA-Oligonukleotid mit einer Länge zwischen 10 und 30 ist;

(e') paralleles Durchführen einer ersten und einer zweiten qPCR-Amplifikation des mindestens einen einzelsträngigen cDNA-Moleküls, um ein erstes und ein zweites Amplifikationsprodukt zu erhalten, wobei:

- die erste qPCR-Amplifikation unter Verwendung eines ersten Primerpaares durchgeführt wird, das an mindestens einem Teil der 3'P-RNA-Sequenz annealt, wobei das erste Primerpaar aus den Primerpaaren Set1, Set2 und Set3 ausgewählt wird, wobei jedes Primerpaar einen Vorwärts- und einen Rückwärtsprimer umfasst, wobei die Vorwärts- und Rückwärtsprimer des Primerpaares Set1 eine Sequenz gemäß den Formeln (IIIa) bzw. (IVa) aufweisen, die Vorwärts- und Rückwärtsprimer des Primerpaares Set2 eine Sequenz gemäß den Formeln (IIIa') bzw. (IVa') aufweisen und die Vorwärts- und Rückwärtsprimer des Primerpaares Set3 eine Sequenz gemäß den Formeln (IIIa") bzw. (IVa") aufweisen:

Set1

5' OH-H2-N-OH 3' (IIIa)

5' OH-H1-M-OH 3' (IVa)

Set2

5' OH-H2-N-OH 3' (IIIa')

5' OH-H1-OH 3' (IVa')

Set3

5' OH-H2-OH 3' (IIIa")

5' OH-H1-M-OH 3' (IVa")

wobei

❖ H1 ein zweites DNA-Oligonukleotid ist, das an E1 annealt, wobei die Komplementarität von H1 zu E1 zwischen 30 % und 100 % liegt, und
❖ M ein drittes DNA-Oligonukleotid mit einer Länge zwischen 6 und 30 ist und an mindestens 6 Nukleotiden des 3'-Endes der 3'-P-RNA-Sequenz annealt,
❖ H2 ein viertes DNA-Oligonukleotid ist, das an E2 annealt, wobei die Komplementarität von H2 zu E2 zwischen 30 % und 100 % liegt, und
❖ N ein fünftes DNA-Oligonukleotid mit einer Länge zwischen 6 und 30 ist und an mindestens 6 Nukleotiden des 5'-Endes der 3'-P-RNA-Sequenz annealt;

- die zweite qPCR-Amplifikation wird unter Verwendung eines zweiten Primerpaares durchgeführt, das an der RNA-basierten Adaptersequenz annealt, wobei das zweite Primerpaar einen zweiten Vorwärtsprimer und einen zweiten Rückwärtsprimer mit der Formel (Va) bzw. (VIa) umfasst:

5’ OH-H2-OH 3’ (Va)

5’ OH-I1-OH 3’ (VIa)

wobei

- H2 die oben angegebene Bedeutung hat und
- I1 ein sechstes DNA-Oligonukleotid ist, das an G annealt, wobei die Komplementarität von I1 zu G zwischen 60 % und 100 % liegt; und

(f) Wiederholen der Schritte (a’) bis (e’) an mindestens einer Kontrollprobe, wobei die Kontrollprobe eine biologische Probe eines gesunden, behandelten oder unbehandelten Probanden ist;
(g’) Bestimmen der Ct-Werte für die mindestens eine 3’-P-RNA und für den RNA-basierten Adapter in den ersten und zweiten Amplifikationsprodukten entweder für die biologische Probe oder für die Kontrollprobe,
(h’) Berechnen einer 3’-P-qPCR-Fold-Change der in Schritt (g’) bestimmten Ct-Werte gemäß der folgenden Gleichung:

$$3'\text{P qPCR fold change} = \frac{2^{Ct(B)_{3'P} - Ct(A)_{3'P}}}{2^{Ct(B)_{adp} - Ct(A)_{adp}}} = \frac{2^{\Delta Ct(3'P)}}{2^{\Delta Ct(adp)}} = 2^{\Delta\Delta Ct}$$

wobei

- $Ct(A)_{3'P}$ der Ct-Wert für die in der biologischen Probe bestimmte 3’P-RNA ist,
- $Ct(B)_{3'P}$ der Ct-Wert für die in der Kontrollprobe bestimmte 3’P-RNA ist,
- $Ct(A)_{adp}$ der Ct-Wert für den RNA-basierten Adapter ist, der in der biologischen Probe bestimmt wurde,
- $Ct(B)_{adp}$ der Ct-Wert für den RNA-basierten Adapter ist, der in der Kontrollprobe bestimmt wurde;

wobei ein 3’P-qPCR-Fold-Change-Wert ≥ 2 oder ≤ 0,5 auf die Diagnose, Prognose, Therapieüberwachung oder Ergebnisvorhersage der Krankheit oder des Zustands hinweist.

**2.** Verfahren nach Anspruch 1, das in Schritt (h’) ferner das Berechnen eines 3’P-qPCR-p-Werts der 3’P-qPCR-Fold-Change umfasst, wobei ein 3’P-qPCR-p-Wert < 0,5 für die Diagnose, Prognose, Therapieüberwachung oder Ergebnisvorhersage der Krankheit oder des Zustands indikativ ist.

**3.** Verfahren nach einem der vorstehenden Ansprüche, wobei der RNA-basierte Adapter der Formel (Ia) eine Länge zwischen 50 und 100 nt aufweist.

**4.** Verfahren nach einem der vorstehenden Ansprüche, wobei der Reverse-Transkriptionsprimer der Formel (IIa) eine Länge zwischen 10 und 100 nt aufweist.

**5.** Verfahren nach einem der vorstehenden Ansprüche, wobei die in den beiden qPCR-Amplifikationen von Schritt (e’) verwendeten Primer mindestens eines der folgenden Merkmale aufweisen:

- sie enthalten 1 bis 20 modifizierte Nukleotide;
- sie haben eine Länge zwischen 15 und 25 Nukleotiden;
- sie haben eine minimale freie Energie zwischen -3 und -150 kcal/mol;
- sie enthalten keine starken oder schwachen Sekundärstrukturen und/oder bilden keine Dimere;
- sie haben eine Schmelztemperatur zwischen 57 und 70 °C;
- sie haben eine Schmelztemperaturdifferenz zwischen dem Vorwärts- und dem Rückwärtsprimer des Paares von weniger als oder gleich 3 °C.

**6.** Verfahren nach einem der vorstehenden Ansprüche, wobei mindestens einer der Vorwärts- und Rückwärtsprimer des ersten Primerpaares, das in der ersten qPCR-Amplifikation von Schritt (e’) verwendet wird, an mindestens 6, vorzugsweise 10, Nukleotiden der 3’-P-RNA am 3’-Ende bzw. am 5’-Ende annealt.

**7.** Verfahren nach einem der vorstehenden Ansprüche, wobei die biologische Probe aus Urin, Vollblut, Speichel, Plasma, Haut, Fibroblasten, Neuronen, Leber, Muskel, primären Zelllinien, immortalisierten Zelllinien, induzierten

pluripotenten Stammzellen (iPSC) und nicht-humanen embryonalen Stammzellen (ESCs) ausgewählt ist.

**8.** Verfahren nach einem der vorstehenden Ansprüche, wobei die Krankheit spinale Muskelatrophie (SMA) ist und der mindestens eine molekulare Marker für spinale Muskelatrophie (SMA) ausgewählt ist aus 3'-P-RNAs mit einer Sequenz, wie in einer der SEQ ID Nr. 1-83, 208-217 oder einer Sequenz mit einer Identität von mindestens 90 %, vorzugsweise 95 %, zu einer der SEQ ID Nr.: 1-83, 208-217 ausgewählt ist.

**9.** Verfahren nach einem der vorstehenden Ansprüche, wobei die Erkrankung ein kutanes Plattenepithelkarzinom (cSCC) ist und der mindestens eine molekulare Marker für ein kutanes Plattenepithelkarzinom (cSCC) aus 3'-P-RNAs mit einer Sequenz ausgewählt ist, die in einer der SEQ ID Nr.: 84 - 172, 218 - 221 oder einer Sequenz mit einer Identität von mindestens 90 %, vorzugsweise 95 %, zu einer der SEQ ID Nr.: 84 - 172, 218 - 221 ausgewählt ist.

**10.** Kit zur Diagnose, Prognose, Therapieüberwachung oder Ergebnisvorhersage bei spinaler Muskelatrophie (SMA), umfassend:

(a') einen RNA-basierten Adapter mit einer Sequenz gemäß SEQ ID Nr.: 185;
(b') einen Reverse-Transkriptionsprimer mit einer Sequenz gemäß SEQ ID Nr.: 186;
(c') sieben erste Primerpaare zur Durchführung einer ersten qPCR-Amplifikation, wobei die ersten Vorwärts- und Rückwärtsprimer der sieben Primerpaare eine Sequenz aufweisen, wie in den folgenden Sequenzpaaren angegeben: SEQ ID Nr.: 187 und 188, SEQ ID Nr.: 187 und 189, SEQ ID Nr.: 190 und 191, SEQ ID Nr.: 192 und 193 und SEQ ID Nr.: 198 und 199, SEQ ID Nr.: 200 und 201, SEQ ID Nr.: 202 und 203; und
(d') ein zweites Primerpaar zur Durchführung einer zweiten qPCR-Amplifikation, wobei der zweite Vorwärtsprimer und der zweite Rückwärtsprimer eine Sequenz gemäß SEQ ID Nr.: 204 bzw. 205 aufweisen.

**11.** Kit zur Diagnose, Prognose, Therapieüberwachung oder Ergebnisvorhersage von kutanem Plattenepithelkarzinom (cSCC), umfassend:

(a') einen RNA-basierten Adapter mit einer Sequenz, wie in SEQ ID Nr.: 185 angegeben,
(b') einen Reverse-Transkriptionsprimer mit einer Sequenz gemäß SEQ ID Nr. 186,
(c') vier erste Primerpaare zur Durchführung einer ersten qPCR-Amplifikation, wobei die ersten Vorwärts- und Rückwärtsprimer der beiden Primerpaare jeweils eine Sequenz aufweisen, wie in den folgenden Sequenzpaaren angegeben: SEQ ID Nr.: 194 und 195, SEQ ID Nr.: 196 und 197, SEQ ID Nr.: 222 und 223, SEQ ID Nr.: 224 und 225, und
(d') ein zweites Primerpaar zur Durchführung einer zweiten qPCR-Amplifikation, wobei der zweite Vorwärtsprimer und der zweite Rückwärtsprimer eine Sequenz aufweisen, wie in SEQ ID Nr.: 204 bzw. 205 angegeben.

**12.** Kit gemäß Anspruch 10 oder Anspruch 11, der ferner mindestens eines der folgenden Elemente umfasst:

(e') Reagenzien zur RNA-Extraktion und -Isolierung aus der biologischen Probe;
(f) ein PNK-Enzym;
(g') ein erstes Ligaseenzym;
(h') ein zweites Ligaseenzym;
(i') ein reverses Transkriptaseenzym (RT);
(j') Ein qPCR-Mastermix;
(k') Positive und negative Kontrollen zur Validierung des RT-qPCR-Assays;
(l') Nukleasefreies Wasser;
(m') Reaktionsröhrchen und -platten;
(n') Benutzerhandbuch und Protokolle.

## Revendications

**1.** Procédé *in vitro* ou *ex vivo* de diagnostic, de pronostic, de surveillance d'une thérapie ou d'évaluation de prédiction de résultat d'une maladie ou d'une affection chez un sujet en détectant et en quantifiant au moins un marqueur moléculaire de la maladie ou de l'affection contenu dans un échantillon biologique du sujet, dans lequel le au moins un marqueur moléculaire est un fragment d'ARN comprenant un phosphate en 3' ou un phosphate cyclique en 2'/3' (ARN P 3'), le procédé comprenant les étapes suivantes consistant à :

(a’) phosphoryler l’extrémité 5’ du au moins un ARN P 3’ contenu dans l’échantillon biologique et obtenir au moins un fragment d’ARN phosphorylé ;
(b’) ligaturer l’extrémité 3’ du au moins un fragment d’ARN phosphorylé à l’extrémité 5’ d’un adaptateur à base d’ARN pour obtenir au moins un premier produit de ligature, dans lequel l’adaptateur à base d’ARN possède la formule (Ia) :

$$5' \text{ OH-E1-}A_z\text{-E2-OH } 3' \quad \text{(Ia)}$$

dans laquelle

- E1 est une première séquence d’oligoribonucléotide possédant une longueur comprise entre 15 et 30,
- A est un site abasique ou un espaceur permettant d’arrêter l’activité enzymatique d’une transcriptase inverse,
- z est un nombre entier compris entre 1 et 5,
- E2 est une deuxième séquence d’oligoribonucléotide possédant une longueur comprise entre 15 et 30 ;

(c’) auto-ligaturer le au moins un premier produit de ligature pour former au moins une molécule d’ARN circulaire ;
(d’) réaliser une transcription inverse de la au moins une molécule d’ARN circulaire pour obtenir au moins une molécule d’ADNc simple brin comprenant la séquence du au moins un ARN P 3’, dans lequel la transcription inverse est réalisée en utilisant une amorce de transcription inverse possédant la formule (IIa) :

5’ OH-G-F1-OH 3’ (IIa)

dans laquelle

- F1 est le désoxyoligoribonucléotide complément inverse de E1, où la complémentarité de F1 avec E1 est comprise entre 60 % et 100 %, et
- G est un premier oligonucléotide d’ADN possédant une longueur comprise entre 10 et 30 ;

(e’) réaliser en parallèle une première et une deuxième amplification de qPCR de la au moins une molécule d’ADNc simple brin pour obtenir un premier et un deuxième produit d’amplification, où :

- la première amplification de qPCR est réalisée en utilisant une première paire d’amorces s’annelant sur au moins une partie de la séquence d’ARN P 3’, la première paire d’amorces étant sélectionnée parmi les paires d’amorces désignées Jeu1, Jeu2 et Jeu3, où chaque paire d’amorces comprend une amorce sens et antisens, où les amorces sens et antisens de la paire d’amorces désignée Jeu1 possèdent une séquence telle que décrite dans les formules (IIIa) et (IVa), respectivement, les amorces sens et antisens de la paire d’amorces désignée Jeu2 possèdent une séquence telle que décrite dans les formules (IIIa’) et (IVa’), respectivement, et les amorces sens et antisens de la paire d’amorces désignée Jeu3 possèdent une séquence telle que décrite dans les formules (IIIa") et (IVa"), respectivement :

Jeu1

5’ OH-H2-N-OH 3’ (IIIa)

5’ OH-H1-M-OH 3’ (IVa)

Jeu2

5’ OH-H2-N-OH 3’ (IIIa’)

5’ OH-H1-OH 3’ (IVa’)

Jeu3

5’ OH-H2-OH 3’ (IIIa")

5’ OH-H1-M-OH 3’ (IVa")

dans lesquelles

- ❖ H1 est un deuxième oligonucléotide d'ADN s'annelant sur E1, où la complémentarité de H1 avec E1 est comprise entre 30 % et 100 %, et
- ❖ M est un troisième oligonucléotide d'ADN possédant une longueur comprise entre 6 et 30 et s'annelant sur au moins 6 nucléotides de l'extrémité 3' de la séquence d'ARN P 3',
- ❖ H2 est un quatrième oligonucléotide d'ADN s'annelant sur E2, où la complémentarité de H2 avec E2 est comprise entre 30 % et 100 %, et
- ❖ N est un cinquième oligonucléotide d'ADN possédant une longueur comprise entre 6 et 30 et s'annelant sur au moins 6 nucléotides de l'extrémité 5' de la séquence d'ARN P 3' ;

- la deuxième amplification de qPCR est réalisée en utilisant une deuxième paire d'amorces s'annelant sur la séquence d'adaptateur à base d'ARN, la deuxième paire d'amorces comprenant une deuxième amorce sens et une deuxième amorce antisens possédant la formule (Va) et (VIa), respectivement :

5' OH-H2-OH 3' (Va)

5' OH-I1-OH 3' (VIa)

dans lesquelles

- ❖ H2 possède la signification décrite ci-dessus, et
- ❖ I1 est un sixième oligonucléotide d'ADN s'annelant sur G, où la complémentarité de I1 avec G est comprise entre 60 % et 100 % ; et

(f') répéter les étapes (a') à (e') sur au moins un échantillon de contrôle, où l'échantillon de contrôle est un échantillon biologique d'un sujet en bonne santé, traité ou non traité ;
(g') déterminer les valeurs Ct pour le au moins un ARN P 3' et pour l'adaptateur à base d'ARN dans les premier et deuxième produits d'amplification pour l'échantillon biologique et l'échantillon de contrôle,
(h') calculer une modification en fois de la qPCR-P 3' des valeurs Ct déterminées à l'étape (g') selon l'équation suivante

$$\text{Modification en fois de la qPCR-P } 3' = \frac{2^{Ct(B)_{P\,3'}-Ct(A)_{P\,3'}}}{2^{Ct(B)_{adp}-Ct(A)_{adp}}} = \frac{2^{\Delta Ct(P\,3')}}{2^{\Delta Ct(adp)}} = 2^{\Delta\Delta Ct}$$

dans laquelle

- $Ct(A)_{P\,3'}$ est la valeur Ct pour l'ARN P 3' déterminée dans l'échantillon biologique,
- $Ct(B)_{P\,3'}$ est la valeur Ct pour l'ARN P 3' déterminée dans l'échantillon de contrôle,
- $Ct(A)_{adp}$ est la valeur Ct pour l'adaptateur à base d'ARN déterminée dans l'échantillon biologique,
- $Ct(B)_{adp}$ est la valeur Ct pour l'adaptateur à base d'ARN déterminée dans l'échantillon de contrôle ;

où une valeur de modification en fois de la qPCR-P 3' ≥ 2 ou ≤ 0,5 est indicative du diagnostic, du pronostic, de la surveillance d'une thérapie ou de l'évaluation de prédiction de résultat de la maladie ou de l'affection.

**2.** Procédé selon la revendication 1, comprenant en outre, à l'étape (h'), le calcul d'une valeur p de qPCR-P 3' de la modification en fois de la qPCR-P 3', dans lequel une valeur p de qPCR-P 3' < 0,5 est indicative du diagnostic, du pronostic, de la surveillance d'une thérapie ou de l'évaluation de prédiction de résultat de la maladie ou de l'affection.

**3.** Procédé selon l'une quelconque des revendications précédentes, dans lequel l'adaptateur à base d'ARN de formule (Ia) possède une longueur comprise entre 50 et 100 nt.

**4.** Procédé selon l'une quelconque des revendications précédentes, dans lequel l'amorce de transcription inverse de formule (IIa) possède une longueur comprise entre 10 et 100 nt.

**5.** Procédé selon l'une quelconque des revendications précédentes, dans lequel les amorces employées dans les deux

amplifications de qPCR de l'étape (e') possèdent au moins une des caractéristiques suivantes :

- elles contiennent entre 1 et 20 nucléotides modifiés ;
- elles possèdent une longueur comprise entre 15 et 25 nucléotides ;
- elles possèdent une énergie libre minimum comprise entre -3 et -150 kcal/mol ;
- elles ne contiennent pas de structures secondaires fortes ou faibles et/ou ne forment pas de dimères ;
- elles possèdent une température de fusion comprise entre 57 et 70 °C ;
- elles présentent une différence de température de fusion entre l'amorce sens et antisens de la paire inférieure ou égale à 3 °C.

**6.** Procédé selon l'une quelconque des revendications précédentes, dans lequel au moins une des amorces sens et antisens de la première paire d'amorces utilisée dans la première amplification de qPCR de l'étape (e') s'annèle sur au moins 6, de préférence 10, nucléotides de l'ARN P 3' à l'extrémité 3' et l'extrémité 5', respectivement.

**7.** Procédé selon l'une quelconque des revendications précédentes, dans lequel l'échantillon biologique est sélectionné parmi de l'urine, du sang total, de la salive, du plasma, de la peau, des fibroblastes, des neurones, le foie, un muscle, des lignées de cellulaires primaires, des lignes de cellules immortalisées, des cellules souches pluripotentes induites (CSPi), des cellules souches embryonnaires (CSE) non humaines.

**8.** Procédé selon l'une quelconque des revendications précédentes, dans lequel la maladie est l'amyotrophie spinale (SMA) et le au moins un marqueur moléculaire de l'amyotrophie spinale (SMA) est sélectionné parmi des ARN P 3' possédant une séquence telle que décrite dans l'une quelconque de SEQ ID NO: 1-83, 208-217, ou une séquence présentant une identité supérieure ou égale à 90 %, de préférence 95 %, avec l'une quelconque de SEQ ID NO: 1-83, 208-217.

**9.** Procédé selon l'une quelconque des revendications précédentes, dans lequel la maladie est le carcinome épidermoïde cutané (cSCC) et le au moins un marqueur moléculaire du carcinome épidermoïde cutané (cSCC) est sélectionné parmi des ARN P 3' possédant une séquence telle que décrite dans l'une quelconque de SEQ ID NO: 84-172, 218-221, ou une séquence présentant une identité supérieure ou égale à 90 %, de préférence 95 %, avec l'une quelconque de SEQ ID NO: 84-172, 218-221.

**10.** Kit pour le diagnostic, le pronostic, la surveillance d'une thérapie ou l'évaluation de prédiction de résultat de l'amyotrophie spinale (SMA) comprenant :

(a') un adaptateur à base d'ARN possédant une séquence telle que décrite dans SEQ ID NO: 185 ;
(b') une amorce de transcription inverse possédant une séquence telle que décrite dans SEQ ID NO: 186 ;
(c') sept premières paires d'amorces pour réaliser une première amplification de qPCR, les premières amorces sens et antisens des sept paires d'amorces possédant une séquence telle que décrite dans les paires de séquences suivantes : SEQ ID NO: 187 et 188, SEQ ID NO: 187 et 189, SEQ ID NO: 190 et 191, SEQ ID NO: 192 et 193, et SEQ ID NO: 198 et 199, SEQ ID NO: 200 et 201, SEQ ID NO: 202 et 203 ; et
(d') une deuxième paire d'amorces pour réaliser une deuxième amplification de qPCR, la deuxième amorce sens et la deuxième amorce antisens possédant une séquence telle que décrite dans SEQ ID NO: 204 et 205, respectivement.

**11.** Kit pour le diagnostic, le pronostic, la surveillance d'une thérapie ou l'évaluation de prédiction de résultat du carcinome épidermoïde cutané (cSCC) comprenant :

(a') un adaptateur à base d'ARN possédant une séquence telle que décrite dans SEQ ID NO: 185,
(b') une amorce de transcription inverse possédant une séquence telle que décrite dans SEQ ID NO: 186,
(c') quatre premières paires d'amorces pour réaliser une première amplification de qPCR, les premières amorces sens et antisens des deux paires d'amorces possédant respectivement une séquence telle que décrite dans les paires de séquences suivantes : SEQ ID NO: 194 et 195, SEQ ID NO: 196 et 197, SEQ ID NO: 222 et 223, SEQ ID NO: 224 et 225, et
(d') une deuxième paire d'amorces pour réaliser une deuxième amplification de qPCR, la deuxième amorce sens et la deuxième amorce antisens possédant une séquence telle que décrite dans SEQ ID NO: 204 et 205, respectivement.

**12.** Kit selon la revendication 10 ou la revendication 11, comprenant en outre au moins un élément parmi :

(e’) des réactifs pour l’extraction et l’isolement de l’ARN à partir de l’échantillon biologique ;
(f’) une enzyme PNK ;
(g’) une première enzyme ligase ;
(h’) une deuxième enzyme ligase ;
(i’) une enzyme transcriptase inverse (RT) ;
(j’) un mélange maître de qPCR ;
(k’) des contrôles positif et négatif pour valider l’essai de RT-qPCR ;
(l’) de l’eau exempte de nucléases ;
(m’) des tubes et plaques de réaction ;
(n’) un manuel utilisateur et des protocoles.

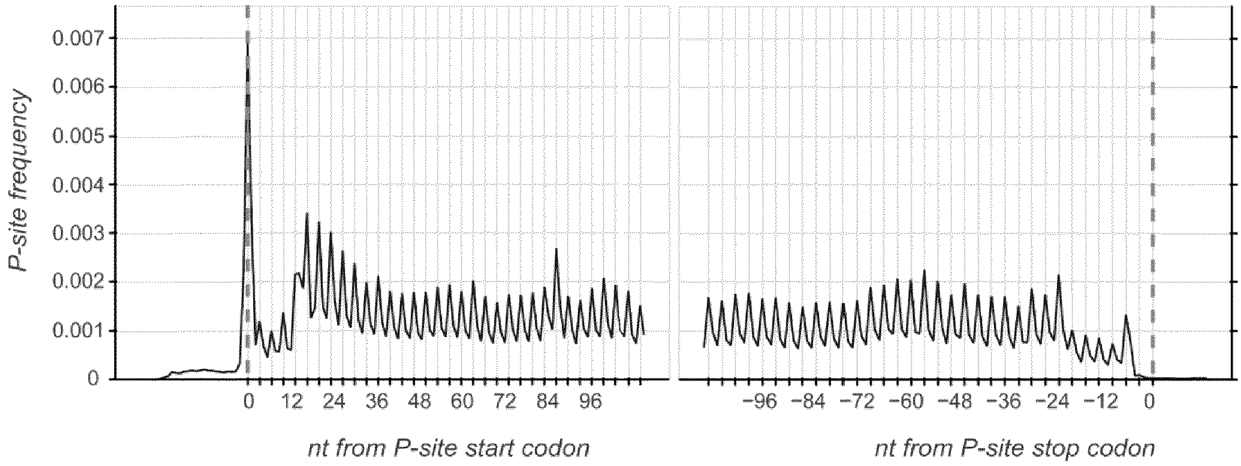


Figure 1

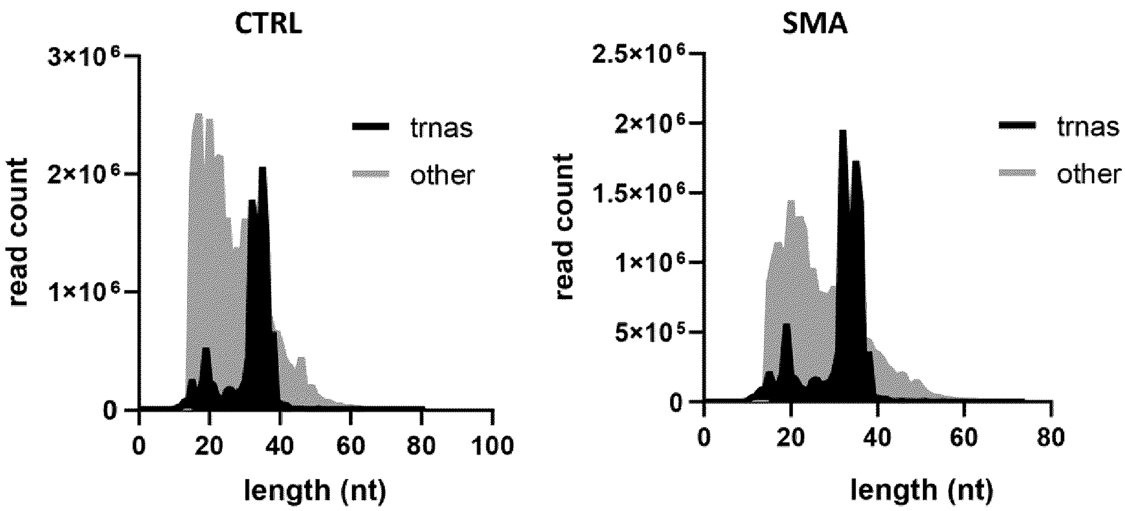


Figure 2

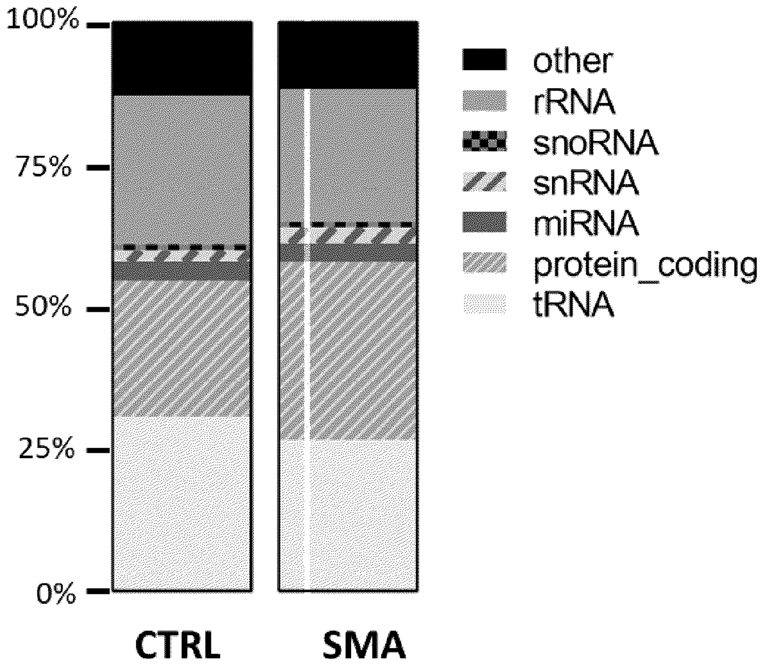


Figure 3

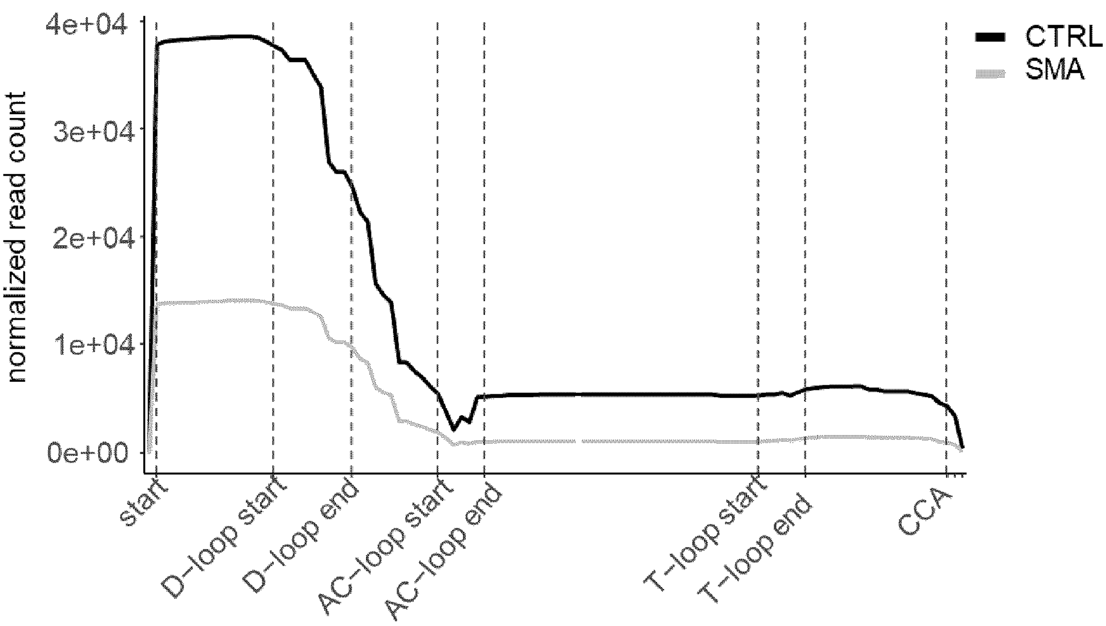


Figure 4

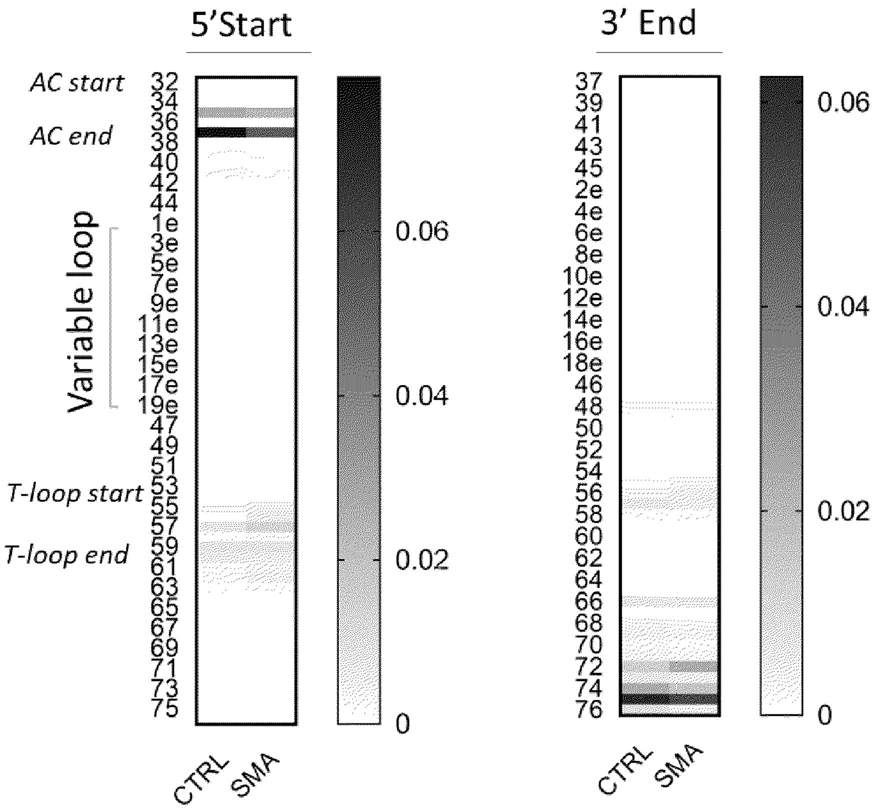


Figure 5

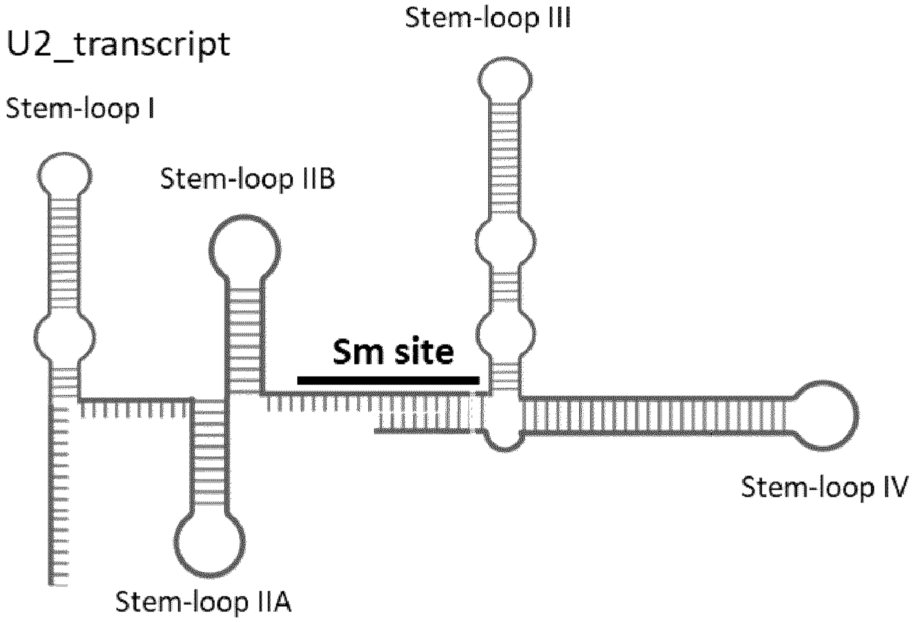


Figure 6

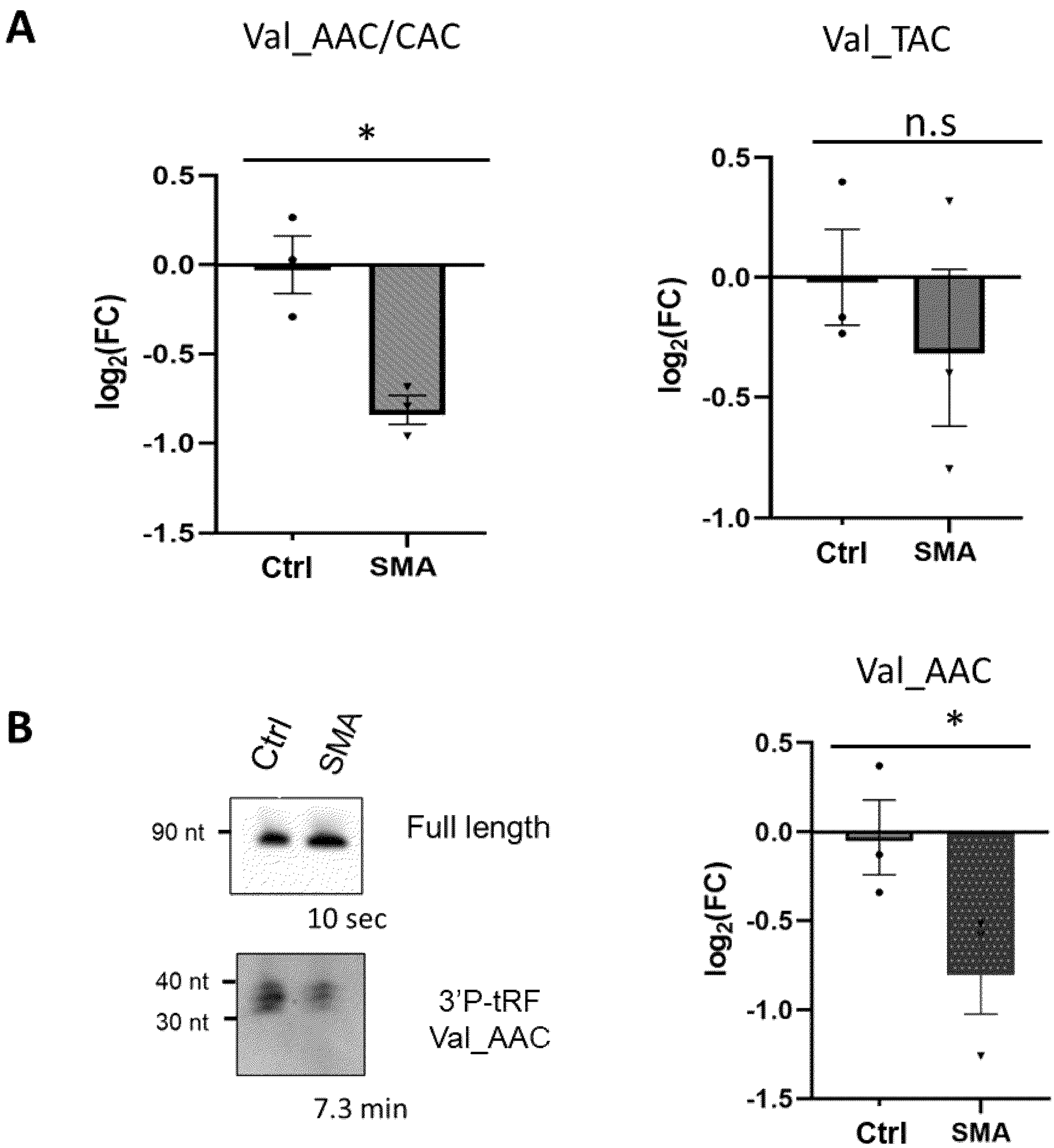
A
Val_AAC/CAC
*
0.5
0.0
-0.5
-1.0
-1.5
log2(FC)
Ctrl
SMA
Val_TAC
n.s
0.5
0.0
-0.5
-1.0
log2(FC)
Ctrl
SMA
B
Ctrl
SMA
90 nt
Full length
10 sec
40 nt
30 nt
3'P-tRF
Val_AAC
7.3 min
Val_AAC
*
0.5
0.0
-0.5
-1.0
-1.5
log2(FC)
Ctrl
SMA

Figure 7

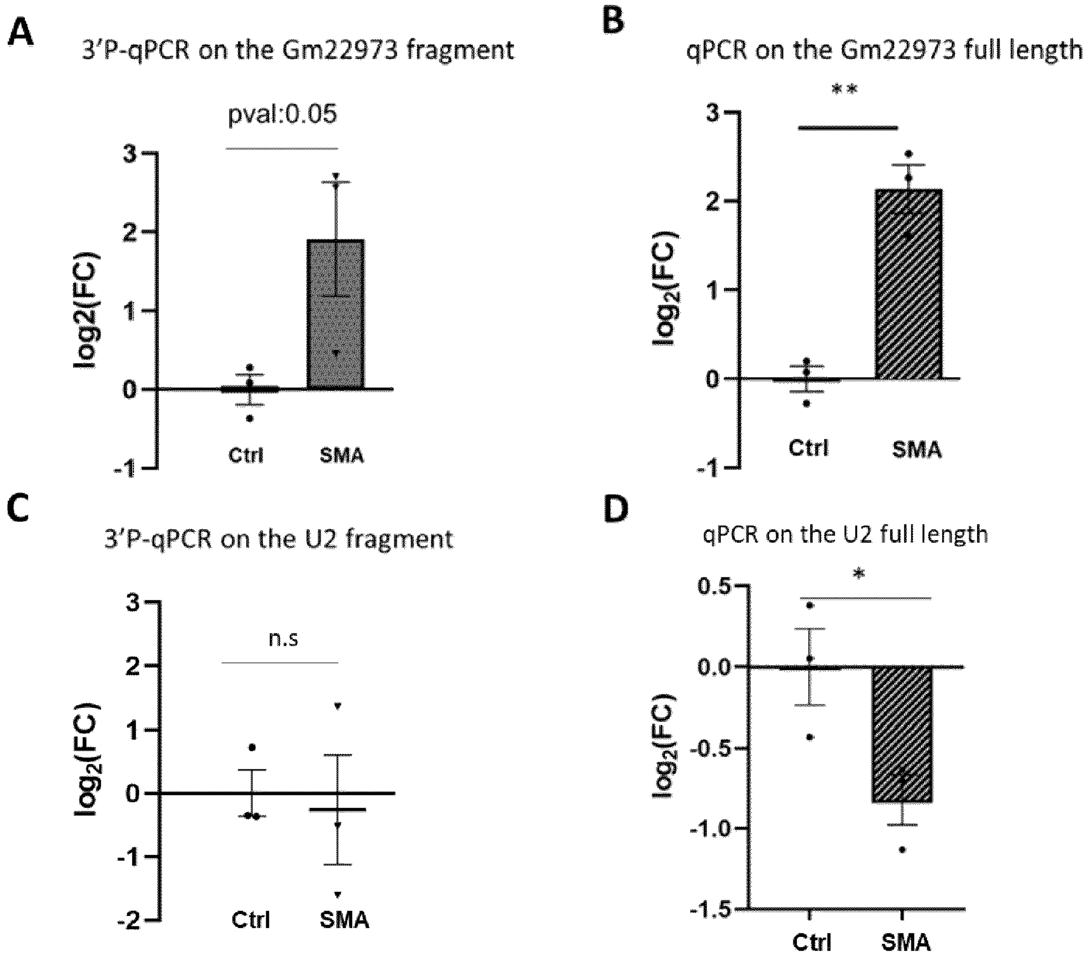


Figure 8

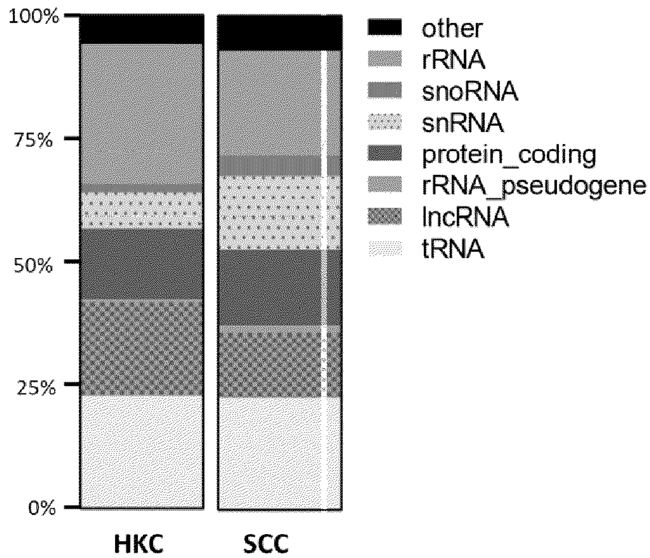


Figure 9

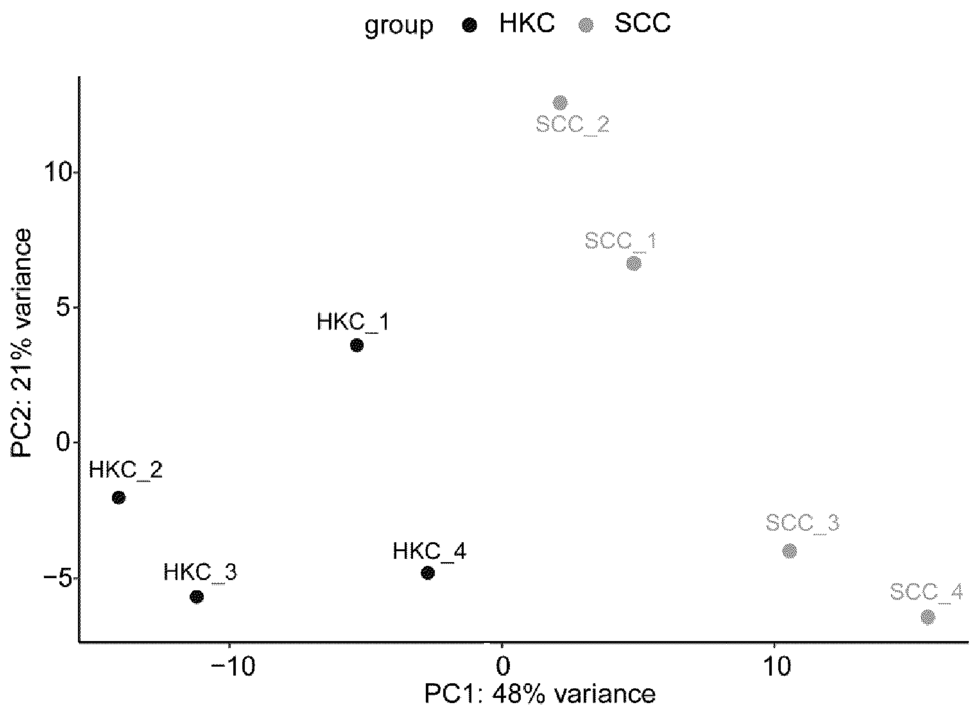


Figure 10

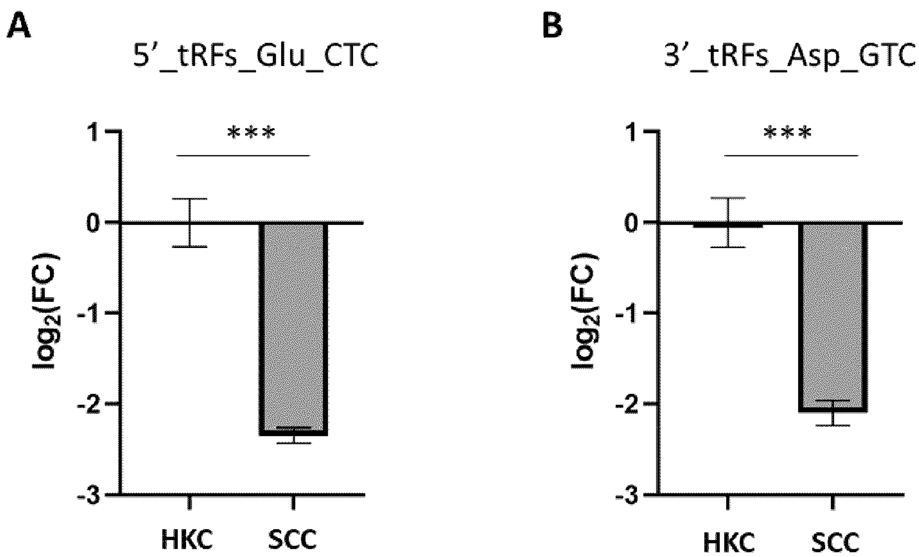


Figure 11

A.

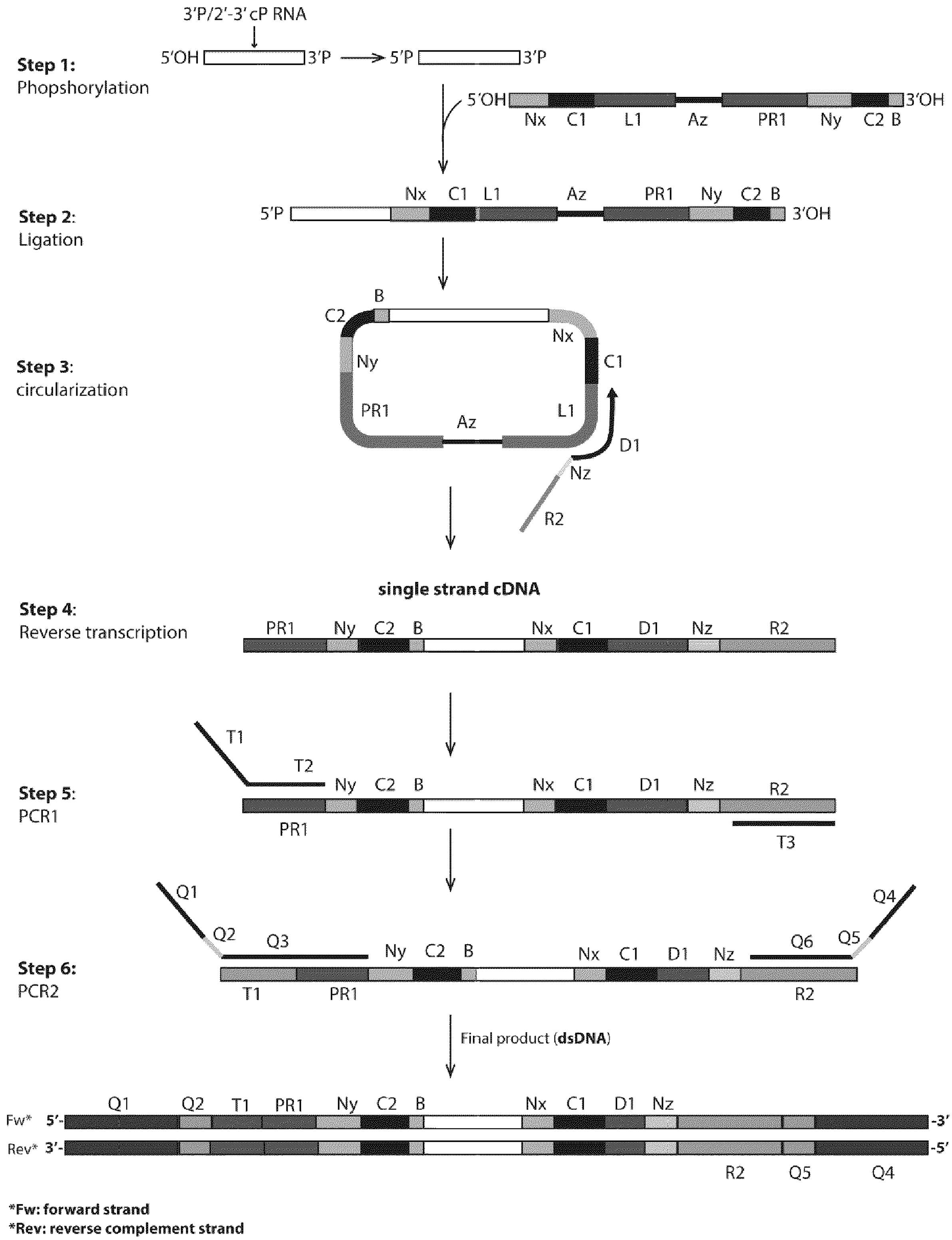
3'P/2'-3' cP RNA
Step 1:
Phopshorylation
5'OH
3'P
5'P
3'P
5'OH
3'OH
Nx C1 L1 Az PR1 Ny C2 B
Step 2:
Ligation
5'P
3'OH
Step 3:
circularization
D1
Nz
R2
single strand cDNA
Step 4:
Reverse transcription
Step 5:
PCR1
T1
T2
T3
Step 6:
PCR2
Q1 Q2 Q3 Q4 Q5 Q6
Final product (dsDNA)
Fw* 5'- -3'
Rev* 3'- -5'
*Fw: forward strand
*Rev: reverse complement strand

Figure 12

B.

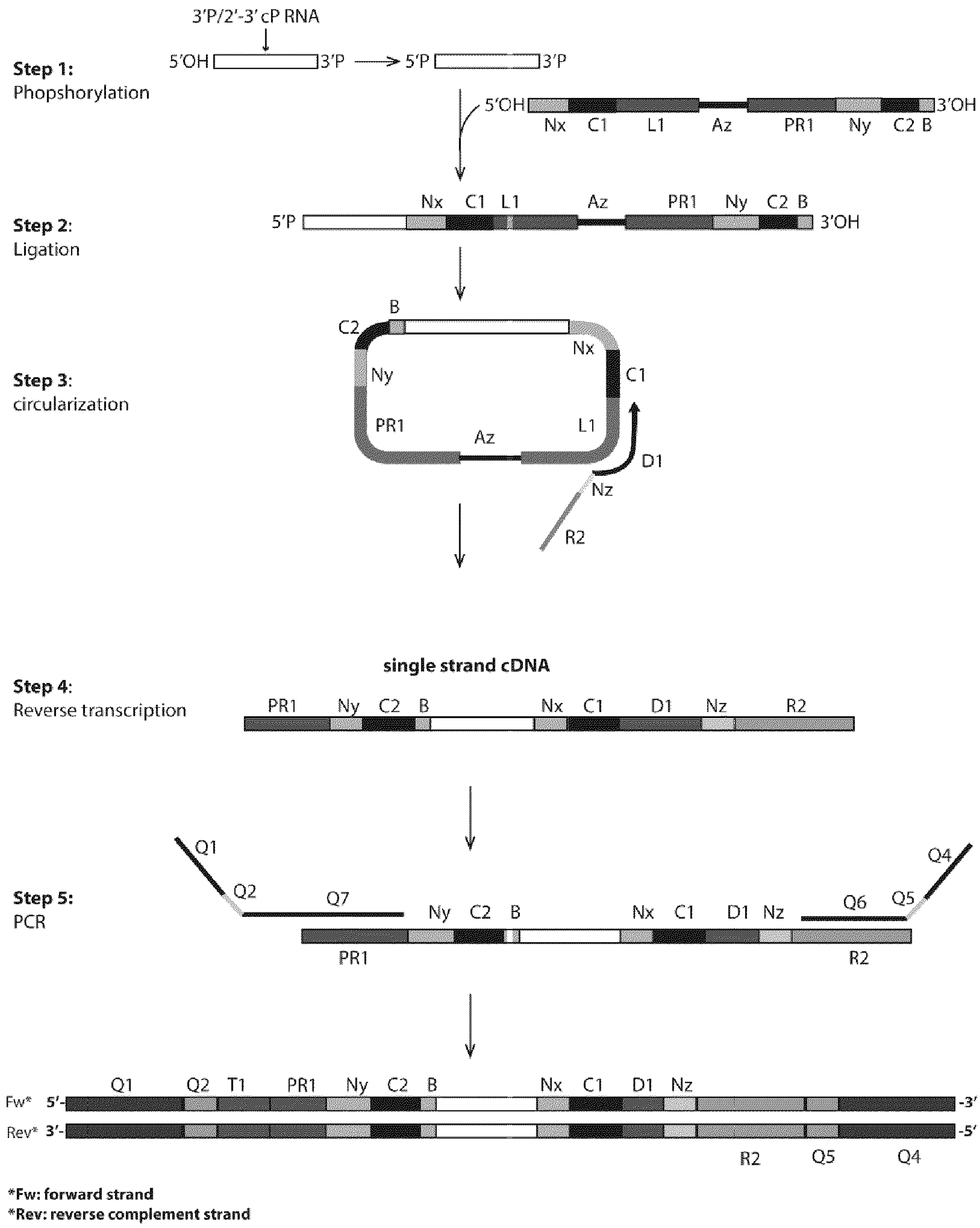


Figure 12/cont.

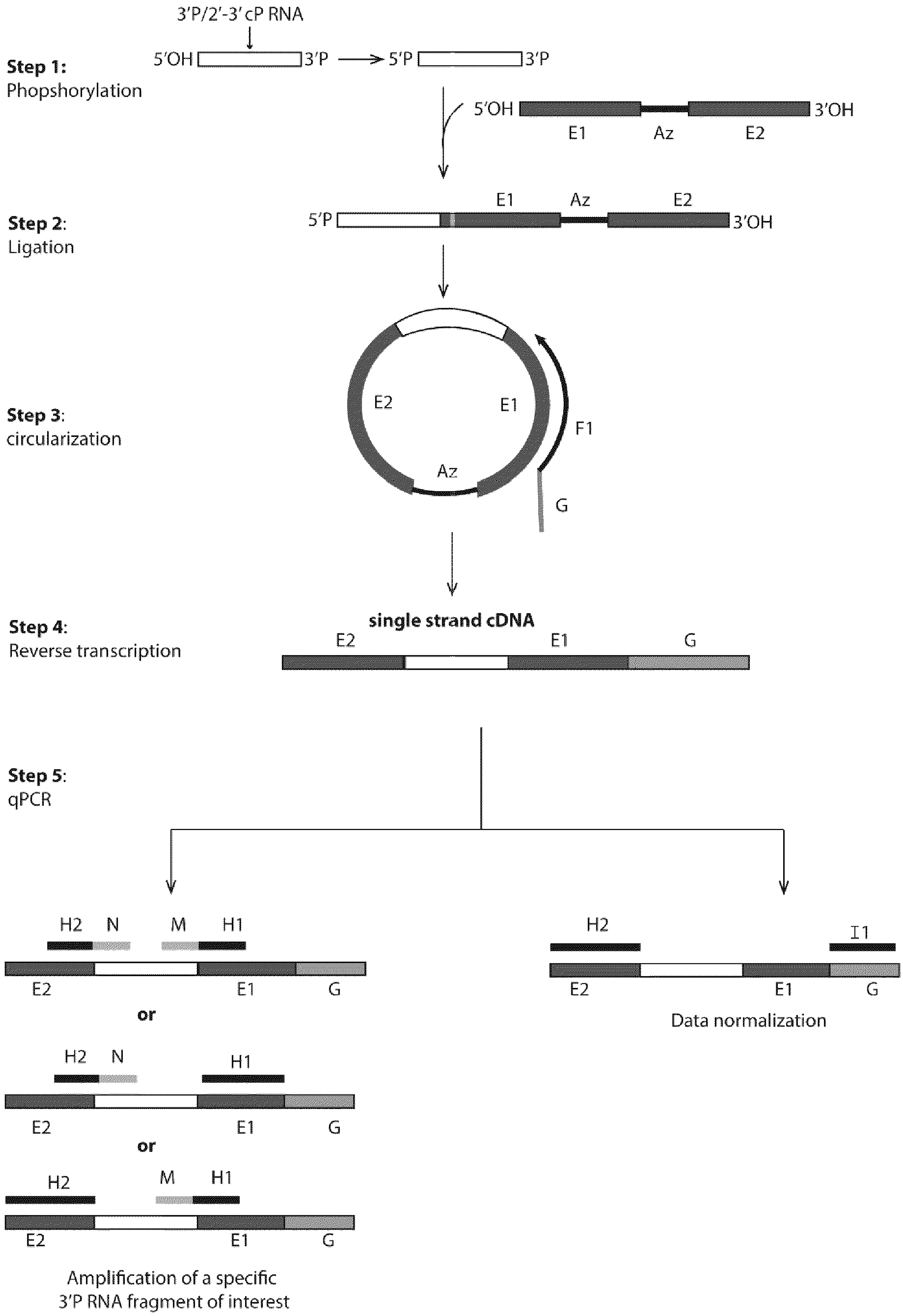
3'P/2'-3' cP RNA
Step 1:
Phopshorylation
5'OH
3'P
5'P
3'P
5'OH
3'OH
E1
Az
E2
Step 2:
Ligation
E1
Az
E2
5'P
3'OH
Step 3:
circularization
E2
E1
F1
Az
G
Step 4:
Reverse transcription
single strand cDNA
E2
E1
G
Step 5:
qPCR
H2
N
M
H1
E2
E1
G
or
H2
N
H1
E2
E1
G
or
H2
M
H1
E2
E1
G
Amplification of a specific
3'P RNA fragment of interest
H2
I1
E2
E1
G
Data normalization

Figure 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Applied Biosystems SOLiD™ 4 System Library Preparation Guide*, April 2010, https://tools.thermofisher.com/content/sfs/manuals/SOLiD4_Library-Preparation_ man.pdf
- GS FLX Titanium General Library Preparation Method Manual. April 2009
- *Applied Biosystems SOLiID™ 4 System Library Preparation Guide*, April 2010, https://tools.thermofisher.com/content/sfs/manuals/SOLiD4_Library-Preparation_ man.pdf
- Illumina Adapter Sequences. *Document # 1000000002694*, May 2019, https://www.science.smith.edu/cmbs/wp-content/uploads/sites/36/2020/01/illumina-adapter-sequences-1000000002694-11.pdf
- Amplicon LoopSeq™ for AVITI. *Document # MA-00023 Rev. C*, June 2023
- Element Elevate™ Library Prep. *Document # MA-00004 Rev. B*, June 2023
- ADAPTING LIBRARIES FOR THE G4™ - INSERT ONLY. *Document #600024 Rev. 0*, May 2023, https://singulargenomics.com/wp-content/uploads/2023/06/Adapting-Library-Insert-600024.pdf
- **SLAMON, D. J. et al.** Use of chemotherapy plus a monoclonal antibody against HER2 for metastatic breast cancer that overexpresses HER2.. *N Engl J Med*, 2001, vol. 344, 783-92
- **GOOSSENS, N.** ; **NAKAGAWA, S.** ; **SUN, X.** ; **HOSHIDA, Y.** Cancer biomarker discovery and validation.. *Transl Cancer Res*, 2015, vol. 4, 256-269
- **SPARANO, J. A. et al.** Adjuvant Chemotherapy Guided by a 21-Gene Expression Assay in Breast Cancer.. *New England Journal of Medicine*, 2018, vol. 379, 111-121
- **WANG, K. et al.** Circular RNA mediates cardiomyocyte death via miRNA-dependent upregulation of MTP18 expression.. *Cell Death Differ*, 2017, vol. 24, 1111-1120
- **HONDA, S. et al.** Sex hormone-dependent tRNA halves enhance cell proliferation in breast and prostate cancers.. *Proceedings of the National Academy of Sciences*, 2015, vol. 112, E3816-E3825
- **TANG, J. et al.** A novel biomarker Linc00974 interacting with KRT19 promotes proliferation and metastasis in hepatocellular carcinoma.. *Cell Death Dis*, 2014, vol. 5, e1549-e1549
- **PARDINI, B.** ; **SABO, A. A.** ; **BIROLO, G.** ; **CALIN, G. A.** Noncoding RNAs in Extracellular Fluids as Cancer Biomarkers: The New Frontier of Liquid Biopsies.. *Cancers (Basel)*, 2019, vol. 11, 1170
- **PAIK, S. et al.** Gene Expression and Benefit of Chemotherapy in Women With Node-Negative, Estrogen Receptor-Positive Breast Cancer.. *Journal of Clinical Oncology*, 2006, vol. 24, 3726-3734
- **ROUNDTREE, I. A.** ; **EVANS, M. E.** ; **PAN, T.** ; **HE, C.** Dynamic RNA Modifications in Gene Expression Regulation.. *Cell*, 2017, vol. 169, 1187-1200
- **FRYE, M.** ; **HARADA, B. T.** ; **BEHM, M.** ; **HE, C.** RNA modifications modulate gene expression during development.. *Science*, 1979, vol. 361, 1346-1349
- **MEYER, K. D.** ; **JAFFREY, S. R.** Rethinking m A Readers, Writers, and Erasers.. *Annu Rev Cell Dev Biol*, 2017, vol. 33, 319-342
- **ZHOU, J. et al.** Dynamic m6A mRNA methylation directs translational control of heat shock response. *Nature*, 2015, vol. 526, 591-594
- **SHIGEMATSU, M.** ; **MORICHIKA, K.** ; **KAWAMURA, T.** ; **HONDA, S.** ; **KIRINO, Y.** Genome-wide identification of short 2',3'-cyclic phosphate-containing RNAs and their regulation in aging.. *PLoS Genet*, 2019, vol. 15, e1008469
- **SHIGEMATSU, M.** ; **KAWAMURA, T.** ; **KIRINO, Y.** Generation of 2',3'-Cyclic Phosphate-Containing RNAs as a Hidden Layer of the Transcriptome.. *Front Genet*, 2018, vol. 9
- **SIDRAUSKI, C.** ; **WALTER, P.** The Transmembrane Kinase Irelp Is a Site-Specific Endonuclease That Initiates mRNA Splicing in the Unfolded Protein Response.. *Cell*, 1997, vol. 90, 1031-1039
- **TROTTA, C. R. et al.** The Yeast tRNA Splicing Endonuclease: A Tetrameric Enzyme with Two Active Site Subunits Homologous to the Archaeal tRNA Endonucleases.. *Cell*, 1997, vol. 89, 849-858
- **ZHU, L. et al.** Exosomal tRNA-derived small RNA as a promising biomarker for cancer diagnosis.. *Mol Cancer*, 2019, vol. 18, 74
- **MAUTE, R. L. et al.** tRNA-derived microRNA modulates proliferation and the DNA damage response and is down-regulated in B cell lymphoma.. *Proceedings of the National Academy of Sciences*, 2013, vol. 110, 1404-1409

- **GOODARZI, H. et al.** Endogenous tRNA-Derived Fragments Suppress Breast Cancer Progression via YBX1 Displacement.. *Cell*, 2015, vol. 161, 790-802
- **HOGG, M. C. et al.** 5'ValCAC tRNA fragment generated as part of a protective angiogenin response provides prognostic value in amyotrophic lateral sclerosis.. *Brain Commun*, 2020, vol. 2
- **GUZZI, N. et al.** Pseudouridine-modified tRNA fragments repress aberrant protein synthesis and predict leukaemic progression in myelodysplastic syndrome.. *Nat Cell Biol*, 2022, vol. 24, 299-306
- **SCHIMMEL, P.** The emerging complexity of the tRNA world: mammalian tRNAs beyond protein synthesis.. *Nat Rev Mol Cell Biol*, 2018, vol. 19, 45-58
- **YU, M. et al.** tRNA-derived RNA fragments in cancer: current status and future perspectives.. *J Hematol Oncol*, 2020, vol. 13, 121
- **WANG, Q. et al.** Identification and Functional Characterization of tRNA-derived RNA Fragments (tRFs) in Respiratory Syncytial Virus Infection.. *Molecular Therapy*, 2013, vol. 21, 368-379
- **IVANOV, P. et al.** G-quadruplex structures contribute to the neuroprotective effects of angiogenin-induced tRNA fragments.. *Proceedings of the National Academy of Sciences*, 2014, vol. 111, 18201-18206
- **BOULTER, N. et al.** A simple, accurate and universal method for quantification of PCR.. *BMC Biotechnol*, 2016, vol. 16, 27
- **PFAFFL, M. W.** Relative expression software tool (REST(C)) for group-wise comparison and statistical analysis of relative expression results in real-time PCR.. *Nucleic Acids Res*, 2002, vol. 30, 36e-336
- **XI, X. et al.** RNA Biomarkers: Frontier of Precision Medicine for Cancer.. *Noncoding RNA*, 2017, vol. 3, 9
- **MERCURI, E.** ; **SUMNER, C. J.** ; **MUNTONI, F.** ; **DARRAS, B. T.** ; **FINKEL, R. S.** Spinal muscular atrophy.. *Nat Rev Dis Primers*, 2022, vol. 8, 52
- **OBERBAUER, V.** ; **SCHAEFER, M.** tRNA-Derived Small RNAs: Biogenesis, Modification, Function and Potential Impact on Human Disease Development.. *Genes (Basel)*, 2018, vol. 9, 607
- **KUMAR, P.** ; **MUDUNURI, S. B.** ; **ANAYA, J.** ; **DUTTA, A.** tRFdb: a database for transfer RNA fragments.. *Nucleic Acids Res*, 2015, vol. 43, D141-D145
- **KUMAR, P** ; **ANAYA, J.** ; **MUDUNURI, S. B.** ; **DUTTA, A.** Meta-analysis of tRNA derived RNA fragments reveals that they are evolutionarily conserved and associate with AGO proteins to recognize specific RNA targets.. *BMC Biol*, 2014, vol. 12, 78
- **SHEN, Y. et al.** Transfer RNA-derived fragments and tRNA halves: biogenesis, biological functions and their roles in diseases.. *J Mol Med*, 2018, vol. 96, 1167-1176
- **FANIA, L. et al.** Cutaneous Squamous Cell Carcinoma: From Pathophysiology to Novel Therapeutic Approaches.. *Biomedicines*, 2021, vol. 9, 171
- **DEL PIANO, A. et al.** Phospho-RNA sequencing with circAID-p-seq.. *Nucleic Acids Res*, 2022, vol. 50, e23-e23
- **CLAMER, M et al.** Active Ribosome Profiling with RiboLace.. *Cell Rep*, 2018, vol. 25, 1097-1108.e5
- **DOKTOR, T. K. et al.** RNA-sequencing of a mouse-model of spinal muscular atrophy reveals tissue-wide changes in splicing of U12-dependent introns. *Nucleic Acids Res*, 2017, vol. 45, 395-416
- **IVANOV, P.** ; **EMARA, M. M.** ; **VILLEN, J.** ; **GYGI, S. P.** ; **ANDERSON, P.** Angiogenin-Induced tRNA Fragments Inhibit Translation Initiation.. *Mol Cell*, 2011, vol. 43, 613-623
- **SAIKIA, M. et al.** Angiogenin-Cleaved tRNA Halves Interact with Cytochrome c, Protecting Cells from Apoptosis during Osmotic Stress.. *Mol Cell Biol*, 2014, vol. 34, 2450-2463
- **SINGH, R. N.** ; **HOWELL, M. D.** ; **OTTESEN, E. W.** ; **SINGH, N. N.** Diverse role of survival motor neuron protein.. *Biochimica et Biophysica Acta (BBA) - Gene Regulatory Mechanisms*, 2017, vol. 1860, 299-315
- **LOVE, M. I.** ; **HUBER, W.** ; **ANDERS, S.** Moderated estimation of fold change and dispersion for RNA-seq data with DESeq2.. *Genome Biol*, 2014, vol. 15, 550